# EUROPEAN PATENT APPLICATION

(11) **EP 2 937 335 A1**
(43) Date of publication of application: **28.10.2015**
(21) Application number: 13864883.7
(22) Date of filing: 17.12.2013
(51) Int. Cl.: C07D 205/04, A61K 31/397, A61K 31/401, A61K 31/4025, A61K 31/427, A61K 31/4439, A61K 31/4709, A61K 31/4725, A61K 31/5377, A61P 1/00, A61P 1/04, A61P 1/08, A61P 11/00, A61P 11/06, A61P 13/10, A61P 17/00, A61P 17/04, A61P 25/00, A61P 25/04, A61P 29/00

(54) **HETEROCYCLIC AMIDE DERIVATIVE, AND MEDICINE CONTAINING SAME**

(30) Priority: 18.12.2012 JP 2012276283
(71) Applicant: Ajinomoto Co., Inc., Tokyo 104-8315 (JP)
(72) Inventor: KOBAYASHI, Kaori, Kawasaki-shi Kanagawa 210-8681 (JP); SUZUKI, Tamotsu, Kawasaki-shi Kanagawa 210-8681 (JP); OKUZUMI, Tatsuya, Kawasaki-shi Kanagawa 210-8681 (JP)
(74) Representative: Nicholls, Kathryn Margaret
(86) International application number: PCT/JP2013/083801
(87) International publication number: WO 2014/098098

(57) **Abstract**

A compound represented by the formula (I): 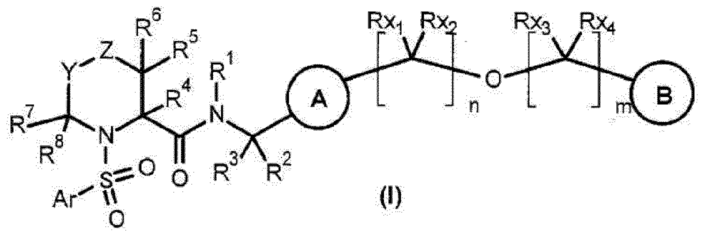 wherein each symbol is as defined in the DESCRIPTION, has a TRPA1 antagonist activity, and the compound and a medicament containing the compound are useful for the prophylaxis or treatment of diseases involving TRPA1 antagonist and TRPA1.

## Description

### Technical Field

The present invention relates to a novel heterocyclic amide compound having a Transient Receptor Potential Ankyrin 1 (TRPA1) antagonist activity and a pharmaceutical composition containing the compound, as well as a medicament useful for the prophylaxis or treatment of a disease involving TRPA1.

### Background Art

Transient Receptor Potential Ankyrin 1 (TRPA1) is a non-selective cation channel belonging to the Transient Receptor Potential (TRP) channel superfamily. Like other TRP channel family, it has 6 transmembrane domains and forms a tetramer consisting of 4 subunits. TRPA1 is a ligand dependent ion channel, which changes structure by the binding of ligand. As a result, the channel opens to allow intracellular flow of cations such as calcium ion, sodium ion and the like, thereby controlling the membrane potential of the cells. As the TRPA1 ligand, stimulant natural substances (e.g., allylisothiocyanate (AITC), cinnamaldehyde and the like), environmental stimulants (e.g., formalin, acrolein and the like), endogenous substances (e.g., 4-hydroxynonenal and the like) and the like are known (non-patent documents 1 - 3). It is known that the ligand is also activated by cold stimulation, intracellular Ca²⁺ and the like (non-patent document 1). Many ligands such as AITC, cinnamaldehyde and the like form a covalent bond with the cysteine residue and the lysine residue at the N-terminal in the cytoplasm, and activate the channel (non-patent document 2). In addition, intracellular Ca²⁺ is considered to bind to the N-terminal EF hand domain and opens the channel (non-patent document 4). TRPA1 has been reported to be highly expressed in the sensory nerves such as spinal cord nerve, vagus nerve, trigeminal nerve and the like. TRPA1 has been reported to be co-expressed with perception·pain-related markers such as TRPV1, calcitonin gene related peptide (CGRP), substance P and the like (non-patent documents 5 - 7). Therefore, it is considered that, once TRPA1 present in the sensory nerve is activated by various stimulations, channel opening and depolarization of the cellular membrane occur, neuropeptides (CGRP, substance P) are liberated from the nerve ending, and perception such as nociception and the like is transmitted.

In fact, it has been reported that TRPA1 gene knockdown by the gene specific antisense method improves hyperalgesia induced by inflammation and nerve damage in pain model (non-patent document 8). Also, it has been reported that a pain behavior induced by formalin disappears in TRPA1 gene knockout mouse (non-patent document 9). From the above, TRPA1 is considered to play an important role in the nociceptive transmission, and is expected as a treatment target in pain-associated diseases such as nociceptive pain, neuropathic pain and the like.

TRPA1 is known to show high expression in the afferent sensory nerve projected on the gastrointestinal tract such as esophagus, stomach, large intestine and the like. It has been reported that TRPA1 knockdown decreases nociception reaction due to extension of stomach (non-patent document 10), and large intestine hyperalgesia induced by AITC and 2,4,6-trinitrobenzenesulfonic acid (TNBS) is normalized in TRPA1 gene knockout mouse (non-patent document 11). From the above, TRPA1 is suggested to play an important role in the perception· nociception transmission in the gastrointestinal tract, and is expected to be effective for the treatment of digestive tract diseases such as functional dyspepsia, irritable bowel syndrome, erosive esophagitis, inflammatory bowel disease (Crohn's disease, ulcerative colitis), pancreatitis and the like.

Furthermore, TRPA1 plays a key role in the detection of a noxious substance in the trachea. It has been reported that TRPA1 gene knockout suppresses inflammation of the trachea in OVA model (non-patent document 12). Therefore, antagonism of TRPA1 is considered to be also useful for pulmonary diseases such as asthma, chronic coughing, COPD and the like.

As other diseases involving TRPA1, dermatic diseases such as pruritus, atopic dermatitis, burn and the like (non-patent documents 13, 14), inflammatory diseases such as burn, osteoarthritis and the like (non-patent document 15), bladder diseases such as overactive bladder·abnormal urination·cystitis and the like (non-patent document 16), neurological diseases such as anticancer agent-induced neuropathy and the like (non-patent document 17) and the like are known. Thus, a compound capable of functional regulation of TRPA1 is industrially and therapeutically useful in many aspects. In particular, a compound that antagonizes TRPA1 is highly expected as a new therapeutic drug for pain diseases, digestive tract diseases, lung diseases, dermatic diseases, inflammatory diseases, bladder diseases and neurological diseases in human.

As a TRPA1 antagonist, a compound of the following formula has been reported (patent document 1).

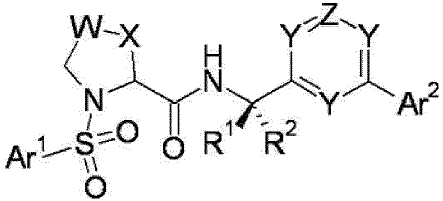

wherein definition of each symbol is as described in patent document 1.

However, these compounds are structurally different from the compound represented by the formula (I) of the present invention to be described later.

Also, a compound having the following structure is known (patent document 2).

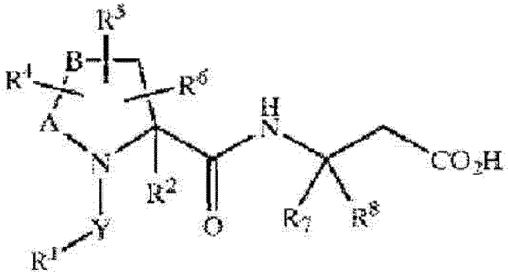

wherein definition of each symbol is as described in patent document 2.

However, these compounds are VLA-4 and α4β7 antagonists, different from the compound of the present invention in the action mechanism, contain carboxylic acid or an alkyl group substituted by carboxylic acid as a substituent on a carbon atom adjacent to the amide bond, and are structurally different from the compound of the present invention.

### Document List

### non-patent documents

non-patent document 1: Bandell M, et al., Neuron. 2004 Mar 25; 41(6):849-57.
non-patent document 2: Macpherson LJ, et al., Nature. 2007 445(7127):541-5.
non-patent document 3: Trevisani M, et al., Proc Natl Acad Sci U S A. 2007 104 (33) :13519-24.
non-patent document 4: Zurborg S, et al., Nat Neurosci. 2007 10(3):277-9.
non-patent document 5: Nagata K, et al., J Neurosci. 2005 25(16):4052-61.
non-patent document 6: Story GM, et al., Cell. 2003 112(6):819-29.
non-patent document 7: Bautista DM, et al., Proc Natl Acad Sci U S A. 2005 102(34):12248-52.
non-patent document 8: Obata K, et al., J Clin Invest. 2005 115(9):2393-401.
non-patent document 9: McNamara CR, et al., Proc Natl Acad Sci U S A. 2007 104(33):13525-30.
non-patent document 10: Kondo T, et al., Digestion. 2010; 82(3):150-5.
non-patent document 11: Cattaruzza F, et al., Am J Physiol Gastrointest Liver Physiol. 2010 298(1):G81-91.
non-patent document 12: Caceres AI, et al., Proc Natl Acad Sci U S A. 2009 106(22):9099-104.
non-patent document 13: Xiao B, and Patapoutian A., Nat Neurosci. 2011 May; 14(5):540-2.
non-patent document 14: Wilson SR, et al., Nat Neurosci. 2011 May; 14(5):595-602.
non-patent document 15: McGaraughty S, et al., Mol Pain. 2010 Mar 5; 6:14.
non-patent document 16: Andersson KE, et al., BJU Int. 2010 Oct; 106(8):1114-27.
non-patent document 17: Nassini R, et al., Pain. 2011 Jul; 152 (7) :1621-31.

### patent document

patent document 1: WO 2010/141805
patent document 2: US-B-6645939

### Summary of the Invention

### Problems to be Solved by the Invention

The present invention aims to provide a novel compound having a transient receptor potential ankyrin 1 (TRPA1) antagonist activity.

The present invention also aims to provide a TRPA1 antagonist.

The present invention also aims to provide a medicament containing the above-mentioned novel compound.

The present invention also aims to provide a medicament useful for the prophylaxis or treatment of a disease involving TRPA1.

### Means of Solving the Problems

In view of the aforementioned situation, the present inventors have conducted various studies and found that a certain particular heterocyclic amide compound has a TRPA1 antagonist activity, and is useful for the prophylaxis and/or treatment of diseases involving TRPA1 (e.g., pain associated diseases, digestive tract diseases, lung diseases, bladder diseases, inflammatory diseases, dermatic diseases, and neurological diseases), which resulted in the completion of the present invention.

Accordingly, the present invention provides the following.
[1] A medicament composed of a compound represented by the formula (I): 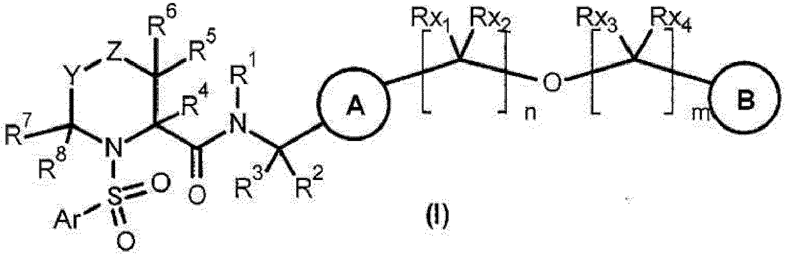 wherein
   Ar is a C₆₋₁₀ aryl group optionally having 1 - 4 substituents or a C₁₋₉ heteroaryl group optionally having 1 - 4 substituents;
   Y is -C(Ry1)(Ry2)-, or a single bond;
   Z is -C(Rz1)(Rz2)-, an oxygen atom, a sulfur atom, or a single bond;
   n is 0 or 1;
   m is 0 or 1;
   provided n+m≤1;
   partial structure (1) 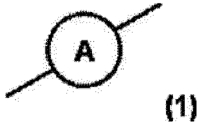 is a phenylene group optionally having 1 - 4 substituents, or a divalent group optionally having 1 - 3 substituents of 5-membered or 6-membered heteroaromatic ring containing any 1 - 3 hetero atoms selected from oxygen atom, nitrogen atom and sulfur atom;
   partial structure (2) 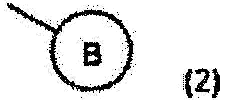 is a C₆₋₁₀ aryl group optionally having 1 - 4 substituents or a C₁₋₉ heteroaryl group optionally having 1 - 4 substituents;
   R¹ is a hydrogen atom, a C₁₋₆ alkyl group, a C₂₋₆ alkenyl group, a C₂₋₆ alkynyl group, a halogeno C₁₋₆ alkyl group, a cyclic C₃₋₆ alkyl group, or a C₁₋₆ alkyl group substituted by cyclic C₃₋₆ alkyl group;
   R² and R³ are the same or different and each is a hydrogen atom, a C₁₋₆ alkyl group, a C₂₋₆ alkenyl group, a C₂₋₆ alkynyl group; R⁴ - R⁸, Ry1 - Ry2, Rz1 and Rz2 are the same or different and each is a hydrogen atom, a C₁₋₆ alkyl group, a C₂₋₆ alkenyl group, a C₂₋₆ alkynyl group, a halogeno C₁₋₆ alkyl group, a C₁₋₆ alkoxy group, a hydroxyl group, a halogeno C₁₋₆ alkoxy group, an amino group, an amino group mono- or di-substituted by a C₁₋₆ alkyl group, or a halogeno group;
   respective R⁴ - R⁸, Ry1 - Ry2, Rz1 or Rz2 on adjacent carbon atom are optionally joined to form a double bond and/or a ring; respective R⁵ and R⁶, R⁷ and R⁸, Ry1 and Ry2, or Rz1 and Rz2 on the same carbon atom are optionally joined to form a ring; Rx1 - Rx4 are the same or different and each is a hydrogen atom, a C₁₋₆ alkyl group, a C₂₋₆ alkenyl group, a C₂₋₆ alkynyl group, a C₁₋₆ alkyl group substituted by a hydroxyl group, a C₁₋₆ alkyl group substituted by an amino group, a C₁₋₆ alkyl group substituted by an amino group mono- or di-substituted by a C₁₋₆ alkyl group;
   and respective Rx1 and Rx2, or Rx3 and Rx4 on the same carbon atom are optionally joined to form a ring, or a pharmaceutically acceptable salt thereof.
[2] The medicament of the above-mentioned [1], wherein Y and/or Z are/is a single bond.
[3] The medicament of the above-mentioned [1] or [2], wherein, in the aforementioned formula (I),
   Ar is a phenyl group optionally having 1 or 2 substituents or a 5- or 6-membered monocyclic heteroaryl group optionally having 1 or 2 substituents.
[4] The medicament of any of the above-mentioned [1] - [3], wherein, in the aforementioned formula (I), the partial structure (1) is either a phenylene group optionally having 1 - 3 substituents, or a divalent group of a pyridine ring optionally having 1 - 3 substituents.
[5] The medicament of any of the above-mentioned [1] - [3], wherein, in the aforementioned formula (I), the partial structure (1) is 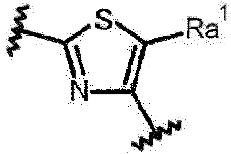 in the above-mentioned structure, Ra¹ is a hydrogen atom or a substituent.
[6] The medicament of any of the above-mentioned [1] - [5], wherein, in the aforementioned formula (I),
   the partial structure (2) is a phenyl group optionally having 1 - 3 substituents, or a pyridyl group, a quinolyl group or an isoquinolyl group, each of which optionally has 1 - 3 substituents.
[7] The medicament of any of the above-mentioned [1] - [6], wherein, in the aforementioned formula (I),
   n=0.
[8] The medicament of any of the above-mentioned [1] - [7], wherein, in the aforementioned formula (I),
   R² and R³ are each a hydrogen atom.
[9] The medicament of any of the above-mentioned [1] - [8], wherein, in the aforementioned formula (I),
   the partial structure (2) is a phenyl group optionally having a substituent or a pyridyl group optionally having a substituent.
[10] The medicament of any of the above-mentioned [1] - [9], which is a TRPA1 antagonist.
[11] The medicament of the above-mentioned [10], which is for the prophylaxis and/or treatment of a disease involving TRPA1.
[12] The medicament of the above-mentioned [11], wherein the disease involving TRPA1 is selected from the group consisting of pain associated diseases, inflammatory diseases, digestive tract diseases, lung diseases, bladder diseases, dermatic diseases, and neurological diseases.
[13] The medicament of the above-mentioned [11], wherein the disease involving TRPA1 is selected from chronic pain, acute pain, asthma, chronic obstructive pulmonary disease, functional gastrointestinal disorder, erosive esophagitis, inflammatory bowel disease, and pruritus.
[14] The compound represented by the formula (IA): 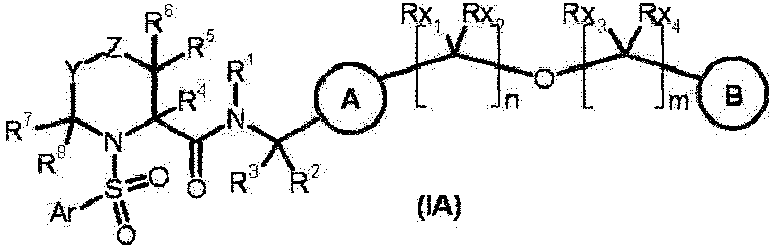 wherein
   Ar is a C₆₋₁₀ aryl group optionally having 1 - 4 substituents or a C₁₋₉ heteroaryl group optionally having 1 - 4 substituents;
   Y is -C(Ry1)(Ry2)-, or a single bond;
   Z is -C(Rz1)(Rz2)-, an oxygen atom, a sulfur atom, or a single bond;
   n is 0 or 1;
   m is 0 or 1;
   provided n+m≤1;
   partial structure (1)

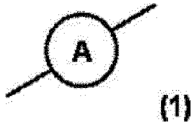

   is a phenylene group optionally having 1 - 4 substituents, or a divalent group optionally having 1 - 3 substituents of 5-membered or 6-membered heteroaromatic ring containing any 1 - 3 hetero atoms selected from oxygen atom, nitrogen atom and sulfur atom;
   partial structure (2)

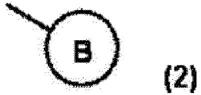

   is a C₆₋₁₀ aryl group optionally having 1 - 4 substituents or a C₁₋₉ heteroaryl group optionally having 1 - 4 substituents;
   R¹ is a hydrogen atom, a C₁₋₆ alkyl group, a C₂₋₆ alkenyl group,
   a C₂₋₆ alkynyl group, a halogeno C₁₋₆ alkyl group, a cyclic C₃₋₆ alkyl group, or a C₁₋₆ alkyl group substituted by a cyclic C₃₋₆ alkyl group;
   R² and R³ are the same or different and each is a hydrogen atom, a C₁₋₆ alkyl group, a C₂₋₆ alkenyl group, a C₂₋₆ alkynyl group;
   R⁴ - R⁸, Ry1 - Ry2, Rz1 and Rz2 are the same or different and each is a hydrogen atom, a C₁₋₆ alkyl group, a C₂₋₆ alkenyl group, a C₂₋₆ alkynyl group, a halogeno C₁₋₆ alkyl group, a C₁₋₆ alkoxy group, a hydroxyl group, a halogeno C₁₋₆ alkoxy group, an amino group, an amino group mono- or di-substituted by a C₁₋₆ alkyl group, or a halogeno group;
   respective R⁴ - R⁸, Ry1 - Ry2, Rz1 or Rz2 on adjacent carbon atom are optionally joined to form a double bond and/or a ring;
   respective R⁵ and R⁶, R⁷ and R⁸, Ry1 and Ry2, or Rz1 and Rz2 on the same carbon atom are optionally joined to form a ring;
   Rx1 - Rx4 are the same or different and each is a hydrogen atom, a C₁₋₆ alkyl group, a C₂₋₆ alkenyl group, a C₂₋₆ alkynyl group, a C₁₋₆ alkyl group substituted by a hydroxyl group, a C₁₋₆ alkyl group substituted by an amino group, a C₁₋₆ alkyl group substituted by an amino group mono- or di-substituted by a C₁₋₆ alkyl group;
   and respective Rx1 and Rx2, or Rx3 and Rx4 on the same carbon atom are optionally joined to form a ring, or a pharmaceutically acceptable salt thereof, excluding the following compounds:
   N-[[2-(2-fluorophenoxy)phenyl]methyl]-1-(2-thienylsulfonyl)pyrrolidine-2-carboxamide (CAS Registry No. 1385210-42-3);
   ethyl 5-[[2-[[[(2-phenoxyphenyl)methyl]amino]carbonyl]-1-pyrrolidinyl]sulfonyl]-2-furancarboxylate (CAS Registry No. 1372166-14-7);
   N-[[3-fluoro-4-(3-pyridinyloxy)phenyl]methyl]-1-(phenylsulfonyl)-pyrrolidine-2-carboxamide (CAS Registry No. 1370950-04-1);
   N-[(3-phenoxyphenyl)methyl]-1-(2-thienylsulfonyl)-pyrrolidine-2-carboxamide (CAS Registry No. 1356772-35-4);
   N-[(2-phenoxyphenyl)methyl]-1-(2-thienylsulfonyl)-pyrrolidine-2-carboxamide (CAS Registry No. 1356571-93-1);
   N-[[6-(2,5-dimethylphenoxy)-3-pyridinyl]methyl]-1-(phenylsulfonyl)-pyrrolidine-2-carboxamide (CAS Registry No. 1315966-17-6);
   N-[[2-(4-fluorophenoxy)-4-pyridinyl]methyl]-1-(phenylsulfonyl)-pyrrolidine-2-carboxamide (CAS Registry No. 1315858-36-6);
   N-[[6-(3-fluorophenoxy)-3-pyridinyl]methyl]-1-(phenylsulfonyl)-pyrrolidine-2-carboxamide (CAS Registry No. 1315848-80-6);
   1-(phenylsulfonyl)-N-[[3-(2-pyridinylmethoxy)phenyl]methyl]-pyrrolidine-2-carboxamide (CAS Registry No. 1315835-86-9);
   (2S)-1-[(4-methylphenyl)sulfonyl]-N-[[2-(phenylmethoxy)-4-pyridinyl]methyl]-pyrrolidine-2-carboxamide (CAS Registry No. 1288924-64-0);
   1-[(4-fluorophenyl)sulfonyl]-N-[[6-(4-methoxyphenoxy)-3-pyridinyl]methyl]-pyrrolidine-2-carboxamide (CAS Registry No. 1277456-75-3);
   1-[(4-fluorophenyl)sulfonyl]-N-[[3-methoxy-4-(3-pyridinylmethoxy)phenyl]methyl]-pyrrolidine-2-carboxamide (CAS Registry No. 1277414-27-3);
   N-[1-[4-(3-pyridinylmethoxy)phenyl]ethyl]-1-(2-thienylsulfonyl)-pyrrolidine-2-carboxamide (CAS Registry No. 1277410-29-3);
   N-[[2-(phenylmethoxy)phenyl]methyl]-1-(2-thienylsulfonyl)-pyrrolidine-2-carboxamide (CAS Registry No. 1277400-48-2);
   1-[(4-fluorophenyl)sulfonyl]-N-[[3-methoxy-4-(phenylmethoxy)phenyl]methyl]-pyrrolidine-2-carboxamide (CAS Registry No. 1277358-57-2);
   N-[[2-(phenylmethoxy)phenyl]methyl]-1-(2-thienylsulfonyl)-pyrrolidine-2-carboxamide (CAS Registry No. 1277358-06-1);
   N-[[6-(4-fluorophenoxy)-3-pyridinyl]methyl]-1-(2-thienylsulfonyl)-pyrrolidine-2-carboxamide (CAS Registry No. 1277336-69-2);
   1-[(4-fluorophenyl)sulfonyl]-N-[1-[3-(2-pyridinylmethoxy)phenyl]ethyl]-pyrrolidine-2-carboxamide (CAS Registry No. 1277268-08-2);
   N-[[4-(phenoxymethyl)phenyl]methyl]-1-(2-thienylsulfonyl)-pyrrolidine-2-carboxamide (CAS Registry No. 1277253-49-2);
   N-[[3-(phenylmethoxy)phenyl]methyl]-1-(2-thienylsulfonyl)-pyrrolidine-2-carboxamide (CAS Registry No. 1277146-58-3);
   1-[(4-fluorophenyl)sulfonyl]-N-[[4-(3-pyridinylmethoxy)phenyl]methyl]-pyrrolidine-2-carboxamide (CAS Registry No. 1277055-81-8);
   1-[(4-fluorophenyl)sulfonyl]-N-[[2-(phenylmethoxy)-4-pyridinyl]methyl]-pyrrolidine-2-carboxamide (CAS Registry No. 1277032-72-0);
   N-[[3-(2-pyridinylmethoxy)phenyl]methyl]-1-[(2,4,6-trimethylphenyl)sulfonyl]-pyrrolidine-2-carboxamide (CAS Registry No. 1276991-71-9);
   1-(phenylsulfonyl)-N-[[3-(2-pyridinylmethoxy)phenyl]methyl]-pyrrolidine-2-carboxamide (CAS Registry No. 1276938-18-1);
   N-[[2-(phenylmethoxy)-4-pyridinyl]methyl]-1-(2-thienylsulfonyl)-pyrrolidine-2-carboxamide (CAS Registry No. 1276907-17-5);
   N-[[6-(2,5-dimethylphenoxy)-3-pyridinyl]methyl]-1-(2-thienylsulfonyl)-2-piperidinecarboxamide (CAS Registry No. 1276905-54-4);
   N-[[2-(phenoxymethyl)phenyl]methyl]-1-(2-thienylsulfonyl)-pyrrolidine-2-carboxamide (CAS Registry No. 1276856-98-4);
   1-[(4-fluorophenyl)sulfonyl]-N-[[3-(phenylmethoxy)phenyl]methyl]-pyrrolidine-2-carboxamide (CAS Registry No. 1276851-87-6);
   1-[(4-fluorophenyl)sulfonyl]-N-[[3-fluoro-4-(3-pyridinyloxy)phenyl]methyl]-pyrrolidine-2-carboxamide (CAS Registry No. 1276808-08-2);
   N-[[3-methoxy-4-(4-pyridinylmethoxy)phenyl]methyl]-1-(phenylsulfonyl)-pyrrolidine-2-carboxamide (CAS Registry No. 1276804-74-0);
   (2S)-1-[(4-methylphenyl)sulfonyl]-N-[1-[3-(2-pyridinylmethoxy)phenyl]ethyl]-pyrrolidine-2-carboxamide (CAS Registry No. 1276564-46-5);
   1-[(3,4-dimethoxyphenyl)sulfonyl]-N-[(3-phenoxyphenyl)methyl]-pyrrolidine-2-carboxamide (CAS Registry No. 1266469-81-1);
   N-[1-[3-methoxy-4-(4-pyridinylmethoxy)phenyl]ethyl]-1-(phenylsulfonyl)-pyrrolidine-2-carboxamide (CAS Registry No. 1219402-61-5);
   2-[(3,4-dimethoxyphenyl)sulfonyl]-1,2,3,4-tetrahydro-N-[(3-phenoxyphenyl)methyl]-isoquinoline-3-carboxamide (CAS Registry No. 1031746-60-7);
   (2S)-1-[(3,5-dimethyl-4-isoxazolyl)sulfonyl]-N-[[4-(4-fluorophenoxy)phenyl]methyl]-pyrrolidine-2-carboxamide (CAS Registry No. 956570-89-1);
   1,2,3,4-tetrahydro-2-[(4-methylphenyl)sulfonyl]-N-[(3-phenoxyphenyl)methyl]-isoquinoline-3-carboxamide (CAS Registry No. 475041-47-5);
   (2S)-N-[[3-methoxy-4-(4-pyridylmethoxy)phenyl]methyl]-1-(p-tolylsulfonyl)pyrrolidine-2-carboxamide (CAS Registry No. 1302762-37-3);
   (2S)-N-[(4-benzyloxy-3-methoxy-phenyl)methyl]-1-(p-tolylsulfonyl)pyrrolidine-2-carboxamide (CAS Registry No. 1297742-69-8);
   (2S)-1-(4-fluorophenyl)sulfonyl-N-[[3-(2-pyridylmethoxy)phenyl]methyl]pyrrolidine-2-carboxamide (CAS Registry No. 1294209-43-0);
   (2S)-N-[[6-(2-ethoxyphenoxy)-3-pyridyl]methyl]-1-(p-tolylsulfonyl)pyrrolidine-2-carboxamide (CAS Registry No. 1277701-32-2);
   N-[[6-(2,5-dimethylphenoxy)-3-pyridyl]methyl]-1-(4-fluorophenyl)sulfonyl-pyrrolidine-2-carboxamide (CAS Registry No. 1277269-26-7); and
   N-[[6-(4-fluorophenoxy)-3-pyridyl]methyl]-1-(4-fluorophenyl)sulfonyl-pyrrolidine-2-carboxamide (CAS Registry No. 1276767-79-3).
[15] The compound of the above-mentioned [14], wherein Y and/or Z are/is a single bond, or a pharmaceutically acceptable salt thereof.
[16] The compound of the above-mentioned [14] or [15], wherein, in the aforementioned formula (IA),
   Ar is a phenyl group optionally having 1 or 2 substituents or a 5- or 6-membered monocyclic heteroaryl group optionally having 1 or 2 substituents, or a pharmaceutically acceptable salt thereof.
[17] The compound of any of the above-mentioned [14] - [16], wherein, in the aforementioned formula (IA),
   the partial structure (1) is either a phenylene group optionally having 1 - 3 substituents, or a divalent group of a pyridine ring optionally having 1 - 3 substituents, or a pharmaceutically acceptable salt thereof.
[18] The compound of any of the above-mentioned [14] - [16], wherein, in the aforementioned formula (IA),
   the partial structure (1) is 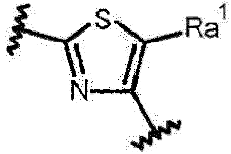 in the above-mentioned structure, Ra¹ is a hydrogen atom or a substituent,
   or a pharmaceutically acceptable salt thereof.
[19] The compound of any of the above-mentioned [14] - [18], wherein, in the aforementioned formula (IA),
   the partial structure (2) is a phenyl group optionally having 1 - 3 substituents, or a pyridyl group, a quinolyl group or an isoquinolyl group, each of which optionally has 1 - 3 substituents, or a pharmaceutically acceptable salt thereof.
[20] The compound of any of the above-mentioned [14] - [19], wherein, in the aforementioned formula (IA),
   n=0, or a pharmaceutically acceptable salt thereof.
[21] The compound of any of the above-mentioned [14] - [20], wherein, in the aforementioned formula (IA),
   R² and R³ are each a hydrogen atom, or a pharmaceutically acceptable salt thereof.
[22] The compound of any of the above-mentioned [14] - [21], wherein, in the aforementioned formula (IA),
   the partial structure (2) is a phenyl group optionally having a substituent or a pyridyl group optionally having a substituent, or a pharmaceutically acceptable salt thereof.
[23] The compound represented by the formula (I) or a pharmaceutically acceptable salt thereof for the prophylaxis and/or treatment of a disease involving TRPA1.
[24] The compound of the above-mentioned [23], or a pharmaceutically acceptable salt thereof, wherein the disease involving TRPA1 is selected from the group consisting of pain associated diseases, inflammatory diseases, digestive tract diseases, lung diseases, bladder diseases, dermatic diseases, and neurological diseases.
[25] The compound of the above-mentioned [23], or a pharmaceutically acceptable salt thereof, wherein the disease involving TRPA1 is selected from the group consisting of chronic pain, acute pain, asthma, chronic obstructive pulmonary diseases, functional gastrointestinal disorder, erosive esophagitis, inflammatory bowel disease, and pruritus.
[26] A method for the prophylaxis and/or treatment of a disease involving TRPA1, comprising administering an effective amount of a compound represented by the formula (I) or a pharmaceutically acceptable salt thereof to a patient in need thereof.
[27] The method of the above-mentioned [26], wherein the disease involving TRPA1 is selected from the group consisting of pain associated diseases, inflammatory diseases, digestive tract diseases, lung diseases, bladder diseases, dermatic diseases, and neurological diseases.
[28] The method of the above-mentioned [26], wherein the disease involving TRPA1 is selected from chronic pain, acute pain, asthma, chronic obstructive pulmonary disease, functional gastrointestinal disorder, erosive esophagitis, inflammatory bowel disease, and pruritus.

### Effect of the Invention

The compound of the present invention has a superior TRPA1 antagonist activity, and useful for the prophylaxis and/or treatment of diseases involving TRPA1 (e.g., pain associated diseases, digestive tract diseases, lung diseases, bladder diseases, inflammatory diseases, dermatic diseases, and neurological diseases).

### Description of Embodiments

The terms used in the present specification are defined below.

The "TRPA1 antagonist activity" refers to an activity capable of inhibiting activation of TRPA1, or down-regulating the biological activity of TRPA1 (e.g., intracellular inflow of ion). The TRPA1 antagonist activity can be evaluated by measuring the level of intracellular inflow of calcium ion into the cell expressing TRPA1.

The "halogen atom" is a fluorine atom, a chlorine atom, a bromine atom or an iodine atom.

The "halogeno group" is fluoro, chloro, bromo or iodo.

The "C₁₋₆ alkyl group" means a straight chain or branched alkyl group having 1 - 6 carbon atoms and, specifically, groups such as methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned.

The "C₂₋₆ alkenyl group" means a straight chain or branched alkenyl group having 2 - 6 carbon atoms and, specifically, groups such as vinyl, allyl, propenyl, butenyl, pentenyl, hexenyl, heptenyl, butadienyl, hexatrienyl, each isomer thereof and the like can be mentioned.

The "C₂₋₆ alkynyl group" means a straight chain or branched alkynyl group having 2 - 6 carbon atoms and, specifically, groups such as ethynyl, propynyl, butynyl, pentynyl, hexynyl, each isomer thereof and the like can be mentioned.

The "C₁₋₆ alkoxy group" means a straight chain or branched alkoxy group having 1 - 6 carbon atoms and, specifically, groups such as methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy and the like can be mentioned.

As the "cyclic C₃₋₆ alkyl group", specifically, groups such as cyclopropyl, cyclobutyl, cyclopentyl, cyclohexyl and the like can be mentioned.

The "cyclic C₃₋₆ alkyl group (optionally containing a hetero atom in the ring)" means the above-mentioned cyclic C₃₋₆ alkyl group, or a C₃₋₅ cyclic alkyl group containing at least one hetero atom and, specifically, those exemplified as the above-mentioned "cyclic C₃₋₆ alkyl group", as well as groups such as tetrahydrofuranyl, tetrahydropyranyl, aziridinyl, azetidinyl, pyrrolidinyl, piperidinyl, morpholinyl and the like can be mentioned.

The "halogeno C₁₋₆ alkyl group" and "halogeno C₁₋₆ alkoxy group" mean a C₁₋₆ alkyl group and a C₁₋₆ alkoxy group, respectively, each of which is substituted by one or more halogeno groups. As the "halogeno C₁₋₆ alkyl group", specifically, groups such as monofluoromethyl, difluoromethyl, trifluoromethyl, monofluoroethyl, difluoroethyl, trifluoroethyl, chloromethyl, chloroethyl, dichloroethyl, each isomer thereof and the like can be mentioned. The "halogeno C₁₋₆ alkoxy group" specifically means a C₁₋₆ alkoxy group substituted by one or more halogeno groups and, specifically, groups such as monofluoromethoxy, difluoromethoxy, trifluoromethoxy, monofluoroethoxy, difluoroethoxy, trifluoroethoxy, chloromethoxy, chloroethoxy, dichloroethoxy, each isomer thereof and the like can be mentioned.

As the "C₁₋₆ alkyl group substituted by a hydroxyl group", specifically, groups such as hydroxymethyl, 1-hydroxyethyl, 2-hydroxyethyl, 1-hydroxypropyl, 2-hydroxypropyl, 3-hydroxypropyl, 4-hydroxybutyl and the like can be mentioned.

As the "C₁₋₆ alkyl group substituted by an amino group", specifically, groups such as aminomethyl, 2-aminoethyl and the like can be mentioned.

As the "amino group mono- or di-substituted by C₁₋₆ alkyl group", specifically, an amino groups monosubstituted by C₁₋₆ alkyl such as methylamino, ethylamino, n-propylamino, isopropylamino, n-butylamino, isobutylamino, tert-butylamino, n-pentylamino, isopentylamino, hexylamino and the like; and amino groups disubstituted by a C₁₋₆ alkyl group such as dimethylamino, diethylamino, di-n-propylamino, methylethylamino, methylpropylamino, ethylpropylamino and the like can be mentioned.

As the "C₁₋₆ alkyl group substituted by an amino group mono- or di-substituted by a C₁₋₆ alkyl group", specifically, groups such as methylaminomethyl, dimethylaminomethyl, 2-methylaminoethyl, 2-dimethylaminoethyl and the like can be mentioned.

As the "C₁₋₆ alkyl group substituted by a cyclic C₃₋₆ alkyl group", specifically, groups such as cyclopropylmethyl, cyclobutylmethyl, cyclopentylmethyl, cyclohexylmethyl, cyclopropylethyl, cyclopropylpropyl and the like can be mentioned.

The "C₆₋₁₀ aryl group" means an aryl group having 6 - 10 carbon atoms and, specifically, groups such as phenyl, naphthyl and the like can be mentioned.

The "C₁₋₉ heteroaryl group" refers to a 5- to 10-membered monocyclic - bicyclic heteroaryl group having 1 - 9 carbon atoms and one or more hetero atoms selected from an oxygen atom, a nitrogen atom and a sulfur atom. Specifically, for example, 5- or 6-membered monocyclic heteroaryl groups such as pyridyl, pyridazinyl, pyrimidinyl, pyrazinyl, furyl, thiophenyl, pyrrolyl, pyrazolyl, imidazolyl, triazolyl, tetrazolyl, oxazolyl, isoxazolyl, thiazolyl, isothiazolyl, oxadiazolyl, thiadiazolyl and the like; bicyclic heteroaryl groups such as benzothiophenyl, indolyl, isoindolyl, benzoxazolyl, benzothiazolyl, benzimidazolyl, indazolyl, benzisoxazolyl, benzisothiazolyl, benzoxadiazolyl, benzothiadiazolyl, purinyl, quinolinyl, isoquinolinyl, cinnolinyl, phthalazinyl, quinazolinyl, quinoxalinyl, pteridinyl, imidazooxazolyl, imidazothiazolyl, imidazoimidazolyl and the like can be mentioned. Preferred is a 5- or 6-membered monocyclic heteroaryl group.

As the substituent that the "C₆₋₁₀ aryl group" and "C₁₋₉ heteroaryl group" optionally have, [substituent group A] below can be mentioned.

### [substituent group A]

(1) halogen atom,
(2) hydroxy group,
(3) cyano group,
(4) nitro group,
(5) carboxyl group,
(6) alkyl group (e.g., methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl),
(7) alkenyl group (e.g., vinyl, allyl, propenyl, butenyl, pentenyl, hexenyl, heptenyl, butadienyl, hexatrienyl, each isomer thereof),
(8) alkynyl group (e.g., ethynyl, propynyl, butynyl, pentynyl, hexynyl, and each isomer thereof),
(9) halogenoalkyl group (e.g., monofluoromethyl, difluoromethyl, trifluoromethyl, monofluoroethyl, difluoroethyl, trifluoroethyl, chloromethyl, chloroethyl, dichloroethyl, each isomer thereof),
(10) cyclic alkyl group (optionally containing a hetero atom in the ring) (e.g., cyclopropyl, cyclobutyl, cyclopentyl, cyclohexyl, tetrahydrofuranyl, tetrahydropyranyl, aziridinyl, azetidinyl, pyrrolidinyl, piperidinyl, morpholinyl),
(11) aryl group (e.g., phenyl, naphthyl),
(12) heteroaryl group (e.g., pyridyl, pyridazinyl, pyrimidinyl, pyrazinyl, furyl, thiophenyl, pyrrolyl, pyrazolyl, imidazolyl, triazolyl, tetrazolyl, oxazolyl, isoxazolyl, thiazolyl, isothiazolyl, oxadiazolyl, thiadiazolyl, benzofuryl, benzothiophenyl, indolyl, isoindolyl, benzoxazolyl, benzothiazolyl, benzimidazolyl, indazolyl, benzisoxazolyl, benzisothiazolyl, benzoxadiazolyl, benzothiadiazolyl, purinyl, quinolinyl, isoquinolinyl, cinnolinyl, phthalazinyl, quinazolinyl, quinoxalinyl, pteridinyl, imidazooxazolyl, imidazothiazolyl, imidazoimidazolyl),
(13) alkoxy group (e.g., methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy),
(14) alkylthio group (e.g., methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio),
(15) alkoxy group (same as in the above-mentioned (13)) substituted by aryl group (same as in the above-mentioned (11)),
(16) alkylthio group (same as in the above-mentioned (14)) substituted by aryl group (same as in the above-mentioned (11)),
(17) alkoxy group (same as in the above-mentioned (13)) substituted by heteroaryl group (same as in the above-mentioned (12)),
(18) alkylthio group (same as in the above-mentioned (14)) substituted by heteroaryl group (same as in the above-mentioned (12)),
(19) cyclic alkyl(optionally containing a hetero atom in the ring)oxy group (e.g., cyclopropyloxy, cyclobutyloxy, cyclopentyloxy, cyclohexyloxy, tetrahydrofuranyloxy, tetrahydropyranyloxy, aziridinyloxy, azetidinyloxy, pyrrolidinyloxy, piperidinyloxy, morpholinyloxy),
(20) aryloxy group (e.g., group wherein aryl group (same as in the above-mentioned (11)) is bonded to oxygen atom),
(21) heteroaryloxy group (e.g., group wherein heteroaryl group (same as in the above-mentioned (12)) is bonded to oxygen atom),
(22) halogenoalkoxy group (e.g., group wherein halogenoalkyl group (same as in the above-mentioned (9)) is bonded to oxygen atom),
(23) halogenoalkylthio group (e.g., group wherein halogenoalkyl group (same as in the above-mentioned (9)) is bonded to sulfur atom),
(24) alkoxy group (same as in the above-mentioned (13)) substituted by hydroxy group,
(25) alkoxy group (same as in the above-mentioned (13)) substituted by alkoxy group (same as in the above-mentioned (13)),
(26) amino group,
(27) amino group mono- or di-substituted by alkyl group (same as in the above-mentioned (6)),
(28) carbamoyl group,
(29) carbamoyl group mono- or di-substituted by alkyl group (same as in the above-mentioned (6)) (e.g., methylcarbamoyl, ethylcarbamoyl, dimethylcarbamoyl, diethylcarbamoyl, ethylmethylcarbamoyl),
(30) sulfamoyl group,
(31) sulfamoyl group mono- or di-substituted by alkyl group (same as in the above-mentioned (6)) (e.g., methylsulfamoyl, ethylsulfamoyl, dimethylsulfamoyl, diethylsulfamoyl, ethylmethylsulfamoyl),
(32) alkanoyl group (e.g., carbonyl group wherein hydrogen atom or alkyl group (same as in the above-mentioned (6)) is bonded to carbon atom),
(33) aroyl group (e.g., carbonyl group wherein aryl group (same as in the above-mentioned (11)) is bonded to carbon atom),
(34) alkylsulfonylamino group (e.g., sulfonylamino group substituted by alkyl group (same as in the above-mentioned (6)),
(35) arylsulfonylamino group (e.g., sulfonylamino group substituted by aryl group (same as in the above-mentioned (11))),
(36) heteroaryl sulfonylamino group (e.g., sulfonylamino group substituted by heteroaryl group (same as in the above-mentioned (12))),
(37) acylamino group (e.g., an amino group substituted by acyl group),
   wherein the "acyl group" is an acyl group having a C₁₋₆ alkyl group, a cyclic C₃₋₆ alkyl group, or C₆₋₁₀ aryl group; as the C₁₋₆ alkyl group, a cyclic C₃₋₆ alkyl group and C₆₋₁₀ aryl group, those recited above can be mentioned; as the acyl group, specifically, acetyl group, propionyl group, butyroyl group, isobutyroyl group, valeroyl group, isovaleroyl group, pivaloyl group, hexanoyl group, acryloyl group, methacryloyl group, crotonoyl group, isocrotonoyl group, benzoyl group, naphthoyl group and the like can be mentioned,
(38) alkoxycarbonylamino group (e.g., carbonylamino group substituted by alkoxy group (same as in the above-mentioned (13))),
(39) alkylsulfonyl group (e.g., sulfonyl group substituted by alkyl group (same as in the above-mentioned (6))),
(40) alkylsulfinyl group (e.g., sulfinyl group substituted by alkyl group (same as in the above-mentioned (6))), (hereinafter to be also referred to as substituent group B),
(41) alkoxycarbonyl group (e.g., methoxycarbonyl group, ethoxycarbonyl group),
   and the like can be mentioned.

When two or more substituents are present, they may be the same or different.

The present invention provides a medicament composed of a compound represented by the formula (I):

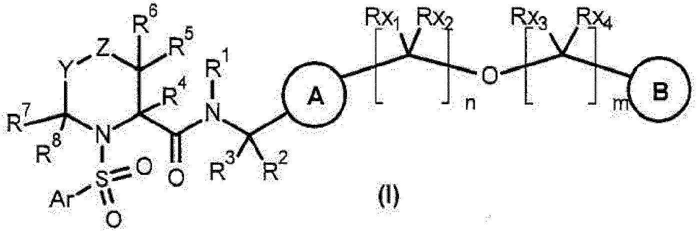

wherein each symbol is as defined above,
(hereinafter to be also referred to as compound (I)), or a pharmaceutically acceptable salt thereof.

In the formula (I), partial structure (1)

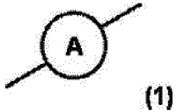

is a phenylene group optionally having 1 - 4 substituents, or a divalent group of 5-membered or 6-membered heteroaromatic ring (ring A) optionally having 1 - 3 substituents and containing any 1 - 3 hetero atoms selected from oxygen atom, nitrogen atom and sulfur atom.

Examples of the heteroaromatic ring (ring A) include rings such as pyridine, pyrimidine, pyridazine, pyrazine, triazine, furan, thiophene, pyrrole, oxazole, isoxazole, thiazole, isothiazole, imidazole, pyrazole, oxadiazole, thiadiazole, triazole, tetrazole and the like. Preferred are a pyridine ring, a pyrimidine ring, a thiazole ring, and a thiophene ring.

As the substituent that the phenylene group and the divalent group of ring A may have, those exemplified for the above-mentioned [substituent group A] can be mentioned. Preferably, a halogen atom, a hydroxy group, a C₁₋₆ alkyl group, a halogeno C₁₋₆ alkyl group, a cyclic C₃₋₆ alkyl group (optionally containing a hetero atom in the ring), a C₆₋₁₀ aryl group, a C₁₋₉ heteroaryl group, a C₁₋₆ alkoxy group, a halogeno C₁₋₆ alkoxy group, an amino group, an amino group mono- or di-substituted by a C₁₋₆ alkyl group, and the like can be mentioned. When two or more substituents are present, they may be the same or different.

As partial structure (1), preferably, a phenylene group optionally having 1 - 3 substituents (e.g., a halogen atom, a halogeno C₁₋₆ alkyl group), and a divalent group of a pyridine ring, a pyrimidine ring, a thiazole ring, a thiophene ring, which divalent group optionally having 1 - 3 substituents, and the like can be mentioned. As partial structure (1), a structure represented by the following formula

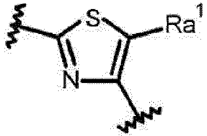

in the above-mentioned structure, Ra¹ is a hydrogen atom or a substituent, is also preferable. As used herein, as the substituent, those exemplified for the above-mentioned [substituent group A] can be mentioned. Preferably, a C₁₋₃ alkyl group, and the like can be mentioned.

In the formula (I), partial structure (2)

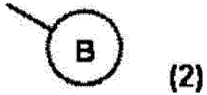

is a C₆₋₁₀ aryl group optionally having 1 - 4 substituents or a C₁₋₉ heteroaryl group optionally having 1 - 4 substituents.

As the substituent that the C₆₋₁₀ aryl group and the C₁₋₉ heteroaryl group optionally have, those exemplified for the above-mentioned [substituent group A] can be mentioned. Preferably, a halogen atom, hydroxy group, a C₁₋₆ alkyl group, a halogeno C₁₋₆ alkyl group, a cyclic C₃₋₆ alkyl group (optionally containing a hetero atom in the ring), C₆₋₁₀ aryl group, C₁₋₉ heteroaryl group, a C₁₋₆ alkoxy group, a halogeno C₁₋₆ alkoxy group, an amino group, an amino group mono- or di-substituted by a C₁₋₆ alkyl group, and the like can be mentioned. When two or more substituents are present, they may be the same or different.

In the formula (I),
Ar is a C₆₋₁₀ aryl group optionally having 1 - 4 substituents or a C₁₋₉ heteroaryl group optionally having 1 - 4 substituents. Preferably, Ar is a phenyl group optionally having 1 - 4 substituents or a C₁₋₅ heteroaryl group optionally having 1 - 4 substituents. Here, the C₆₋₁₀ aryl group is preferably a phenyl group, and the like, and the C₁₋₉ heteroaryl group is preferably a thienyl group, a furanyl group, and the like. As the substituent that the C₆₋₁₀ aryl group or C₁₋₉ heteroaryl group has, those exemplified for the above-mentioned [substituent group A] can be mentioned. Preferably, a halogen atom, and the like can be mentioned. When two or more substituents are present, they may be the same or different.

In the formula (I),
R¹ is a hydrogen atom, a C₁₋₆ alkyl group, a C₂₋₆ alkenyl group,
a C₂₋₆ alkynyl group, a halogeno C₁₋₆ alkyl group, a cyclic C₃₋₆ alkyl group, or a C₁₋₆ alkyl group substituted by a cyclic C₃₋₆ alkyl group. Preferably, it is a hydrogen atom or a C₁₋₃ alkyl group, more preferably a hydrogen atom.

In the formula (I),
R² and R³ are the same or different and each is a hydrogen atom, a C₁₋₆ alkyl group, a C₂₋₆ alkenyl group, a C₂₋₆ alkynyl group. Preferably, it is a hydrogen atom or a C₁₋₃ alkyl group, more preferably a hydrogen atom.

In the formula (I),
R⁴ - R⁸, Ry1 - Ry2, Rz1 and Rz2 are the same or different and each is a hydrogen atom, a C₁₋₆ alkyl group, a C₂₋₆ alkenyl group, a C₂₋₆ alkynyl group, a halogeno C₁₋₆ alkyl group, a C₁₋₆ alkoxy group, a hydroxyl group, a halogeno C₁₋₆ alkoxy group, an amino group, an amino group mono- or di-substituted by a C₁₋₆ alkyl group, or a halogeno group, respective R⁴ - R⁸, Ry1 - Ry2, Rz1 or Rz2 on adjacent carbon atom are optionally joined to form a double bond and/or a ring;
respective R⁵ and R⁶, R⁷ and R⁸, Ry1 and Ry2, or Rz1 and Rz2 on the same carbon atom are optionally joined to form a ring. R⁴ - R⁸, Ry1 - Ry2, Rz1 and Rz2 is preferably a hydrogen atom, a C₁₋₆ alkyl group, a hydroxyl group, an amino group mono- or di-substituted by a C₁₋₆ alkyl group, or a halogeno group, and the like. Respective R⁴ - R⁸, Ry1 - Ry2, Rz1 or Rz2 on adjacent carbon atom are preferably joined to form a double bond and/or a ring, and respective R⁵ and R⁶, R⁷ and R⁸, Ry1 and Ry2, or Rz1 and Rz2 on the same carbon atom are preferably joined to form a ring.

In the formula (I),
when respective R⁴ - R⁸, Ry1 - Ry2, Rz1 or Rz2 on the adjacent carbon atom jointly form a ring, a cyclopropane ring, a cyclobutane ring, a cyclopentane ring, a cyclohexane ring, a cyclohexene ring, a piperidine ring, a morpholine ring, a benzene ring, a pyridine ring, and the like can be mentioned.

In the formula (I),
when respective R⁵ and R⁶, R⁷ and R⁸, Ry1 and Ry2, or Rz1 and Rz2 on the same carbon atom jointly form a ring, a cyclopropane ring, a cyclobutane ring, a cyclopentane ring, a cyclohexane ring, a cyclohexene ring, a piperidine ring, an oxetane ring, a morpholine ring, and the like can be mentioned.
(1) A preferable embodiment of compound (I) is a compound of the formula (I), wherein
   Y and/or Z are/is a single bond, or a pharmaceutically acceptable salt thereof.
(2) Another preferable embodiment of compound (I) is a compound of the formula (I), wherein
   Ar is a phenyl group optionally having 1 or 2 substituents (e.g., a halogen atom), or a 5- or 6-membered monocyclic heteroaryl group (e.g., thienyl) optionally having 1 or 2 substituents (e.g., a halogen atom), or a pharmaceutically acceptable salt thereof.
(3) Another preferable embodiment of compound (I) is a compound of the formula (I), wherein
   the partial structure (1) is a phenylene group optionally having 1 - 3 (preferably 1 or 2) substituents (e.g., a halogen atom, a halogenoalkyl group), or a divalent group of a pyridine ring optionally having 1 - 3 substituents (preferably unsubstituted), or a pharmaceutically acceptable salt thereof.
(4) Another preferable embodiment of compound (I) is a compound of the formula (I), wherein
   the partial structure (1) is 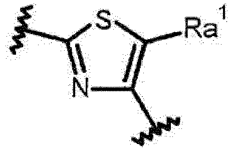 wherein Ra¹ is as defined above, or a pharmaceutically acceptable salt thereof. Ra¹ is preferably a hydrogen atom.
(5) Another preferable embodiment of compound (I) is a compound of the formula (I), wherein
   the partial structure (2) is a phenyl group optionally having 1 - 3 (preferably 1 or 2) substituents (e.g., a halogen atom, a halogeno C₁₋₆ alkyl group, a halogeno C₁₋₆ alkoxy group, a C₁₋₆ alkyl group, a C₁₋₆ alkoxy group, a C₁₋₆ alkoxycarbonyl group, a cyano group, an amino group mono- or di-substituted by a C₁₋₆ alkyl group), or a pyridyl group, a quinolyl group or an isoquinolyl group, each optionally having 1 - 3 (preferably 1 or 2) substituents (e.g., a halogen atom, a halogeno C₁₋₆ alkyl group), or a pharmaceutically acceptable salt thereof.
(6) Another preferable embodiment of compound (I) is a compound of the formula (I), wherein
   n=0, or a pharmaceutically acceptable salt thereof.
(7) Another preferable embodiment of compound (I) is a compound of the formula (I), wherein
   R² and R³ are each a hydrogen atom, or a pharmaceutically acceptable salt thereof.
(8) Another preferable embodiment of compound (I) is a compound of the formula (I), wherein
   the partial structure (2) is a phenyl group optionally having a substituent (e.g., a halogen atom, a halogeno C₁₋₆ alkyl group, a halogeno C₁₋₆ alkoxy group, a C₁₋₆ alkyl group, a C₁₋₆ alkoxy group, a C₁₋₆ alkoxycarbonyl group, a cyano group, an amino group mono- or di-substituted by a C₁₋₆ alkyl group), or a pyridyl group optionally having a substituent (e.g., a halogen atom, a halogenoalkyl group), or a pharmaceutically acceptable salt thereof.
(9) Another preferable embodiment of compound (I) includes the compounds described in Examples 6, 15, 32, 33, 34, 36, 37, 38, 95, 96, 100, 111, 112, 115, 118, 134, 135, 136, 137, 138, 141, 149, 150, 151, 160, 163, 176, 177, 178, 181, 182, 189, 190, 200, 208, 216, which are described in the following Examples, or a pharmaceutically acceptable salt thereof.

The present invention provides a compound represented by the formula (IA):

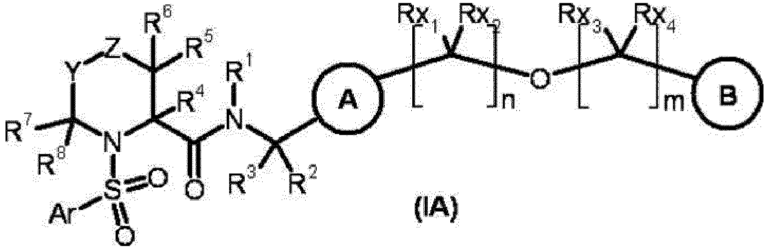

wherein each symbol is as defined above, excluding the following compounds:
N-[[2-(2-fluorophenoxy)phenyl]methyl]-1-(2-thienylsulfonyl)pyrrolidine-2-carboxamide (CAS Registry No. 1385210-42-3);
ethyl 5-[[2-[[[(2-phenoxyphenyl)methyl]amino]carbonyl]-1-pyrrolidinyl]sulfonyl]-2-furancarboxylate (CAS Registry No. 1372166-14-7);
N-[[3-fluoro-4-(3-pyridinyloxy)phenyl]methyl]-1-(phenylsulfonyl)-pyrrolidine-2-carboxamide (CAS Registry No. 1370950-04-1);
N-[(3-phenoxyphenyl)methyl]-1-(2-thienylsulfonyl)-pyrrolidine-2-carboxamide (CAS Registry No. 1356772-35-4);
N-[(2-phenoxyphenyl)methyl]-1-(2-thienylsulfonyl)-pyrrolidine-2-carboxamide (CAS Registry No. 1356571-93-1);
N-[[6-(2,5-dimethylphenoxy)-3-pyridinyl]methyl]-1-(phenylsulfonyl)-pyrrolidine-2-carboxamide (CAS Registry No. 1315966-17-6);
N-[[2-(4-fluorophenoxy)-4-pyridinyl]methyl]-1-(phenylsulfonyl)-pyrrolidine-2-carboxamide (CAS Registry No. 1315858-36-6);
N-[[6-(3-fluorophenoxy)-3-pyridinyl]methyl]-1-(phenylsulfonyl)-pyrrolidine-2-carboxamide (CAS Registry No. 1315848-80-6);
1-(phenylsulfonyl)-N-[[3-(2-pyridinylmethoxy)phenyl]methyl]-pyrrolidine-2-carboxamide (CAS Registry No. 1315835-86-9);
(2S)-1-[(4-methylphenyl)sulfonyl]-N-[[2-(phenylmethoxy)-4-pyridinyl]methyl]-pyrrolidine-2-carboxamide (CAS Registry No. 1288924-64-0);
1-[(4-fluorophenyl)sulfonyl]-N-[[6-(4-methoxyphenoxy)-3-pyridinyl]methyl]-pyrrolidine-2-carboxamide (CAS Registry No. 1277456-75-3);
1-[(4-fluorophenyl)sulfonyl]-N-[[3-methoxy-4-(3-pyridinylmethoxy)phenyl]methyl]-pyrrolidine-2-carboxamide (CAS Registry No. 1277414-27-3);
N-[1-[4-(3-pyridinylmethoxy)phenyl]ethyl]-1-(2-thienylsulfonyl)-pyrrolidine-2-carboxamide (CAS Registry No. 1277410-29-3);
N-[[2-(phenylmethoxy)phenyl]methyl]-1-(2-thienylsulfonyl)-pyrrolidine-2-carboxamide (CAS Registry No. 1277400-48-2);
1-[(4-fluorophenyl)sulfonyl]-N-[[3-methoxy-4-(phenylmethoxy)phenyl]methyl]-pyrrolidine-2-carboxamide (CAS Registry No. 1277358-57-2);
N-[[2-(phenylmethoxy)phenyl]methyl]-1-(2-thienylsulfonyl)-pyrrolidine-2-carboxamide (CAS Registry No. 1277358-06-1);
N-[[6-(4-fluorophenoxy)-3-pyridinyl]methyl]-1-(2-thienylsulfonyl)-pyrrolidine-2-carboxamide (CAS Registry No. 1277336-69-2);
1-[(4-fluorophenyl)sulfonyl]-N-[1-[3-(2-pyridinylmethoxy)phenyl]ethyl]-pyrrolidine-2-carboxamide (CAS Registry No. 1277268-08-2);
N-[[4-(phenoxymethyl)phenyl]methyl]-1-(2-thienylsulfonyl)-pyrrolidine-2-carboxamide (CAS Registry No. 1277253-49-2);
N-[[3-(phenylmethoxy)phenyl]methyl]-1-(2-thienylsulfonyl)-pyrrolidine-2-carboxamide (CAS Registry No. 1277146-58-3);
1-[(4-fluorophenyl)sulfonyl]-N-[[4-(3-pyridinylmethoxy)phenyl]methyl]-pyrrolidine-2-carboxamide (CAS Registry No. 1277055-81-8);
1-[(4-fluorophenyl)sulfonyl]-N-[[2-(phenylmethoxy)-4-pyridinyl]methyl]-pyrrolidine-2-carboxamide (CAS Registry No. 1277032-72-0);
N-[[3-(2-pyridinylmethoxy)phenyl]methyl]-1-[(2,4,6-trimethylphenyl)sulfonyl]-pyrrolidine-2-carboxamide (CAS Registry No. 1276991-71-9);
1-(phenylsulfonyl)-N-[[3-(2-pyridinylmethoxy)phenyl]methyl]-pyrrolidine-2-carboxamide (CAS Registry No. 1276938-18-1);
N-[[2-(phenylmethoxy)-4-pyridinyl]methyl]-1-(2-thienylsulfonyl)-pyrrolidine-2-carboxamide (CAS Registry No. 1276907-17-5);
N-[[6-(2,5-dimethylphenoxy)-3-pyridinyl]methyl]-1-(2-thienylsulfonyl)-2-piperidinecarboxamide (CAS Registry No. 1276905-54-4);
N-[[2-(phenoxymethyl)phenyl]methyl]-1-(2-thienylsulfonyl)-pyrrolidine-2-carboxamide (CAS Registry No. 1276856-98-4);
1-[(4-fluorophenyl)sulfonyl]-N-[[3-(phenylmethoxy)phenyl]methyl]-pyrrolidine-2-carboxamide (CAS Registry No. 1276851-87-6);
1-[(4-fluorophenyl)sulfonyl]-N-[[3-fluoro-4-(3-pyridinyloxy)phenyl]methyl]-pyrrolidine-2-carboxamide (CAS Registry No. 1276808-08-2);
N-[[3-methoxy-4-(4-pyridinylmethoxy)phenyl]methyl]-1-(phenylsulfonyl)-pyrrolidine-2-carboxamide (CAS Registry No. 1276804-74-0);
(2S)-1-[(4-methylphenyl)sulfonyl]-N-[1-[3-(2-pyridinylmethoxy)phenyl]ethyl]-pyrrolidine-2-carboxamide (CAS Registry No. 1276564-46-5);
1-[(3,4-dimethoxyphenyl)sulfonyl]-N-[(3-phenoxyphenyl)methyl]-pyrrolidine-2-carboxamide (CAS Registry No. 1266469-81-1);
N-[1-[3-methoxy-4-(4-pyridinylmethoxy)phenyl]ethyl]-1-(phenylsulfonyl)-pyrrolidine-2-carboxamide (CAS Registry No. 1219402-61-5);
2-[(3,4-dimethoxyphenyl)sulfonyl]-1,2,3,4-tetrahydro-N-[(3-phenoxyphenyl)methyl]-isoquinoline-3-carboxamide (CAS Registry No. 1031746-60-7);
(2S)-1-[(3,5-dimethyl-4-isoxazolyl)sulfonyl]-N-[[4-(4-fluorophenoxy)phenyl]methyl]-pyrrolidine-2-carboxamide (CAS Registry No. 956570-89-1);
1,2,3,4-tetrahydro-2-[(4-methylphenyl)sulfonyl]-N-[(3-phenoxyphenyl)methyl]-isoquinoline-3-carboxamide (CAS Registry No. 475041-47-5);
(2S)-N-[[3-methoxy-4-(4-pyridylmethoxy)phenyl]methyl]-1-(p-tolylsulfonyl)pyrrolidine-2-carboxamide (CAS Registry No. 1302762-37-3);
(2S)-N-[(4-benzyloxy-3-methoxy-phenyl)methyl]-1-(p-tolylsulfonyl)pyrrolidine-2-carboxamide (CAS Registry No. 1297742-69-8);
(2S)-1-(4-fluorophenyl)sulfonyl-N-[[3-(2-pyridylmethoxy)phenyl]methyl]pyrrolidine-2-carboxamide (CAS Registry No. 1294209-43-0);
(2S)-N-[[6-(2-ethoxyphenoxy)-3-pyridyl]methyl]-1-(p-tolylsulfonyl)pyrrolidine-2-carboxamide (CAS Registry No. 1277701-32-2);
N-[[6-(2,5-dimethylphenoxy)-3-pyridyl]methyl]-1-(4-fluorophenyl)sulfonyl-pyrrolidine-2-carboxamide (CAS Registry No. 1277269-26-7); and
N-[[6-(4-fluorophenoxy)-3-pyridyl]methyl]-1-(4-fluorophenyl)sulfonyl-pyrrolidine-2-carboxamide (CAS Registry No. 1276767-79-3)

hereinafter to be also referred to as compound (IA), or a novel compound which is a pharmaceutically acceptable salt thereof.

Preferable embodiments of compound (IA) are according to the preferable embodiments of the above-mentioned compound (I).

Compound (I) and compound (IA) are sometimes generically referred to as the compound of the present invention.

When the compound of the present invention can form a salt, the salt only needs to be pharmaceutically acceptable. For example, when an acidic group such as a carboxyl group and the like is present in the formula, ammonium salt, salts with alkali metal such as sodium, potassium and the like, salts with alkaline earth metal such as calcium, magnesium and the like, aluminum salt, zinc salt, salts with organic amine such as triethylamine, ethanolamine, morpholine, piperidine, dicyclohexylamine and the like, and salts with basic amino acid such as arginine, lysine and the like can be mentioned with regard to the acidic group. When a basic group is present in the formula, salts with inorganic acid such as hydrochloric acid, sulfuric acid, phosphoric acid, nitric acid, hydrobromic acid and the like, salts with organic carboxylic acid such as acetic acid, trifluoroacetic acid, citric acid, benzoic acid, maleic acid, fumaric acid, tartaric acid, succinic acid, tannic acid, butyric acid, hibenzoic acid, pamoic acid, enanthic acid, decanoic acid, teoclic acid, salicylic acid, lactic acid, oxalic acid, mandelic acid, malic acid and the like, and salts with organic sulfonic acid such as methanesulfonic acid, benzenesulfonic acid, p-toluenesulfonic acid and the like can be mentioned with regard to the basic group. As a method for forming a salt, the compound of the present invention and necessary acid or base are mixed at a suitable quantitative ratio in a solvent or a dispersing agent, or cation exchange or anion exchange of other salt form is employed.

The compound of the present invention also encompasses optical isomer, stereoisomer, tautomer, rotamer, and mixtures thereof at optional ratios. These can be obtained each as a single product according to a synthesis method and separation method known per se. For example, an optical isomer can be obtained by using an optically active synthetic intermediate or by optically resolving a racemate of a synthetic intermediate or final product by a conventional method.

Furthermore, it also encompasses a stable isotope and a radioactive isotope.

The compound of the present invention also includes solvates of the compound such as hydrate, alcohol adduct and the like.

The compound of the present invention can also be converted to a prodrug. The prodrug in the present invention is a compound that is converted in the body to produce the compound of the present invention. For example, when the active component contains a carboxyl group or a phosphate group, an ester, amide and the like thereof can be mentioned. When the active component contains an amino group, an amide, carbamate and the like thereof can be mentioned. When the active component contains a hydroxyl group, an ester, carbonate, carbamate and the like thereof can be mentioned. When the compound of the present invention is converted to a prodrug, it may be bonded to an amino acid or saccharides.

The present invention also encompasses a metabolite of the compound of the present invention. The metabolite of the compound of present invention means a compound resulting from the conversion of the compound of the present invention by a metabolic enzyme and the like in the body. For example, a compound wherein a hydroxyl group is introduced on the benzene ring of the compound of the present invention due to the metabolism, a compound wherein glucuronic acid, glucose or amino acid is bonded to the carboxylic acid moiety of the compound of the present invention or a hydroxyl group added by the metabolism, and the like can be mentioned.

The compound of the present invention has a superior TRPA1 antagonist activity for mammals such as human, bovine, horse, dog, mouse, rat and the like, and can be used as a medicament, which is administered as it is or as a pharmaceutical composition containing the same mixed with a pharmaceutically acceptable carrier according to a method known per se. While oral administration is generally preferable, parenteral administration (e.g., routes such as intravenous, subcutaneous, intramuscular, suppository, enema, ointment, patch, sublingual, eye drop, inhalation administrations and the like) can also be employed. While the dose used for the above-mentioned objects is determined according to the desired treatment effect, administration method, duration of treatment, age, body weight and the like, a daily dose of 1 µg - 10 g for oral administration and 0.01 µg - 1 g for parenteral administration is used, which is generally administered to an adult by an oral or parenteral route in one to several portions per day. In addition, the content of the compound of the present invention in the above-mentioned pharmaceutical composition is about 0.01 wt% - 100 wt% of the whole composition.

Examples of the pharmaceutically acceptable carrier for the pharmaceutical composition of the present invention include various organic or inorganic carrier substances conventionally used as preparation materials. For example, excipient, lubricant, binder, disintegrant, water-soluble polymer and basic inorganic salt in solid preparation; solvent, solubilizing agents, suspending agent, isotonicity agent, buffering agent and soothing agent in liquid preparation, and the like can be mentioned. Where necessary, general additives such as preservative, antioxidant, colorant, sweetening agent, souring agent, foaming agent, flavor and the like can also be used.

The dosage form of such pharmaceutical composition may be tablet, powder, pill, granule, capsule, suppository, solution, sugar-coated agent, depot, syrup, suspension, emulsion, troche, sublingual agent, adhesive preparation, oral disintegrant (tablet), inhalant, enema, ointment, patch, tape and eye drop, and these can be produced using conventional formulation auxiliaries and according to a conventional method.

The pharmaceutical composition of the present invention can be produced according to a method conventionally used in the technical field of pharmaceutical formulation, for example, the method described in the Japanese Pharmacopoeia and the like. Specific production methods of the preparation are explained in detail in the following.

For example, when the compound of the present invention is prepared as an oral preparation, excipient and, where necessary, binder, disintegrant, lubricant, colorant, flavoring agent and the like are further added and the mixture is processed to give, for example, tablet, powder, pill, granule, capsule, suppository, solution, sugar-coated agent, depot, syrup and the like according to a conventional method. Examples of the excipient include lactose, cornstarch, sucrose, glucose, sorbitol, crystalline cellulose and the like. Examples of the binder include polyvinyl alcohol, polyvinyl ether, ethylcellulose, methylcellulose, gum arabic, tragacanth, gelatin, shellac, hydroxypropylcellulose, hydroxypropylstarch, polyvinylpyrrolidone and the like. Examples of the disintegrant include starch, agar, gelatin powder, crystalline cellulose, calcium carbonate, sodium hydrogen carbonate, calcium citrate, dextran, pectin and the like. Examples of the lubricant include magnesium stearate, talc, polyethylene glycol, silica, hydrogenated vegetable oil and the like. As the colorant, one allowed to add to a pharmaceutical product is used, and as the flavoring agent, cocoa powder, menthol, aromatic acid, peppermint oil, borneol, powdered cinnamon bark and the like are used. Where necessary, these tablets and granules are applied with a coating as appropriate such as sugar coating, gelatin coating, and the like.

When an injection is to be prepared, pH adjuster, buffering agent, stabilizer, preservative and the like are added where necessary and the mixture is processed to give subcutaneous, intramuscular or intravenous injection according to a conventional method.

As mentioned above, since the compound of the present invention shows a superior TRPA1 antagonist activity for mammals (e.g., mouse, rat, hamster, rabbit, cat, dog, swine, bovine, sheep, horse, monkey, human etc., preferably human), it is useful as a TRPA1 antagonist. Moreover, the compound of the present invention is useful for the prophylaxis and/or treatment of diseases involving TRPA1, and the compound of the present invention can be provided as a medicament for the prophylaxis and/or treatment of such diseases.

As the disease involving TRPA1, pain associated disease, digestive tract diseases, lung disease, bladder disease, inflammatory disease, dermatic diseases, and neurological disease and the like can be mentioned.

As the pain-associated disease, specifically, chronic pain, neuropathic pain, inflammatory pain, postherpetic neuralgia, neuropathy, neuralgia, diabetic neuropathy, HIV related neuropathy, nerve damage, rheumatoid arthritis pain, osteoarthritis pain, back pain, carcinomatous pain, toothache, headache, migraine, carpal-tunnel syndrome, fibromyalgia syndrome, neuritis, sciatic neuralgia, pelvic hypersensitivity, pelvic pain, menstrual pain, organ pain, pain after operation and the like can be mentioned.

As the digestive tract disease, functional gastrointestinal disorder {dysphagia, functional dyspepsia (FD), irritable bowel syndrome (IBS)}, erosive esophagitis (GERD), ulcer, inflammatory bowel disease (IBD), vomiting (cancer chemotherapy-induced vomiting), pancreatitis and the like can be mentioned.

As the lung disease, asthma, chronic obstructive pulmonary diseases (COPD), bronchoconstriction and the like can be mentioned.

As the bladder disease, overactive bladder, abnormal urination, cystitis and the like can be mentioned.

As the inflammatory disease, burn, osteoarthritis and the like can be mentioned.

As the dermatic disease, atopic dermatitis, pruritus and the like can be mentioned.

As the neurological disease, anticancer agent-induced neuropathy and the like can be mentioned.

As the disease involving TRPA1, preferably, chronic pain, acute pain, asthma, chronic obstructive pulmonary diseases, functional gastrointestinal disorder, erosive esophagitis, inflammatory bowel disease, pruritus and the like can be mentioned.

The production methods of the representative compounds among the compounds of the present invention are shown below. Each symbol in the drawings is as defined above. Synthesis Method 1: In the formula (I) (and the formula (IA)), n and m are each 0 (synthesis of compound S7)

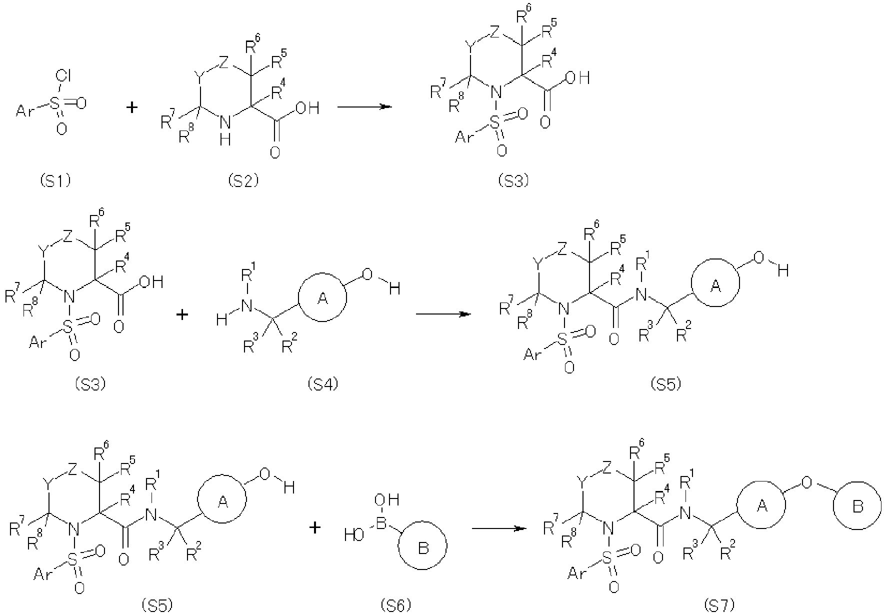

Sulfoneamide derivative (S3) can be synthesized by reacting sulfonyl chloride (S1) and amine derivative (S2) in a solvent that does not adversely influence the reaction such as tetrahydrofuran, a mixed solvent of water and the like in the presence of a base such as sodium hydroxide and the like. Amide derivative (S5) can be synthesized by reacting carboxylic acid derivative (S3) and amine derivative (S4) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like. The object compound (S7) can be produced by reacting amide derivative (S5) and boronic acid derivative (S6) in a solvent that does not adversely influence the reaction such as dichloromethane, dichloroethane and the like in the presence or absence of a base such as triethylamine, N-ethyldiisopropylamine or pyridine and the like, in the presence or absence of molecular sieves 4Å and the like by using copper acetate(I) and the like.

Here, amine derivative (S4) can be synthesized as follows.

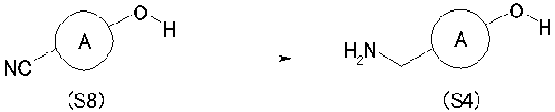

Amine derivative (S4) can be synthesized by reacting nitrile derivative (S8) having alcohol protected or not protected by an appropriate protecting group, in a solvent that does not adversely influence the reaction such as tetrahydrofuran and the like by using lithium aluminum hydride, borane tetrahydrofuran complex and the like. Alternatively, amine derivative (S4) can also be synthesized by reducing nitrile derivative (S8) in a solvent that does not adversely influence the reaction such as water, methanol, ethanol, tetrahydrofuran and the like in the presence of a catalyst such as palladium/carbon, palladium hydroxide, platinum/carbon and the like in the presence or absence of an acid such as acetic acid, hydrochloric acid and the like, under a hydrogen atmosphere at normal pressure or under pressurization.

Compound (S7) can also be synthesized as follows.

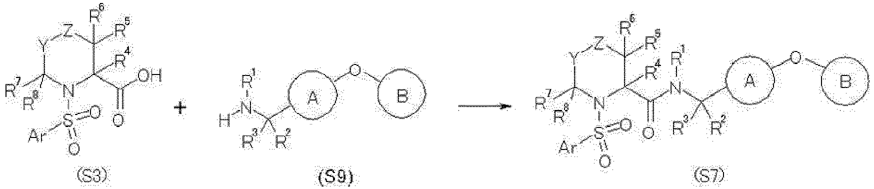

The object compound (S7) can be produced by reacting carboxylic acid derivative (S3) and amine derivative (S9) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like.

Here, amine derivative (S9) can be synthesized as follows.

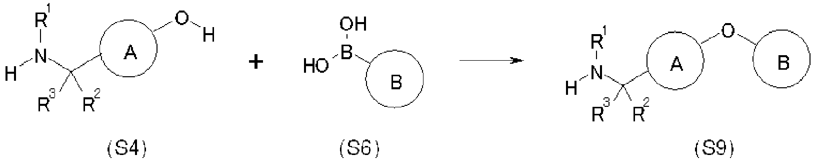

Amine derivative (S9) can be synthesized by reacting amine derivative (S4) and boronic acid derivative (S6) in a solvent that does not adversely influence the reaction such as dichloromethane, dichloroethane and the like in the presence or absence of a base such as triethylamine, N-ethyldiisopropylamine, pyridine and the like in the presence or absence of molecular sieves 4Å and the like by using copper acetate(I) and the like.

Amine derivative (S9) can also be synthesized as follows.

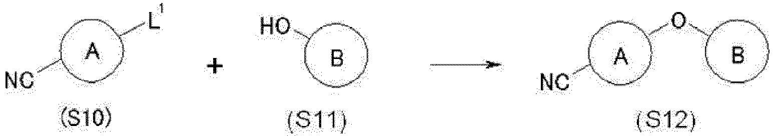

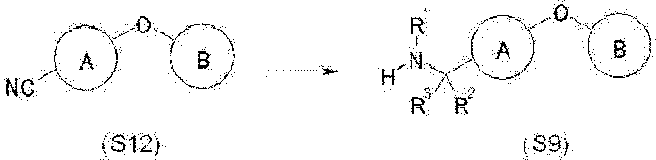

wherein L¹ is a suitable leaving group such as a fluorine atom, a chlorine atom and the like.

Nitrile derivative (S12) can be synthesized by heating nitrile derivative (S10) and alcohol derivative (S11) in a solvent that does not adversely influence the reaction such as tetrahydrofuran, N,N-dimethylformamide and the like in the presence of a base such as sodium hydride, potassium carbonate, cesium carbonate, triethylamine or N-ethyldiisopropylamine and the like. Amine derivative (S9) can be synthesized by reducing the nitrile group of nitrile derivative (S12) with lithium aluminum hydride, borane tetrahydrofuran complex and the like in a solvent that does not adversely influence the reaction such as tetrahydrofuran, diethyl ether and the like. Amine derivative (S9) can also be synthesized by reducing nitrile derivative (S12) in a solvent that does not adversely influence the reaction such as water, methanol, ethanol, tetrahydrofuran and the like in the presence of a catalyst such as palladium/carbon, palladium hydroxide, platinum/carbon and the like in the presence or absence of an acid such as acetic acid,hydrochloric acid and the like under a hydrogen atmosphere at normal pressure or under pressurization.

Compound (S7) can also be synthesized as follows.

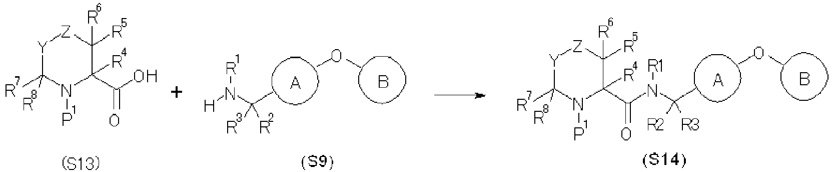

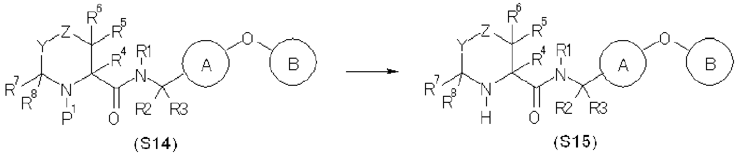

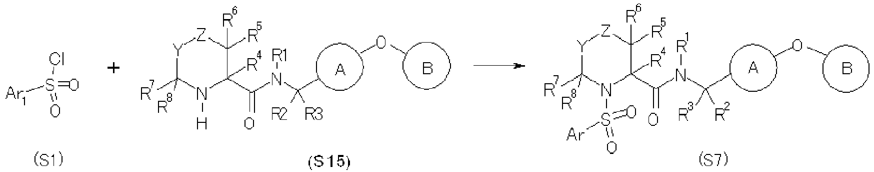

wherein P¹ is a suitable protecting group such as tert-butoxycarbonyl group (Boc group), benzyloxycarbonyl group (Cbz group) and the like.

Amide derivative (S14) can be synthesized by reacting carboxylic acid derivative (S13) and amine derivative (S9) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like. The object compound (S7) can be produced by sulfonating amine derivative (S15) obtained by removing the protecting group P¹ of amide derivative (S14) with sulfonylchloride (S1) in a solvent that does not adversely influence the reaction such as dichloromethane and the like in the presence of a base such as triethylamine and the like.

The deprotection reaction is known and, for example, when P¹ is a tert-butoxycarbonyl group, a method using a proton acid such as hydrochloric acid and trifluoroacetic acid and a method using a Lewis acid such as boron trifluoride and tin tetrachloride can be mentioned. For example, when P¹ is a benzyloxycarbonyl group, a method using a hydrogenation reaction in the presence of a catalytic amount of palladium/carbon and the like under a hydrogen atmosphere at normal pressure or under pressurization, a method using hydrobromic acid/acetic acid and the like can be mentioned.

### Synthesis Method 2: In the formula (I) (and the formula (IA)) wherein n is 1 and m is 0 (synthesis of compound S18)

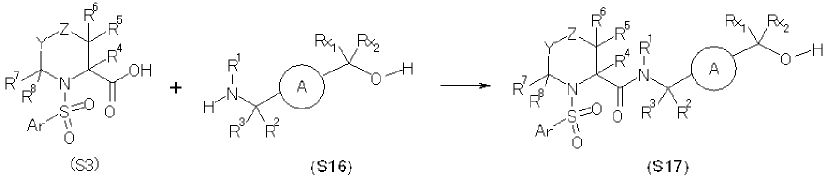

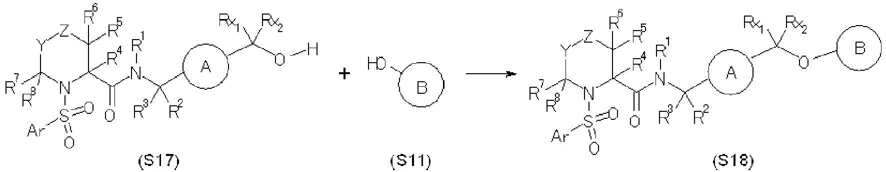

Alcohol derivative (S17) can be synthesized by reacting carboxylic acid derivative (S3) and amine derivative (S16) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like. The object compound (S18) can be produced by reacting alcohol derivative (S17) and alcohol derivative (S11) in a solvent that does not adversely influence the reaction such as dichloromethane, tetrahydrofuran and the like in the presence of triphenylphosphine and the like by using diisopropyl azodicarboxylate (DIAD), diethyl azodicarboxylate (DEAD) and the like.

Here, amine derivative (S16) can be synthesized by the method shown below.

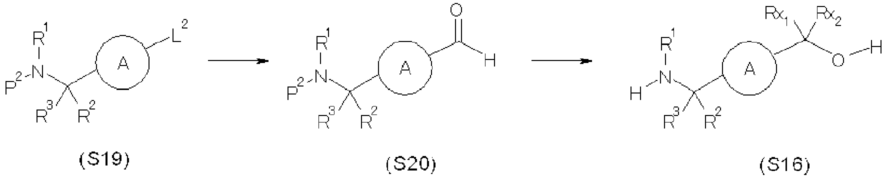

wherein P² is a suitable protecting group such as a tert-butoxycarbonyl group (Boc group) and the like, and L² is a halogen atom such as bromine atom, iodine atom and the like.

Aldehyde derivative (S20) can be synthesized by reacting halogen derivative (S19) with n-butyllithium, sec-butyllithium, tert-butyllithium and the like and then with N,N-dimethylformamide, N-formylpiperidine and the like in a solvent that does not adversely influence the reaction such as tetrahydrofuran, diethyl ether and the like in the presence or absence of N,N,N',N'-tetramethylethylenediamine, hexamethylphosphoramide, dimethylpropyleneurea and the like. Amine derivative (S16) can be synthesized by reducing aldehyde derivative (S20) with sodium borohydride and the like in a solvent that does not adversely influence the reaction such as methanol, ethanol, tetrahydrofuran and the like and removing the protecting group P² by a suitable method.

Alcohol derivative (S17) can also be synthesized by the method shown below.

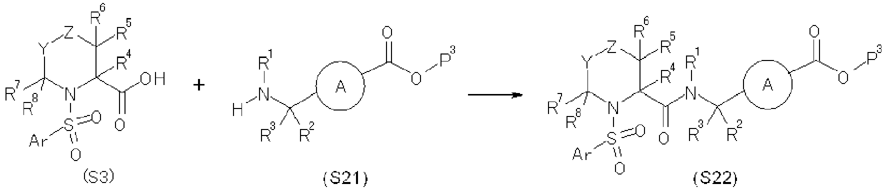

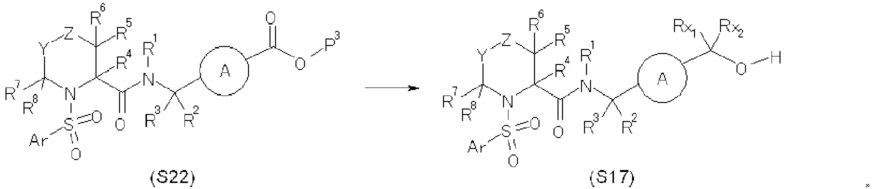

wherein P³ is a suitable protecting group such as methyl group, ethyl group, benzyl group, tert-butyl group and the like.

Ester derivative (S22) can be synthesized by reacting carboxylic acid derivative (S3) and amine derivative (S21) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like. Amide derivative (S17) can be synthesized by reducing ester derivative (S22) with lithium borohydride and the like in a solvent that does not adversely influence the reaction such as tetrahydrofuran and the like. Alcohol derivative (S17) can also be synthesized by reducing mixed acid anhydride obtained by removing the protecting group P³ of ester derivative (S22) by a suitable method, reacting same with ethyl chloroformate and the like in a solvent that does not adversely influence the reaction such as tetrahydrofuran and the like in the presence of a base such as triethylamine or N-ethyldiisopropylamine and the like, with sodium borohydride and the like.

Here, amine derivative (S21) can also be synthesized by the method shown below.

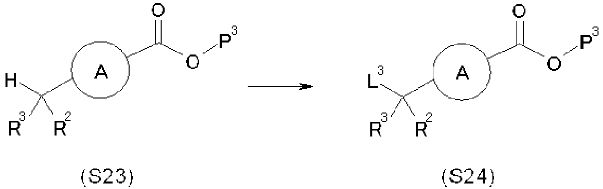

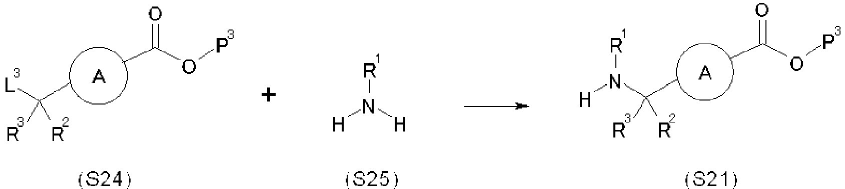

wherein P³ is a suitable protecting group such as methyl group, ethyl group, benzyl group, tert-butyl group and the like.

Halogen derivative (S24) (wherein L³ is a halogen atom such as bromine atom and chlorine atom) can be synthesized by heating carboxylic acid protected form (S23) together with N-bromosuccinimide, N-chlorosuccinimide and the like in a solvent that does not adversely influence the reaction such as carbon tetrachloride, benzene and the like in the co-presence of a radical initiator such as benzoyl peroxide, N,N'-azobisisobutyronitrile and the like. Amine derivative (S21) can be synthesized by reacting halogen derivative (S24) and amine derivative (S25) in a solvent that does not adversely influence the reaction such as acetonitrile, tetrahydrofuran, methanol, ethanol and the like in the presence or absence of a base such as sodium carbonate, potassium carbonate, triethylamine, N-ethyldiisopropylamine and the like.

Compound (S18) can also be synthesized as follows.

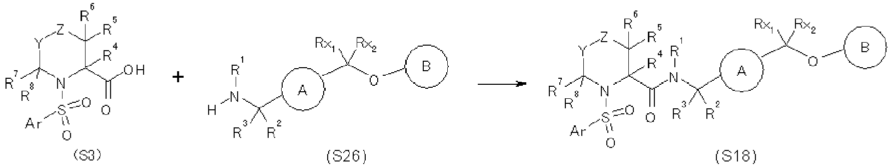

The object compound (S18) can be produced by reacting carboxylic acid derivative (S3) and amine derivative (S26) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like.

Here, amine derivative (S26) can be synthesized as follows.

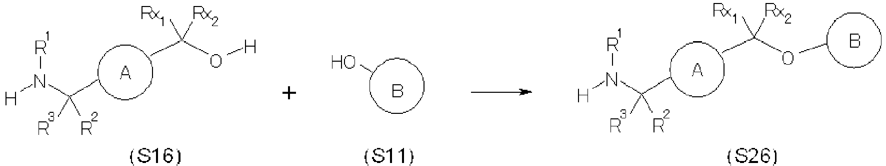

Amine derivative (S26) can be synthesized by reacting amine derivative (S16) and alcohol derivative (S11) in a solvent that does not adversely influence the reaction such as dichloromethane, tetrahydrofuran and the like in the presence of triphenylphosphine and the like, by using diisopropyl azodicarboxylate (DIAD), diethyl azodicarboxylate (DEAD) and the like.

Amine derivative (S26) can also be synthesized as follows.

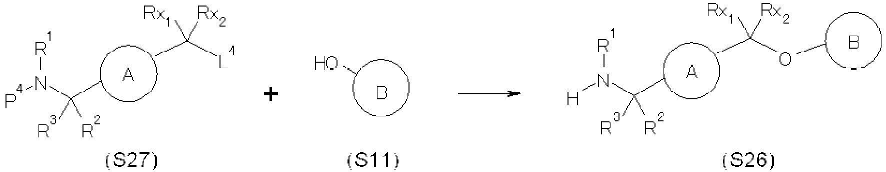

wherein P⁴ is a suitable protecting group such as tert-butoxycarbonyl group (Boc group) and the like, and L⁴ is a suitable leaving group such as chlorine atom, bromine atom, methanesulfonyloxy group, 4-toluenesulfonyloxy group and the like.

Amine derivative (S26) can be synthesized by reacting amine derivative (S27) with alcohol derivative (S11) in a solvent that does not adversely influence the reaction such as acetone or tetrahydrofuran and the like in the presence of a base such as potassium carbonate, sodium carbonate, cesium carbonate, sodium hydride and the like in the presence or absence of potassium iodide, sodium iodide, tetra-n-butylammonium iodide and the like, and removing the protecting group P⁴ by a suitable method.

Compound (S18) can also be synthesized as follows.

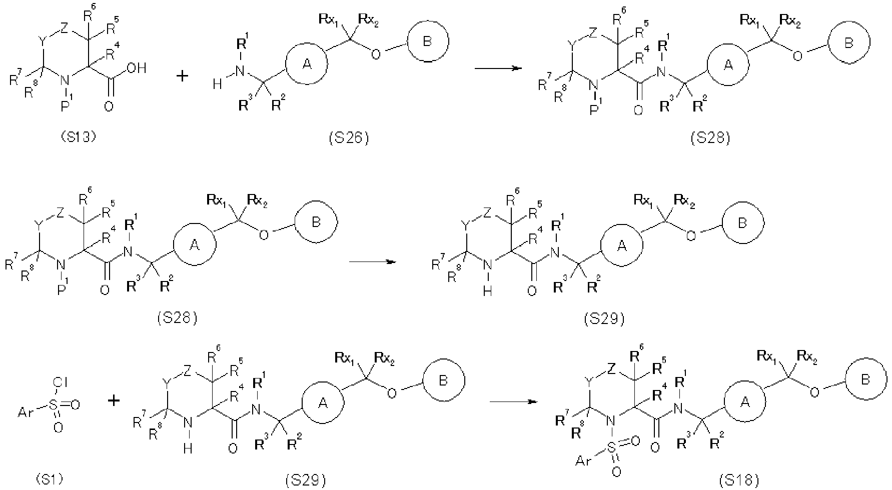

wherein P¹ is a suitable protecting group such as tert-butoxycarbonyl group (Boc group), benzyloxycarbonyl group (Cbz group) and the like.

Amide derivative (S28) can be synthesized by reacting carboxylic acid derivative (S13) and amine derivative (S26) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like. The object compound (S18) can be produced by sulfonating amine derivative (S29) obtained by removing the protecting group P¹ of amide derivative (S28) by the aforementioned method with sulfonyl chloride (S1) in a solvent that does not adversely influence the reaction such as dichloromethane and the like in the presence of a base such as triethylamine and the like.

### Synthesis Method 3: In the formula (I) (and the formula (IA)) wherein n is 0 and m is 1 (synthesis of compound S31)

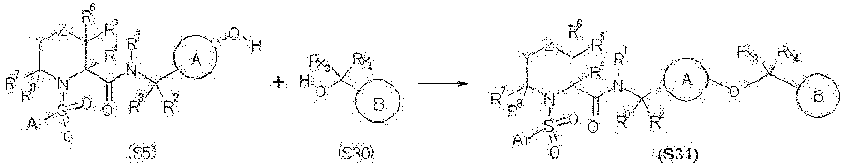

The object compound (S31) can be produced by reacting amide derivative (S5) and alcohol derivative (S30) in a solvent that does not adversely influence the reaction such as dichloromethane, tetrahydrofuran and the like in the presence of triphenylphosphine and the like by using diisopropyl azodicarboxylate (DIAD), diethyl azodicarboxylate (DEAD) and the like.

The object compound (S31) can also be synthesized as follows.

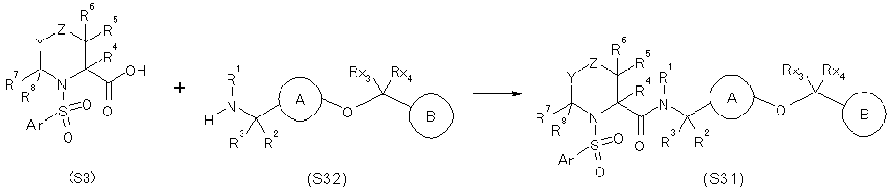

The object compound (S31) can be produced by reacting carboxylic acid derivative (S3) and amine derivative (S32) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like.

Here, amine derivative (S32) can be synthesized as follows.

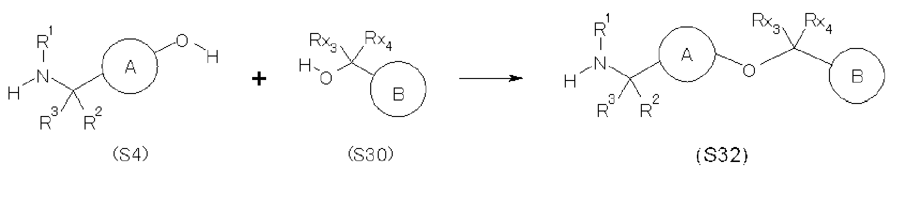

Amine derivative (S32) can be synthesized by reacting amide derivative (S4) and alcohol derivative (S30) in a solvent that does not adversely influence the reaction such as dichloromethane, tetrahydrofuran and the like in the presence of triphenylphosphine and the like by using diisopropyl azodicarboxylate (DIAD), diethyl azodicarboxylate (DEAD) and the like.

Compound (S31) can also be synthesized as follows. 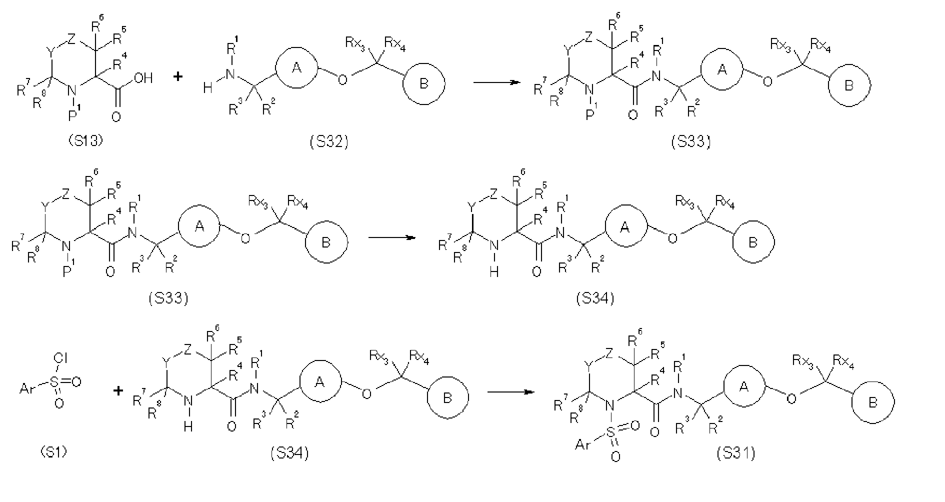 wherein P¹ is a suitable protecting group such as tert-butoxycarbonyl group (Boc group), benzyloxycarbonyl group (Cbz group) and the like.

Amide derivative (S33) can be synthesized by reacting carboxylic acid derivative (S13) and amine derivative (S32) in a solvent that does not adversely influence the reaction such as dichloromethane and the like in the presence or absence of 1-hydroxybenzotriazole and the like with a condensing agent represented by 1-ethyl-3-(3'-dimethylaminopropyl)carbodiimide (WSC) in the presence of a base such as triethylamine and the like. The object compound (S31) can be produced by sulfonating amine derivative (S34) obtained by removing the protecting group P¹ of amide derivative (S33) by the aforementioned method with sulfonyl chloride (S1) in a solvent that does not adversely influence the reaction such as dichloromethane and the like in the presence of a base such as triethylamine and the like..

### Examples

The present invention is explained in detail in the following by referring to Reference Example, Example and Experimental Example, which are not to be construed as limitative.

### Reference Example A-1: Synthesis of (2S)-1-(5-chlorothiophene-2-sulfonyl)pyrrolidine-2-carboxylic acid (A-1)

L-Proline (1.0 g, 8.7 mmol) was dissolved in 2 mol/L aqueous sodium hydroxide solution (10 mL) and tetrahydrofuran (10 mL), 5-chloro-thiophene-2-sulfonylchloride (1.4 mL, 10 mmol) was added, and the mixture was stirred at room temperature overnight. The reaction mixture was extracted with dichloromethane, and the aqueous layer was neutralized with 2 mol/L hydrochloric acid and extracted with dichloromethane. The obtained organic layer was dried over sodium sulfate. The desiccant was filtered off, and the solvent was evaporated to give the title compound as pale-brown - brown crystals (2.5 g, 8.4 mmol, 97%).
MS (ESI) m/z 296 (M+H)⁺
¹H NMR (400 MHz, DMSO) δ 7.64 (d, J = 4.1 Hz, 1H), 7.34 (d, J = 4.1 Hz, 1H), 4.11 (dd, J = 8.7, 4.1 Hz, 1H), 3.47 - 3.38 (m, 1H), 3.28 - 3.19 (m, 1H), 2.08 - 1.94 (m, 1H), 1.94 - 1.78 (m, 2H), 1.74 - 1.62 (m, 1H).

A-2 - A-12 described in Table 1 and Table 2 were synthesized using the corresponding commercially available reagents and by an operation similar to Reference Example A-1. Similarly, A-13 - A-31 described in Table 3 were synthesized using the corresponding commercially available reagents and by an operation similar to Reference Example A-1.

**Table 1**

| Reference Example No. | Structural Formula | MS(ESI) *m*/*z* (M+H)⁺ | NMR |
|---|---|---|---|
| A-1 | 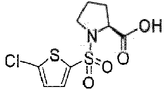 | 296 | ¹H NMR (400 MHz, DMSO) δ 7.64 (d, J = 4.1 Hz, 1H), 7.34 (d, J = 4.1 Hz, 1H), 4.11 (dd, J = 8.7, 4.1 Hz, 1H), 3.47 - 3.38 (m, 1H), 3.28-3.19 (m, 1H), 2.08 - 1.94 (m, 1H), 1.94 -1.78 (m, 2H), 1.74 - 1.62 (m,1H). |
| A-2 | 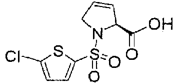 | 294 | - |
| A-3 | 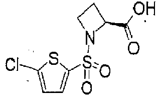 | 282 | - |
| A-4 | 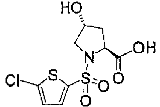 | 312 | - |
| A-5 | 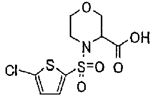 | 312 | ¹H NMR (400 MHz, DMSO) δ 13.23 (s, 1H), 7.57 (d, J = 4.1 Hz, 1H), 7.29 (d, J = 4.1 Hz, 1H), 4.42 (d, J = 3.4 Hz, 1H), 4.14 (d, J = 11.7 Hz, 1H), 3.83 (d, J = 8.4 Hz, 1H), 3.58 (dd, J = 11.7, 3.7 Hz, 1H), 3.51 - 3.45 (m, 1H), 3.44 - 3.36 (m,2H). |
| A-6 | 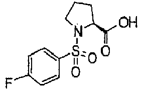 | 274 | - |

**Table 2**

| Reference Example No. | Structural Formula | MS(ESI) *m*/*z* (M+H)⁺ | NMR |
|---|---|---|---|
| A-7 | 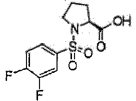 | 292 | ¹H NMR (400 MHz, DMSO) δ 12.79 (s, 1H), 7.99 - 7.92 (m, 1H), 7.76 - 7.65 (m, 2H), 4.21 (dd, J = 8.7, 3.9 Hz, 1H), 3.40 - 3.34 (m, 1H), 3.22 (dt, J = 9.8, 7.1 Hz, 1H), 2.05 - 1.60 (m, 4H). |
| A-8 | 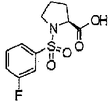 | 274 | ¹H NMR (400 MHz, DMSO) δ 12.78 (s, 1H), 7.77 - 7.51 (m, 4H), 4.19 (dd, J = 8.6,3.9 Hz, 1H), 3.41 - 3.34 (m, 1H), 3.27 - 3.15 (m, 1H), 2.04 - 1.73 (m, 3H), 1.70 - 1.56 (m, 1H). |
| A-9 | 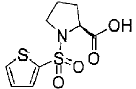 | 276 | - |
| A-10 | 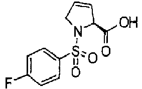 | 272 | - |
| A-11 | 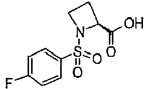 | 260 | - |
| A-12 | 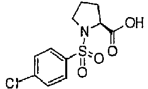 | 290 | - |

**Table 3**

| Reference Example No. | Structural Formula | **MS(ESI) *m*/*z* (M+H)⁺** | Reference Example | Structural Formula | **MS(ESI) *m*/*z* (M+H)⁺** |
|---|---|---|---|---|---|
| A-13 | 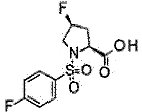 | 292 | A-22 | 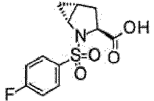 | 286 |
| A-14 | 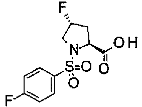 | 292 | A-23 | 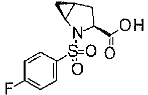 | 286 |
| A-15 | 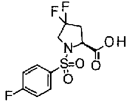 | 310 | A-24 | 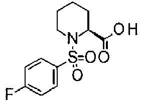 | 288 |
| A-16 | 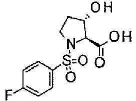 | 290 | A-25 | 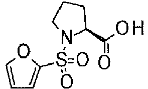 | 246 |
| A-17 | 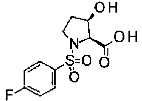 | 290 | A-26 | 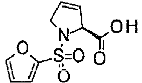 | 244 |
| A-18 | 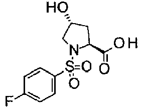 | 290 | A-27 | 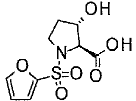 | 262 |
| A-19 | 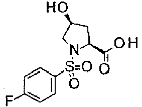 | 290 | A-28 | 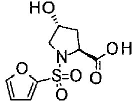 | 262 |
| A-20 | 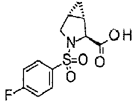 | 286 | A-29 | 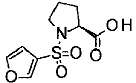 | 246 |
| A-21 | 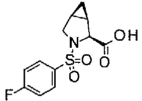 | 286 | A-30 | 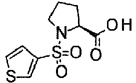 | 262 |
| | | | A-31 | 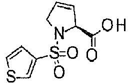 | 260 |

### Reference Example A-32: Synthesis of (2S)-1-(thiazol-2-ylsulfonyl)pyrrolidine-2-carboxylic acid (A-32)

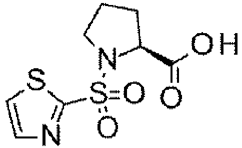

2-Mercaptothiazole (0.12 g, 1.0 mmol) was suspended in conc. sulfuric acid (5 mL), and the suspension was cooled to - 15°C. Aqueous sodium hypochlorite solution (10% chlorine, 11 mL) was slowly added while adjusting the titration rate such that the temperature in the reaction was within the range from -15°C to 10°C, and the mixture was warmed to 0°C, and stirred for 1 hr. Water (10 mL) was added to the reaction mixture, and the mixture was extracted three times with dichloromethane. The dichloromethane layer was washed with water and dried over sodium sulfate. The desiccant was filtered off, and the solvent was evaporated to give a crude product (0.16 g) of thiazol-2-ylsulfonyl chloride. The obtained sulfonyl chloride was dissolved in acetonitrile (5 mL), L-proline tert-butyl ester hydrochloride (0.19 g, 0.90 mmol) and triethylamine (0.21 mL, 1.5 mmol) were added, and the mixture was stirred at room temperature overnight. Ethyl acetate was added to the reaction mixture, the mixture was washed successively with 1 mol/L aqueous hydrochloric acid solution, saturated aqueous sodium hydrogen carbonate and saturated brine, and the organic layer was dried over sodium sulfate. The desiccant was filtered off, and the solvent was evaporated. Trifluoroacetic acid (3 mL) was added to the obtained residue, and the mixture was stirred at room temperature for 2 hr. The solvent was evaporated to give the title compound (0.10 g, 0.38 mmol, 97%).
MS (ESI) m/z 263 (M+H)⁺

### Reference Example B-1: Synthesis of (2S)-1-(5-chlorothiophene-2-sulfonyl)-N-[(4-hydroxyphenyl)methyl]pyrrolidine-2-carboxamide (B-1)

To A-1 (0.36 g, 1.2 mmol) and 4-(aminomethyl)phenol (0.15 g, 1.2 mmol), WSC hydrochloride (0.46 g, 2.4 mmol) and HOAt (0.33 g, 2.4 mmol) were added triethylamine (510 µL, 3.7 mmol), dichloromethane (12 mL), and the mixture was stirred at room temperature for several hours. Ethyl acetate was added, and the mixture was washed successively with water and saturated aqueous sodium hydrogen carbonate. The organic layer was dried over sodium sulfate, and the desiccant was filtered off. The solvent was evaporated, and the obtained residue was purified by high performance liquid chromatography (water-acetonitrile, each containing 0.1% trifluoroacetic acid) to give the title compound (0.35 g, 0.87 mmol, 72%).
MS (ESI) m/z 401 (M+H)⁺
¹H NMR (400 MHz, DMSO) δ 9.26 (br-s, 1H), 8.40 (t, J = 5.9 Hz, 1H), 7.64 (d, J = 4.1 Hz, 1H), 7.35 (d, J = 4.1 Hz, 1H), 7.07 (d, J = 8.6 Hz, 2H), 6.74 - 6.65 (m, 2H), 4.22 (dd, J = 14.8, 6.2 Hz, 1H), 4.14 (dd, J = 14.8, 5.7 Hz, 1H), 4.07 (t, J = 5.9 Hz, 1H), 3.54 - 3.47 (m, 1H), 3.23 (dt, J = 10.2, 7.2 Hz, 1H), 1.89 - 1.79 (m, 3H), 1.67 - 1.56 (m, 1H).

B-2 - B-4 described in Table 4 were synthesized using the corresponding commercially available reagents and by an operation similar to Reference Example B-1. Similarly, B-14 and B-15 described in Table 5 were synthesized using the corresponding commercially available reagents and by an operation similar to Reference Example B-1.

**Table 4**

| Reference Example No. | Structural Formula | MS(ESI) *m*/*z* (M+H)⁺ | NMR |
|---|---|---|---|
| B-1 | 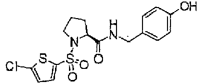 | 401 | ¹H NMR (400 MHz, DMSO) δ 9.26 (br-s, 1H), 8.40 (t, J = 5.9 Hz, 1H), 7.64 (d, J = 4.1 Hz, 1H), 7.35 (d, J = 4.1 Hz, 1H), 7.07 (d, J = 8.6 Hz, 2H), 6.74 - 6.65 (m, 2H), 4.22 (dd, J =14.8, 6.2 Hz, 1H), 4.14 (dd, J = 14.8, 5.7 Hz, 1H), 4.07 (t, J = 5.9 Hz, 1H), 3.54 - 3.47 (m, 1H), 3.23 (dt, J = 10.2, 7.2 Hz, 1H), 1.89 - 1.79 (m, 3H), 1.67 - 1.56 (m, 1H). |
| B-2 | 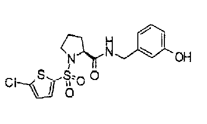 | 401 | ¹H NMR (400 MHz, DMSO) δ 9.34 (br-s, 1H), 8.49 (t, J = 6.0 Hz, 1H), 7.65 (d, J = 4.1 Hz, 1H), 7.35 (d, J = 4.1 Hz, 1H), 7.10 (t, J = 7.7 Hz, 1H), 6.72 - 6.60 (m, 3H), 4.26 (dd, J = 15.3, 6.3 Hz, 1H), 4.17 (dd, J = 15.1, 5.8 Hz, 1H), 4.09 (t, J = 5.8 Hz, 1H), 3.52 - 3.49 (m, 1H), 3.28 - 3.19 (m, 1H), 1.91 - 1.78 (m, 3H), 1.69 - 1.57 (m, 1H). |
| B-3 | 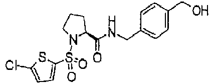 | 415 | ¹H NMR (400 MHz, DMSO) δ 8.59 (t, J = 6.0 Hz, 1H), 7.64 (d, J = 4.1 Hz, 1H), 7.34 (d, J = 4.1 Hz, 1H), 7.29 - 7.18 (m, 4H), 4.46 (s, 2H), 4.32 (dd, J = 15.3, 6.3 Hz, 1H), 4.24 (dd, J = 15.2, 5.8 Hz, 1H), 4.06 (dd, J = 7.0, 4.7 Hz, 1H), 3.52 - 3.48 (m, 1H), 3.22 (dt, J = 9.7,6.7 Hz, 1H), 1.88 - 1.77 (m, 3H), 1.66 -1.56 (m, 1H). |
| B-4 | 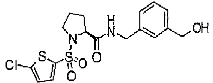 | 415 | - |

**Table 5**

| Reference Example No. | Structural Formula | **MS(ESI) *m*/*z* (M+H)⁺** |
|---|---|---|
| B-14 | 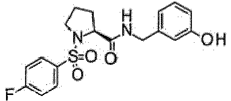 | 379 |
| B-15 | 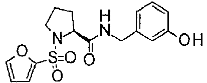 | 351 |

### Reference Example B-5: Synthesis of (2S)-N-[(3-chloro-4-hydroxy-phenyl)methyl]-1-(5-chlorothiophene-2-sulfonyl)pyrrolidine-2-carboxamide (B-5)

### (step 1) Synthesis of 4-(aminomethyl)-2-chloro-phenol hydrobromide

To a solution of 3-chloro-4-methoxy-benzylamine hydrochloride (0.17 g, 0.84 mmol) in dichloromethane (21 mL) was slowly added boron tribromide (1.0 mol/L dichloromethane solution, 3.8 mL, 3.8 mmol) at 0°C. After stirring for 2 hr, the mixture was concentrated under reduced pressure.
MS (ESI) m/z 158 (M+H)⁺

### (step 2) Synthesis of (2S)-N-[(3-chloro-4-hydroxy-phenyl)methyl]-1-(5-chlorothiophene-2-sulfonyl)pyrrolidine-2-carboxamide (B-5)

By an operation similar to that of Reference Example B-1 and using the compound (0.20 mg, 0.84 mmol) of step 1 instead of 4-(aminomethyl)phenol, the title compound (0.030 g, 0.069 mmol, 8.2%) was obtained.
MS (ESI) m/z 435 (M+H)⁺

B-6 - B-9 described in Table 6 were synthesized using the corresponding commercially available reagents and by an operation similar to Reference Example B-5.

**Table 6**

| Reference Example No. | Structural Formula | MS(ESI) *m*/*z* (M+H)⁺ | NMR |
|---|---|---|---|
| B-5 | 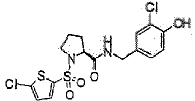 | 435 | - |
| B-6 | 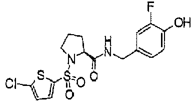 | 419 | - |
| B-7 | 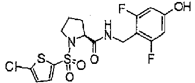 | 437 | - |
| B-8 | 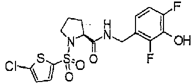 | 437 | - |
| B-9 | 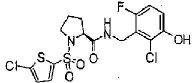 | 453 | - |

### Reference Example B-10: Synthesis of (2S)-1-(5-chlorothiophene-2-sulfonyl)-N-{(1S)-1-[4-(hydroxymethyl)phenyl]ethyl}pyrrolidine-2-carboxamide (B-10)

### (step 1) Synthesis of tert-butyl N-[(S)-1-(4-bromophenyl)ethyl]carbamate

To a solution of (S)-1-(4-bromophenyl)ethylamine (0.30 g, 1.5 mmol) in dichloromethane (5 mL) was added di-tert-butyl dicarbonate (0.33 g, 1.5 mmol), and the mixture was stirred at room temperature overnight. The reaction mixture was concentrated under reduced pressure to give the title compound (0.45 g, 1.5 mmol, 100%).
MS (ESI) m/z 300 (M+H)⁺

### (step 2) Synthesis of tert-butyl N-{(S)-1-[4-(hydroxymethyl)phenyl]ethyl}carbamate

To a solution of the compound (0.082 g, 0.27 mmol) obtained in step 1 in tetrahydrofuran (2 mL) was slowly added n-butyllithium (1.6 mol/L n-hexane solution, 0.34 mL, 0.54 mmol) at -78°C. After stirring for 10 min, N,N-dimethylformamide (0.2 mL) was added to the reaction mixture, and the mixture was further stirred for 1 hr. After warming to room temperature, sodium borohydride (0.026 g, 0.68 mmol) and a piece of ice were added to the reaction mixture, and the mixture was stirred for 20 min. 1 mol/L Aqueous hydrochloric acid solution was added to the reaction mixture, and the mixture was extracted with dichloromethane. The organic layer was washed with saturated brine, dried over anhydrous sodium sulfate and concentrated under reduced pressure. The obtained residue was purified by silica gel column chromatography (hexane/ethyl acetate) to give the title compound (0.045 g, 0.18 mmol, 66%).
MS (ESI) m/z 252 (M+H)⁺

### (step 3) Synthesis of {4-[(1S)-1-aminoethyl]phenyl}methanol hydrochloride

To the compound (0.045 g, 0.18 mmol) obtained in step 2 was added 4 mol/L hydrochloric acid/1,4-dioxane solution (2 mL), and the mixture was stirred at room temperature for 2 hr, and dried under reduced pressure to give the title compound as a white powder (0.034 g, 0.18 mmol, 100%).

### (step 4) Synthesis of (2S)-N-[(3-chloro-4-hydroxy-phenyl)methyl]-1-(5-chlorothiophene-2-sulfonyl)pyrrolidine-2-carboxamide (B-10)

By an operation similar to that of Reference Example B-1 and using the compound (0.034 g, 0.18 mmol) of step 1 instead of 4-(aminomethyl)phenol, the title compound (0.060 g, 0.14 mmol, 78%) was obtained.
MS (ESI) m/z 429 (M+H)⁺

### Reference Example B-11: Synthesis of (2S)-N-{[2-chloro-4-(hydroxymethyl)phenyl]methyl}-1-(5-chlorothiophene-2-sulfonyl)pyrrolidine-2-carboxyamide (B-11)

### (step 1) Synthesis of (2S)-N-{[2-chloro-4-(methoxycarbonyl)phenyl]methyl}-1-(5-chlorothiophene-2-sulfonyl)pyrrolidine-2-carboxyamide

Methyl 3-chloro-4-methylbenzoate (0.30 mL, 2.0 mmol) was dissolved in carbon tetrachloride (5 mL), N-bromosuccinimide (0.39 g, 2.2 mmol) and benzoyl peroxide (48 mg, 0.20 mmol) were added, and the mixture was stirred at 80°C overnight. The reaction mixture was filtered and concentrated under reduced pressure, 8 mol/L ammonia-methanol solution was added to the obtained residue, and the mixture was stirred at room temperature for 90 min. The reaction mixture was concentrated under reduced pressure, and the obtained residue was dissolved in dichloromethane (5 mL). A-1 (0.36 g, 1.2 mmol), WSC hydrochloride (0.29 g, 1.5 mmol) and HOAt (0.16 g, 1.2 mmol) were added, and the mixture was stirred at room temperature overnight. The reaction mixture was concentrated under reduced pressure, and the obtained residue was purified by reversed-phase high performance liquid chromatography (water-acetonitrile, each containing 0.1% trifluoroacetic acid) to give the title compound (0.40 g, 0.84 mmol, 42%).

### (step 2) Synthesis of (2S)-N-{[2-chloro-4-(hydroxymethyl)phenyl]methyl}-1-(5-chlorothiophene-2-sulfonyl)pyrrolidine-2-carboxyamide (B-11)

To the compound (0.40 g, 0.84 mmol) obtained in step 1 were added 1,4-dioxane (4 mL) and 6 mol/L aqueous hydrochloric acid solution (4 mL), and the mixture was stirred at 60°C overnight. The reaction mixture was concentrated under reduced pressure, and the obtained residue was dissolved in tetrahydrofuran (5 mL). Ethyl chloroformate (0.084 mL, 0.88 mmol) and triethylamine (0.23 mL, 1.7 mmol) were added, and the mixture was stirred at room temperature for 30 min. After stirring, the resultant insoluble material was removed by filtration. To the filtrate were added sodium borohydride (0.079 g, 2.1 mmol) and a piece of ice, and the mixture was stirred at room temperature for 3 hr. To the reaction mixture was added 1 mol/L aqueous hydrochloric acid solution, and the mixture was extracted with ethyl acetate. The organic layer was washed successively with saturated aqueous sodium hydrogen carbonate and saturated brine, dried over anhydrous sodium sulfate and concentrated under reduced pressure. The obtained residue was purified by silica gel column chromatography (dichloromethane-methanol) to give the title compound (0.29 g, 0.66 mmol, 78%).
MS (ESI) m/z 448 (M+H)⁺
1H NMR (400 MHz, CDCl3) δ 7.43 (d, J = 4.0 Hz, 1H), 7.41 (s, 1H), 7.38 (d, J = 7.8 Hz, 1H), 7.26 - 7.19 (m, 2H), 7.02 (d, J = 4.0 Hz, 1H), 4.68 (d, J = 5.6 Hz, 2H), 4.57 (d, J = 6.1 Hz, 2H), 4.14 (dd, J = 8.4, 2.7 Hz, 1H), 3.60 (ddd, J = 10.4, 6.7, 3.0 Hz, 1H), 3.23 (ddd, J = 10.4, 9.8, 6.5 Hz, 1H), 2.28 (ddd, J = 11.5, 4.8, 3.3 Hz, 1H), 1.89 - 1.68 (m, 4H).

### Reference Example B-12: Synthesis of (2S)-N-{[2-trifluoromethyl-4-(hydroxymethyl)phenyl]methyl}-1-(5-chlorothiophene-2-sulfonyl)pyrrolidine-2-carboxyamide (B-12)

3-Trifluoromethyl-4-methylbenzoic acid (0.50 g, 2.5 mmol) was dissolved in methanol (20 mL), and thionyl chloride (0.53 mL, 7.4 mmol) was slowly added. After stirring at room temperature overnight, the reaction mixture was concentrated under reduced pressure, and an operation similar to that of Reference Example B-11 was performed to give the title compound (0.18 g, 0.36 mmol, 15%).
MS (ESI) m/z 483 (M+H)⁺
1H NMR (400 MHz, CDCl3) δ 8.68 (t, J = 5.9 Hz, 1H), 7.68 (d, J = 4.1 Hz, 1H), 7.66 (s, 1H), 7.55 (d, J = 8.1 Hz, 1H), 7.50 (d, J = 8.1 Hz, 1H), 7.37 (d, J = 4.1 Hz, 1H), 4.56 (s, 2H), 4.51 (dd, J = 15.9, 5.9 Hz, 1H), 4.41 (dd, J = 16.0, 5.5 Hz, 1H), 4.13 (t, J = 5.8 Hz, 1H), 3.53 - 3.50 (m, 1H), 3.30 - 3.21 (m, 1H), 1.94 - 1.83 (m, 3H), 1.70 - 1.59 (m, 1H).

### Reference Example B-13: Synthesis of (2S)-N-{[2-(hydroxymethyl)phenyl]methyl}-1-(5-chlorothiophene-2-sulfonyl)pyrrolidine-2-carboxyamide (B-13)

### (step 1) Synthesis of methyl 2-({[(2S)-1-(5-chlorothiophene-2-sulfonyl)pyrrolidine-2-carbonyl]amino}methyl)benzoate

By an operation similar to that of Reference Example B-1 and using 2-carbomethoxybenzylamine hydrochloride (1.1 g, 3.8 mmol) instead of 4-(aminomethyl)phenol, the title compound (1.5 g, 3.4 mmol, 89%) was obtained.
MS (ESI) m/z 443 (M+H)⁺

### (step 2) Synthesis of 2-({[(2S)-1-(5-chlorothiophene-2-sulfonyl)pyrrolidine-2-carbonyl]amino}methyl)benzoic acid

To the compound (1.5 g, 3.4 mmol) obtained in step 1 were added 4 mol/L hydrochloric acid/1,4-dioxane solution (28 mL) and water (7 mL), and the mixture was stirred at 90°C overnight. The reaction mixture was concentrated under reduced pressure, and the obtained residue was purified by high performance liquid chromatography (water-acetonitrile, each containing 0.1% trifluoroacetic acid) to give the title compound (0.20 g, 0.47 mmol, 14%).
MS (ESI) m/z 429 (M+H)⁺

### (step 3) Synthesis of (2S)-N-{[2-(hydroxymethyl)phenyl]methyl}-1-(5-chlorothiophene-2-sulfonyl)pyrrolidine-2-carboxyamide (B-13)

The compound (0.20 g, 0.47 mmol) obtained in step 2 was dissolved in tetrahydrofuran (7 mL), ethyl chloroformate (0.047 mL, 0.49 mmol) and triethylamine (0.13 mL, 0.93 mmol) were added, and the mixture was stirred at room temperature for 30 min. After stirring, the resultant insoluble material was removed by filtration. To the filtrate were added sodium borohydride (0.044 g, 1.2 mmol) and a piece of ice, and the mixture was stirred at room temperature for 3 hr. To the reaction mixture was added 1 mol/L aqueous hydrochloric acid solution, and the mixture was extracted with ethyl acetate.

The organic layer was washed successively with saturated aqueous sodium hydrogen carbonate and saturated brine, dried over anhydrous sodium sulfate and concentrated under reduced pressure. The obtained residue was purified by silica gel column chromatography (dichloromethane-methanol) to give the title compound (0.18 g, 0.44 mmol, 95%).
MS (ESI) m/z 415 (M+H)⁺
1H NMR (400 MHz, DMSO) δ 8.45 (t, J = 5.8 Hz, 1H), 7.66 (d, J = 4.1 Hz, 1H), 7.42 - 7.37 (m, 1H), 7.36 (d, J = 4.1 Hz, 1H), 7.31 - 7.19 (m, 3H), 5.16 (t, J = 5.4 Hz, 1H), 4.55 (d, J = 5.3 Hz, 2H), 4.39 (dd, J = 15.4, 6.1 Hz, 1H), 4.29 (dd, J = 15.4, 5.6 Hz, 1H), 4.11 (t, J = 5.8 Hz, 1H), 3.55 - 3.48 (m, 1H), 3.24 (dt, J = 9.5, 6.6 Hz, 1H), 1.90 - 1.79 (m, 3H), 1.69-1.59 (m, 1H).

**Table 7**

| Reference Example No. | Structural Formula | MS(ESI) *m*/*z* (M+H)⁺ | NMR |
|---|---|---|---|
| B-10 | 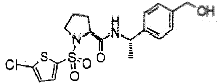 | 429 | - |
| B-11 | 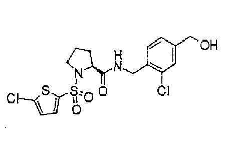 | 448 | ¹H NMR (400 MHz, CDCl₃) δ 7.43 (d, J = 4.0 Hz, 1H), 7.41 (s, 1H), 7.38 (d, J = 7.8 Hz, 1H), 7.26 - 7.19 (m, 2H), 7.02 (d, J = 4.0 Hz, 1H), 4.68 (d, J = 5.6 Hz, 2H), 4.57 (d, J = 6.1 Hz, 2H), 4.14 (dd, J = 8.4, 2.7 Hz, 1H), 3.60 (ddd, J = 10.4, 6.7, 3.0 Hz, 1H), 3.23 (ddd, J = 10.4, 9.8, 6.5 Hz, 1H), 2.28 (ddd, J = 11.5, 4.8, 3.3 Hz, 1H), 1.89 - 1.68 (m, 4H). |
| B-12 | 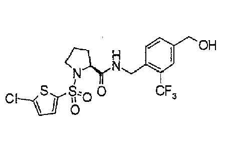 | 483 | ¹H NMR (400 MHz, DMSO) δ 8.68 (t, J = 5.9 Hz, 1H), 7.68 (d, J = 4.1 Hz, 1H), 7.66 (s, 1H), 7.55 (d, J = 8.1 Hz, 1H), 7.50 (d, J = 8.1 Hz, 1H), 7.37 (d, J = 4.1 Hz, 1H), 4.56 (s, 2H), 4.51 (dd, J = 15.9, 5.9 Hz, 1H), 4.41 (dd, J =16.0, 5.5 Hz, 1H), 4.13 (t, J = 5.8 Hz, 1H), 3.53 - 3.50 (m, 1H), 3.30 - 3.21 (m, 1H), 1.94 - 1.83 (m, 3H), 1.70 -1.59 (m, 1H). |
| B-13 | 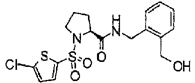 | 415 | ¹H NMR (400 MHz, DMSO) δ 8.45 (t, J = 5.8 Hz, 1H), 7.66 (d, J = 4.1 Hz, 1H), 7.42 - 7.37 (m, 1H), 7.36 (d, J = 4.1 Hz, 1H), 7.31 - 7.19 (m, 3H), 5.16 (t, J = 5.4 Hz, 1H), 4.55 (d, J = 5.3 Hz, 2H), 4.39 (dd, J = 15.4, 6.1 Hz, 1H), 4.29 (dd, J = 15.4, 5.6 Hz, 1H), 4.11 (t, J = 5.8 Hz, 1H), 3.55 - 3.48 (m, 1H), 3.24 (dt, J = 9.5, 6.6 Hz, 1H), 1.90 -1.79 (m, 3H), 1.69 - 1.59 (m, 1H). |

### Reference Example C-1: Synthesis of {4-[3-(trifluoromethyl)phenoxy]phenyl}methanamine trifluoroacetate (C-1)

### (step 1) Synthesis of tert-butyl N-[(4-hydroxyphenyl)methyl]carbamate

To 4-hydroxybenzylamine (500 mg, 4.1 mmol) were added BOC2O (1.1 g, 4.9 mmol), triethylamine (840 µL), water (2 mL) and THF (10 mL), and the mixture was stirred at room temperature for 4 hr. The mixture was diluted with ethyl acetate, and washed with 0.1 mol/L aqueous hydrochloric acid solution. The organic layer was dried over sodium sulfate, and the desiccant was filtered off. The solvent was evaporated and the obtained residue was purified by silica gel column chromatography (hexane/ethyl acetate) to give the title compound (980 mg).
MS(ESI) m/z 224 (M+H)⁺

### (step 2) Synthesis of tert-butyl N-({4-[3-(trifluoromethyl)phenoxy]phenyl}methyl)carbamate

To the compound (500 mg, 2.2 mmol) obtained in step 1 were added 3-trifluoromethylphenylboronic acid (420 mg, 2.2 mmol), copper acetate (410 mg, 2.2 mmol), triethylamine (1.5 mL, 11 mmol), molecular sieves 4Å (1.5 g) and dichloromethane (15 mL), and the mixture was stirred at room temperature overnight. The reaction mixture was filtered to remove the insoluble material, and the solvent was evaporated. Ethyl acetate was added to the residue, and the mixture was washed with 0.1 mol/L aqueous hydrochloric acid solution. The organic layer was dried over sodium sulfate, and the desiccant was filtered off. The solvent was evaporated, and the obtained residue was purified by silica gel column chromatography (hexane/ethyl acetate) to give the title compound (380 mg).
MS(ESI) m/z 368 (M+H)⁺

### (step 3) Synthesis of {4-[3-(trifluoromethyl)phenoxy]phenyl}methanamine hydrochloride (C-1)

To the compound (380 mg) obtained in step 2 was added 4 mol/L hydrochloric acid/1,4-dioxane solution (5 mL), and the mixture was stirred at room temperature for 3 hr. The solvent was evaporated to give the title compound.
MS(ESI) m/z 268 (M+H)⁺
1H NMR (400 MHz, DMSO) δ 8.40 (brs, 3H), 7.65 (t, J = 8.0 Hz, 1H), 7.56 (d, J = 6.8 Hz, 2H), 7.52 (d, J = 7.6 Hz, 1H), 7.30 (dd, J = 8.0, 2.0 Hz, 1H), 7.27 (s, 1H), 7.15 (d, J = 9.6 Hz, 2H), 4.03 (s, 2H).

C-2 described in Table 8 and C-11 described in Table 9 were synthesized using the corresponding commercially available reagents and by an operation similar to Reference Example C-1.

**Table 8**

| Reference Example No. | Structural Formula | MS(ESI) *m*/*z* (M+H)⁺ | NMR |
|---|---|---|---|
| C-1 | 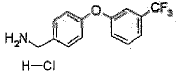 | 268 | ¹H NMR (400 MHz, DMSO) δ 8.40 (brs, 3H), 7.65 (t, J = 8.0 Hz, 1H), 7.56 (d, J = 6.8 Hz, 2H), 7.52 (d, J = 7.6 Hz, 1H), 7.30 (dd, J = 8.0,2.0 Hz, 1H), 7.27 (s, 1H), 7.15 (d, J = 9.6 Hz, 2H), 4.03 (s, 2H). |
| C-2 | 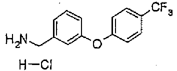 | 268 | ¹H NMR (400 MHz, DMSO) δ 8.45 (brs, 3H), 7.76 (d, J = 8.4 Hz, 2H), 7.50 (t, J = 7.8 Hz, 1H), 7.37 (brd, J = 8.0 Hz, 1H), 7.31 (brs, 1H), 7.17 (d, J = 8.4 Hz, 2H), 7.16-7.14 (m, 1H), 4.04 (s, 2H). |

**Table 9**

| Reference Example No. | Structural Formula | **MS(ESI) *m*/*z* (M+H)⁺** |
|---|---|---|
| C-11 | 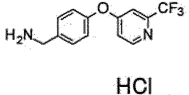 | 269 |

### Reference Example C-3: Synthesis of {2-[4-(trifluoromethyl)phenoxy]pyridin-4-yl}methylamine (C-3)

To 2-chloro-4-cyanopyridine (0.20 g, 1.4 mmol), 4-trifluorophenol (0.23 g, 1.4 mmol) and potassium carbonate (0.30 g, 2.2 mmol) was added dimethyl sulfoxide (3 mL). The mixture was stirred at 60°C overnight, warmed to 80°C, and further stirred for 2 days. The reaction mixture was diluted with ethyl acetate, washed three times with water, and the obtained organic layer was dried over anhydrous sodium sulfate, and concentrated under reduced pressure. The residue was dissolved in tetrahydrofuran (3 mL), and lithium aluminum hydride (0.16 g, 4.3 mmol) was added at 0°C. The mixture was stirred for 2 hr, water was slowly added, and the insoluble material was filtered off. The filtrate was concentrated under reduced pressure, and dichloromethane was added to the obtained residue. The mixture was washed with 2 mol/L aqueous sodium hydroxide solution, and the organic layer was dried over anhydrous sodium sulfate and concentrated under reduced pressure to give a crude product of the title compound.
MS(ESI) m/z 269 (M+H)⁺

C-4 described in Table 10 was synthesized using the corresponding commercially available reagents and by an operation similar to Reference Example C-3.

**Table 10**

| Reference Example No. | Structural Formula | MS(ESI) *m*/*z* (M+H)⁺ | NMR |
|---|---|---|---|
| C-3 | 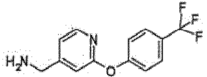 | 269 | - |
| C-4 | 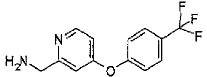 | 269 | - |

### Reference Example C-5: Synthesis of [4-(phenoxymethyl)phenyl]methanamine trifluoroacetate (C-5)

### (step 1) Synthesis of tert-butyl N-{[4-(hydroxymethyl)phenyl]methyl}carbamate

By an operation similar to that of Reference Example C-1, step 1 and using [4-(aminomethyl)phenyl]methanol (1.2 g, 9.7 mmol) instead of 4-(aminomethyl)phenol, the title compound (2.0 g, 8.4 mmol, 87%) was obtained.
MS(ESI) m/z 238 (M+H)⁺

### (step 2) Synthesis of tert-butyl N-{[4-(phenoxymethyl)phenyl]methyl}carbamate

The compound (2.0 g, 8.4 mmol) of step1, phenol (900 µL, 10 mmol) and triphenylphosphine (2.2 g, 13 mmol) were dissolved in dichloromethane (84 mL), diisopropyl azodicarboxylate (2.7 mL, 13 mmol) was added dropwise, and the mixture was stirred for several hours. The solvent was evaporated, and the obtained residue was purified by silica gel column chromatography (hexane/ethyl acetate) to give the title compound (1.8 g, 5.9 mmol, 70%).
MS(ESI) m/z 314 (M+H)⁺

### (step 3) Synthesis of [4-(phenoxymethyl)phenyl]methanamine trifluoroacetate

To the compound (1.8 g, 5.9 mmol) obtained in step 2 was added 4 mol/L hydrochloric acid/1,4-dioxane solution (20 mL), and the mixture was stirred for several hours. The solvent was evaporated, and the obtained residue was purified by high performance liquid chromatography (water-acetonitrile, each containing 0.1% trifluoroacetic acid) to give the title compound (1.7 g, 5.1 mmol, 86%).
MS(ESI) m/z 214 (M+H)⁺
1H NMR (400 MHz, DMSO) δ 7.97 (br-s, 1H), 7.53 - 7.42 (m, 4H), 7.35 - 7.25 (m, 2H), 7.02 - 6.97 (m, 2H), 6.95 (tt, J = 7.3, 1.0 Hz, 1H), 5.13 (s, 2H), 4.03 (s, 2H).

C-6 - C-7 described in Table 11 were synthesized using the corresponding commercially available reagents and by an operation similar to Reference Example C-5.

**Table 11**

| Reference Example | Structural Formula | MS(ESI) *m*/*z* (M+H)⁺ | NMR |
|---|---|---|---|
| C-5 | 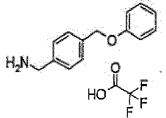 | 214 | ¹H NMR (400 MHz, DMSO) δ 7.97 (br-s, 1H), 7.53 - 7.42 (m, 4H), 7.35 - 7.25 (m, 2H), 7.02 - 6.97 (m, 2H), 6.95 (tt, J = 7.3, 1.0 Hz, 1H), 5.13 (s, 2H), 4.03 (s, 2H). |
| C-6 | 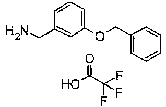 | 214 | - |
| C-7 | 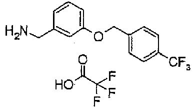 | 282 | - |

### Reference Example C-8: Synthesis of (4-{[3-(trifluoromethyl)phenoxy]methyl}thiazol-2-yl)methylamine hydrochloride (C-8)

### (step 1) Synthesis of tert-butyl N-[{4-(chloromethyl)thiazol-2-yl}methyl]carbamate

To tert-butyl 2-amino-2-thioxoethylcarbamate (0.50 g, 2.6 mmol) and 1,3-dichloroacetone (0.33 g, 2.6 mmol) was added ethanol (4 mL), and the mixture was stirred at 50°C for 2 days. The reaction mixture was concentrated under reduced pressure, ethyl acetate was added to the obtained residue, and the mixture was washed successively with saturated aqueous sodium hydrogen carbonate and saturated brine. The organic layer was dried over anhydrous sodium sulfate, concentrated under reduced pressure, and the obtained residue was purified by silica gel column chromatography (hexane/ethyl acetate) to give the title compound (0.14 g, 0.55 mmol, 21%).
MS (ESI) m/z 263 (M+H)⁺

### (step 2) Synthesis of (4-{[3-(trifluoromethyl)phenoxy]methyl}thiazol-2-yl)methylamine hydrochloride

To the compound (0.035 g, 0.13 mmol) obtained in step 1, 3-trifluoromethylphenol (0.019 mL, 0.16 mmol) and potassium carbonate (0.055 g, 0.40 mmol) was added acetone (1 mL), and the mixture was stirred at 50°C overnight. Ethyl acetate was added to the reaction mixture, and the mixture was washed with saturated aqueous sodium hydrogen carbonate. The organic layer was dried over anhydrous sodium sulfate, and concentrated under reduced pressure. 4 mol/L Hydrochloric acid-1,4-dioxane solution was added to the obtained residue, and the mixture was stirred at room temperature for 3 hr. Diethylether was added to the reaction mixture, and the precipitate was collected by filtration, and washed with a small amount of diethylether to give the title compound (0.034 g, 0.10 mmol, 77%).
MS (ESI) m/z 289 (M+H)⁺

C-9 and C-10 described in Table 12 were synthesized using the corresponding commercially available reagents and by an operation similar to Reference Example C-8.

**Table 12**

| Reference Example | Structural Formula | MS(ESI) m/z (M+H)+ | NMR |
|---|---|---|---|
| C-8 | 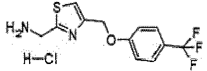 | 289 | - |
| C-9 | 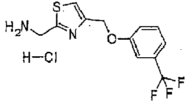 | 289 | - |
| C-10 | 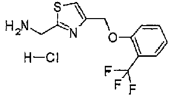 | 289 | - |

### Reference Example D-1: Synthesis of (2S)-N-[(3-{[4-(trifluoromethyl)phenyl]methoxy}phenyl]methyl-1,2,3,6-tetrahydropyridine-2-carboxamide hydrochloride (D-1)

(S)-N-Boc-1,2,3,6-tetrahydro-2-pyridinecarboxylic acid (0.10 g, 0.44 mmol), C-7 (0.15 g, 0.46 mmol), WSC hydrochloride (0.10 g, 0.53 mmol) and HOAt (60 mg, 0.44 mmol) were suspended in dichloromethane (2 mL), triethylamine (0.092 mL, 0.66 mmol) was added, and the mixture was stirred at room temperature overnight. Ethyl acetate was added to the reaction mixture, the mixture was washed successively with 0.5 mol/L aqueous hydrochloric acid solution, saturated aqueous sodium hydrogen carbonate and saturated brine, and the organic layer was dried over sodium sulfate. The desiccant was filtered off, and the solvent was evaporated. 4 mol/L Hydrochloric acid/1,4-dioxane solution (3 mL) was added to the obtained residue, and the mixture was stirred at room temperature for 5 hr. The solvent was evaporated to give the title compound (0.19 g, 0.44 mmol, 99%).
MS (ESI) m/z 391 (M+H)⁺

D-2 - D-4 described in Table 13 were synthesized using the corresponding commercially available reagents and by an operation similar to Reference Example D-1.

**Table 13**

| Reference Example No. | Structural Formula | **MS(ESI) *m*/*z* (M+H)⁺** |
|---|---|---|
| D-1 | 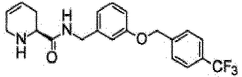 | 391 |
| | HCl | |
| D-2 | 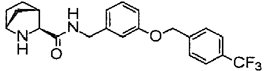 | 405 |
| | HCl | |
| D-3 | 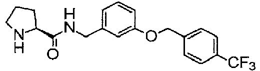 | 379 |
| | HCl | |
| D-4 | 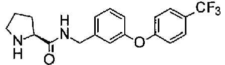 | 365 |
| | HCl | |

### Example 1: Synthesis of (2S)-1-(5-chlorothiophene-2-sulfonyl)-N-[(4-phenoxyphenyl)methyl]pyrrolidine-2-carboxamide (1)

B-1 (35 mg, 0.088 mmol), phenylboronic acid (19 mg, 0.12 mmol), copper acetate (15 mg, 0.12 mmol), molecular sieves 4Å (200 mg, 570 wt%) and triethylamine (61 µL, 0.44 mmol) were dissolved in dichloromethane (1 mL), and the mixture was stirred for several hours. The solid was filtered, dichloromethane was added to the obtained solution, and the mixture was washed successively with 1 mol/L hydrochloric acid, water and saturated aqueous sodium hydrogen carbonate. The organic layer was dried over sodium sulfate, and the desiccant was filtered off. The solvent was evaporated and the obtained residue was purified by high performance liquid chromatography (water-acetonitrile, each containing 0.1% trifluoroacetic acid) to give the title compound (36mg, 0.076 mmol, 87%).
MS(ESI) m/z 477 (M+H)⁺
1H NMR (400 MHz, DMSO) δ 8.59 (t, J = 6.0 Hz, 1H), 7.67 (d, J = 4.1 Hz, 1H), 7.42 - 7.35 (m, 3H), 7.29 (d, J = 8.6 Hz, 2H), 7.11 (tt, J = 7.4, 1.0 Hz, 1H), 7.01 - 6.94 (m, 4H), 4.34 (dd, J = 15.2, 6.2 Hz, 1H), 4.25 (dd, J = 15.2, 5.8 Hz, 1H), 4.09 (t, J = 5.8 Hz, 1H), 3.52 (ddd, J = 9.5, 6.6, 4.5 Hz, 1H), 3.24 (dt, J = 9.7, 7.0 Hz, 1H), 1.91 - 1.77 (m, 3H), 1.73 - 1.55 (m, 1H).

Examples 2 - 28 described in Table 14 - Table 20 were synthesized using the corresponding B-1 - B-2, B-5 - B-9 and commercially available reagents and by an operation similar to Example 1.

**Table 14**

| Example No. | structure compound name | MS(ESI) *m*/*z*(M+H)⁺ | NMR |
|---|---|---|---|
| 1 | 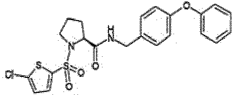 | 477 | ¹H NMR (400 MHz, DMSO) δ 8.59 (t, J = 6.0 Hz, 1H), 7.67 (d, J = 4.1 Hz, 1H), 7.42 - 7.35 (m, 3H), 7.29 (d, J = 8.6 Hz, 2H), 7.11 (tt, J = 7.4, 1.0 Hz, 1H), 7.01-6.94 (m, 4H), 4.34 (dd, J= 15.2, 6.2 Hz, 1H), 4.25 (dd, J = 15.2,5.8 Hz, 1H), 4.09 (t, J = 5.8 Hz, 1H), 3.52 (dd, J = 9.5, 6.6, 4.5 Hz, 1H), 3.24 (dt, J = 9.7, 7.0 Hz, 1H), 1.91 - 1.77 (m, 3H), 1.73 - 1.55 (m, 1H). |
| | (2S)-1-(5-chlorothiophene-2-sulfonyl)-N-[(4-phenoxyphenyl)methyl] pyrrolidine-2-carboxamide | | |
| 2 | 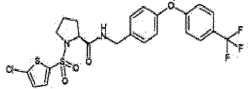 | 545 | ¹H NMR (400 MHz, DMSO) δ 8.63 (d, J = 6.0 Hz, 1H), 7.73 (d, J = 8.7 Hz, 2H), 7.68 (d, J = 4.1 Hz, 1H), 7.38 (d, J = 4.1 Hz, 2H), 7.35 (d, J = 8.6 Hz, 2H), 7.14-7.08 (m, 4H), 4.37 (dd, J = 15.4, 6.2 Hz, 1H), 4.29 (dd, J = 15.4, 5.8 Hz, 1H), 4.10 (t, J = 5.8 Hz, 1H), 3.57 - 3.49 (m, 1H), 3.24 (dt, J = 9.9, 7.0 Hz, 1H), 1.93 - 1.78 (m, 3H), 1.68 - 1.58 (m, 1H). |
| | (2S)-1-(5-chlorothiophene-2-sulfonyl)-N-({4-[4-(trifluoromethyl)phenoxy] phenyl]methyl}pyrrolidine-2-carboxamide | | |
| 3 | 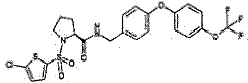 | 561 | ¹H NMR (400 MHz, DMSO) δ 8.65-8.57 (m, 1H), 7.67 (d, J = 4.1 Hz, 1H), 7.42 - 7.34 (m, 3H), 7.31 (d, J = 8.6 Hz, 2H), 7.11 - 6.99 (m, 4H), 4.35 (dd, J = 15.4, 6.3 Hz, 1H), 4.27 (dd, J = 15.5,5.7 Hz, 1H), 4.09 (t, J = 5.9 Hz, 1H), 3.57 - 3.49 (m, 1H), 3.28 - 3.18 (m, 1H), 1.90 - 1.80 (m, 3H), 1.69 - 1.57 (m, 1H). |
| | (2S)-1-(5-chlorothiophene-2-sulfonyl)-N-({4-[4-(trifluoromethoxy)phenoxy] phenyl}methyl)pyrrolidine-2-carboxamide | | |
| 4 | 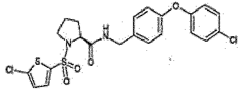 | 511 | ¹H NMR (400 MHz, DMSO) δ 8.60 (s, 1H), 7.67 (d, J = 4.1 Hz, 1H), 7.45 - 7.39 (m, 2H), 7.37 (d, J = 4.1 Hz, 1H), 7.30 (d, J = 8.6 Hz, 2H), 7.03 - 6.97 (m, 4H), 4.34 (dd, J = 15.7, 6.5 Hz, 1H), 4.26 (dd, J = 14.7, 5.8 Hz, 1H), 4.08 (t, J = 5.7 Hz, 1H), 3.53 - 3.50 (m, 1H), 3.27 - 3.22 (m, 1H), 1.89 - 1.81 (m, 3H), 1.68 - 1.59 (m, 1H). |
| | (2S)-N-{[4-(4-chlorophenoxy) phenyl]methyl}-1-(5-chlorothiophene-2-sulfonyl) pyrrolidine-2-carboxamide | | |

**Table 15**

| Example No. | structure compound name | MS(ESI) *m*/*z*(M+H)⁺ | NMR |
|---|---|---|---|
| 5 | 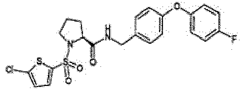 | 495 | ¹H NMR (400 MHz, DMSO) δ 8.58 (t, J = 6.0 Hz, 1H), 7.66 (d, J = 4.1 Hz, 1H), 7.37 (d, J = 4.1 Hz, 1H), 7.28 (d, J = 8.7 Hz, 2H), 7.25 - 7.18 (m, 2H), 7.07 - 7.00 (m, 2H), 6.97 - 6.92 (m, 2H), 4.32 (dd, J = 15.1, 6.1 Hz, 1H), 4.24 (dd, J = 15.3, 6.0 Hz, 1H), 4.08 (t, J = 5.9 Hz, 1H), 3.58-3.49 (m, 1H), 3.23 (dt, J = 9.6, 6.8 Hz, 1H), 1.91 - 1.78 (m, 3H), 1.68 - 1.57 (m, 1H). |
| | (2S)-1-(5-chlorothiophene-2-sulfonyl)-N-{[4-(4-fluorophenoxy)phenyl]methyl} pyrrolidine-2-carboxamide | | |
| 6 | 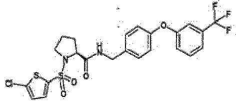 | 545 | ¹H NMR (400 MHz, DMSO) δ 8.62 (t, J = 5.7 Hz, 1H), 7.67 (d, J = 4.1 Hz, 1H), 7.61 (d, J = 7.6 Hz, 1H), 7.47 (d, J = 7.6 Hz, 1H), 7.37 (d, J = 4.1 Hz, 1H), 7.34 (d, J = 8.4 Hz, 2H), 7.27 (d, J = 7.6 Hz, 1H), 7.26 (s, 1H), 7.07 (d, J = 8.5 Hz, 2H), 4.36 (dd, J = 15.3, 6.2 Hz, 1H), 4.28 (dd, J = 15.3, 5.8 Hz, 1H), 4.09 (t, J = 5.6 Hz, 1H), 3.57 - 3.51 (m, 1H), 3.29 - 3.18 (m, 1H), 1.92 - 1.79 (m, 3H), 1.69 - 1.57 (m, 1H). |
| | (2S)-1-(5-chlorothiophene-2-sulfonyl)-N-({4-[3-(trifluoromethyl)phenoxy] phenyl}methyl)pyrrolidine-2-carboxamide | | |
| 7 | 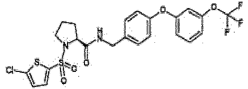 | 561 | ¹H NMR (400 MHz, DMSO) δ 8.62 (t, J = 6.0 Hz, 1H), 7.67 (d, J = 4.1 Hz, 1H), 7.50 (t, J = 8.3 Hz, 1H), 7.37 (d, J = 4.1 Hz, 1H), 7.33 (d, J = 8.6 Hz, 2H), 7.11 (dt, J = 8.3, 1.1 Hz, 1H), 7.08 - 7.03 (m, 2H), 7.00 - 6.93 (m, 2H), 4.36 (dd, J = 15.4, 6.2 Hz, 1H), 4.28 (dd, J = 15.3, 5.8 Hz, 1H), 4.09 (t, J = 5.8 Hz, 1H), 3.57-3.49 (m, 1H), 3.24 (dt, J = 10.0,6.9 Hz, 1H), 1.93 - 1.78 (m, 3H), 1.68 - 1.58 (m, 1H). |
| | (2S)-1-(5-chlorothiophene-2-sulfonyl)-N-({4-[3-(trifluoromethoxy)phenoxy] phenyl}methyl)pyrrolidine-2-carboxamide | | |
| 8 | 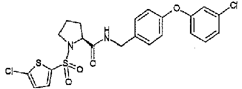 | 511 | ¹H NMR (400 MHz, DMSO) δ 8.61 (t, J = 6.0 Hz, 1H), 7.67 (d, J = 4.1 Hz, 1H), 7.43 - 7.37 (m, 1H), 7.37 (d, J = 4.1 Hz, 1H), 7.32 (d, J = 8.5 Hz, 2H), 7.20 - 7.15 (m, 1H), 7.06 - 7.01 (m, 2H), 7.00 (t, J = 2.2 Hz, 1H), 6.94 (dd, J = 8.3, 2.4 Hz, 1H), 4.35 (dd, J = 15.3, 6.2 Hz, 1H), 4.27 (dd, J = 15.3, 5.7 Hz, 1H), 4.09 (t, J = 5.8 Hz, 1H), 3.55 - 3.50 (m, 1H), 3.30 - 3.21 (m, 1H), 1.92 - 1.80 (m, 3H), 1.63 (d, J = 4.9 Hz, 1H). |
| | (2S)-N-{[4-(3-chlorophenoxy) phenyl]methyl}-1-(5-chlorothiophene-2-sulfonyl) pyrrolidine-2-carboxamide | | |

**Table 16**

| Example No. | structure compound name | MS(ESI) *m*/*z*(M+H)⁺ | NMR |
|---|---|---|---|
| 9 | 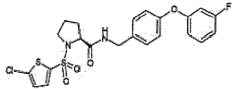 | 495 | ¹H NMR (400 MHz, DMSO) δ 8.61 (t, J = 6.1 Hz, 1H), 7.67 (d, J = 4.1 Hz, 1H), 7.41 (t, J = 7.6 Hz, 1H), 7.37 (d, J = 4.1 Hz, 1H), 7.32 (d, J = 8.6 Hz, 2H), 7.06-7.01 (m, 2H), 6.98 - 6.92 (m, 1H), 6.85-6.77 (m, 2H), 4.35 (dd, J = 15.2, 6.2 Hz, 1H), 4.27 (dd, J = 15.3, 5.9 Hz, 1H), 4.09 (t, J = 5.9 Hz, 1H), 3.58 - 3.48 (m, 1H), 3.30 - 3.19 (m, 1H), 1.90 - 1.80 (m, 3H), 1.67 - 1.59 (m, 1H). |
| | (2S)-1-(5-cholrothiophene-2-sulfonyl)-N-{[4-(3-fluorophenoxy)phenyl] methyl}pyrrolidine-2-carboxamide | | |
| 10 | 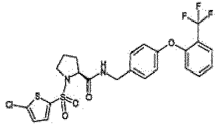 | 545 | - |
| | (2S)-1-(5-chlorothiophene-2-sulfonyl)-N-({4-[2-(trifluoromethyl)phenoxy] phenyl}methyl)pyrrolidine-2-carboxamide | | |
| 11 | 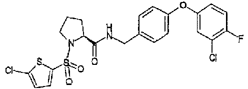 | 529 | ¹H NMR (400 MHz, DMSO) δ 8.60 (t, *J* = 5.9 Hz, 1H), 7.67 (d, *J* = 4.1 Hz, 1H), 7.43 (t, *J* = 9.1 Hz, 1H), 7.37 (d, *J* = 4.1 Hz, 1H), 7.31 (d, *J* = 8.6 Hz, 2H), 7.23 (dd, *J* = 6.2, 3.0 Hz, 1H), 7.04 - 6.98 (m, 3H), 4.34 (dd, *J* = 15.2, 6.2 Hz, 1H), 4.26 (dd, *J* = 15.1, 5.6 Hz, 1H), 4.08 (t, *J* = 5.9 Hz, 1H), 3.56 - 3.49 (m, 1H), 3.30 - 3.17 (m, 1H), 1.90 - 1.80 (m, 3H), 1.68 - 1.57 (m, 1H). |
| | (2S)-N-{[4-(3-chloro-4-fluoro-phenoxy)phenyl]methyl}-1-(5-chlorothiophene-2-sulfonyl) pyrrolidine-2-carboxamide | | |
| 12 | 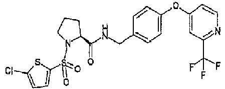 | 546 | ¹H NMR (400 MHz, DMSO) δ 8.67 (t, *J* = 6.2 Hz, 1H), 8.63 (d, *J* = 5.7 Hz, 1H), 7.67 (d, *J* = 4.1 Hz, 1H), 7.42 (d, *J* = 8.6 Hz, 2H), 7.37 (d, *J* = 4.0 Hz, 2H), 7.27-7.21 (m, 2H), 7.14 (dd, *J* = 5.7, 2.4 Hz, 1H), 4.41 (dd, *J* = 15.4, 6.5 Hz, 1H), 4.32 (dd, *J* = 15.7, 6.1 Hz, 1H), 4.13 - 4.06 (m, 1H), 3.50 - 3.46 (m, 1H), 3.30 - 3.20 (m, 1H), 1.91 - 1.80 (m, 3H), 1.67 - 1.58 (m, 1H). |
| | (2S)-1-(5-chlorothiophene-2-sulfonyl)-N-((4-{[2-(trifluoromethyl)-4-pyridyl] oxy}phenyl)methyl) pyrrolidine-2-carboxamide | | |

**Table 17**

| Example No. | structure compound name | MS(ESI) *m*/*z*(M+H)⁺ | NMR |
|---|---|---|---|
| 13 | 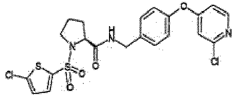 | 512 | - |
| | (2S)-N-({4-[(2-chloro-4-pyridyl) oxy]phenyl}methyl)-1-(5-chlorothiophene-2-sulfonyl) pyrrolidine-2-carboxamide | | |
| 14 | 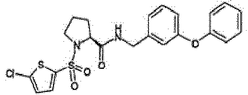 | 477 | ¹H NMR (400 MHz, DMSO) δ 8.60 (t, J = 6.1 Hz, 1H), 7.65 (d, J = 4.1 Hz, 1H), 7.42 - 7.35 (m, 3H), 7.33 (t, J = 7.8 Hz, 1H), 7.13 (t, J = 7.4 Hz, 1H), 7.04 (d, J = 7.6 Hz, 1H), 7.02 - 6.97 (m, 2H), 6.92-6.85 (m, 2H), 4.32 (dd, J = 15.4, 6.3 Hz, 1H), 4.25 (dd, J = 15.5, 6.0 Hz, 1H), 4.05 (dd, J = 7.8, 3.5 Hz, 1H), 3.53 - 3.43 (m, 1H), 3.26 - 3.17 (m, 1H), 1.85 - 1.69 (m, 3H), 1.65 - 1.55 (m, 1H). |
| | (2S)-1-(5-chlorothiophene-2-sulfonyl)-N-[(3-phenoxyphenyl)methyl] pyrrolidine-2-carboxamide | | |
| 15 | 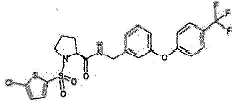 | 545 | ¹H NMR (400 MHz, DMSO) δ 8.63 (t, J = 5.8 Hz, 1H), 7.72 (d, J = 8.6 Hz, 2H), 7.64 (d, J = 4.1 Hz, 1H), 7.40 (t, J = 8.2 Hz, 1H), 7.36 (d, J = 4.0 Hz, 1H), 7.19 - 7.10 (m, 3H), 7.04 - 6.97 (m, 2H), 4.35 (dd, J = 15.9, 6.2 Hz, 1H), 4.29 (dd, J = 15.6, 5.9 Hz, 1H), 4.05 (dd, J = 7.7, 3.4 Hz, 1H), 3.53 - 3.44 (m, 1H), 3.25 - 3.17 (m, 1H), 1.88 - 1.70 (m, 3H), 1.65 - 1.52 (m, 1H). |
| | (2S)-1-(5-chlorothiophene-2-sulfonyl)-N-({3-[4-(trifluoromethyl)phenoxy] phenyl}methyl)pyrrolidine-2-carboxamide | | |
| 16 | 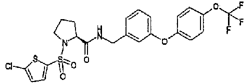 | 561 | ¹H NMR (400 MHz, DMSO) δ 8.63 (t, J = 6.1 Hz, 1H), 7.65 (d, J = 4.1 Hz, 1H), 7.49 (t, J = 8.3 Hz, 1H), 7.39 (t, J = 7.8 Hz, 1H), 7.36 (d, J = 4.1 Hz, 1H), 7.15-7.09 (m, 2H), 7.02 - 6.94 (m, 4H), 4.34 (dd, J = 15.6, 6.1 Hz, 1H), 4.28 (dd, J = 15.6, 6.1 Hz, 1H), 4.05 (dd, J = 8.0,3.6 Hz, 1H), 3.53 - 3.42 (m, 1H), 3.26 - 3.17 (m, 1H), 1.88 -1.71 (m, 3H), 1.65 - 1.54 (m, 1H). |
| | (2S)-1-(5-chlorothiophene-2-sulfonyl)-N-({3-[4-(trifluoromethoxy)phenoxy] phenyl}methyl)pyrrolidine-2-carboxamide | | |

**Table 18**

| Example No. | structure compound name | MS(ESI) *m*/*z*(M+H)⁺ | NMR |
|---|---|---|---|
| 17 | 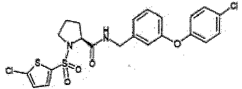 | 511 | ¹H NMR (400 MHz, DMSO) δ 8.63 (t, *J* = 6.1 Hz, 1H), 7.65 (d, *J* = 4.1 Hz, 1H), 7.43 - 7.34 (m, 3H), 7.19 (ddd, *J* = 8.0, 1.9,0.8 Hz, 1H), 7.10 (d, *J* = 7.7 Hz, 1H), 7.03 (t, *J* = 2.2 Hz, 1H), 6.99 - 6.92 (m, 3H), 4.34 (dd, *J* = 15.8, 6.3 Hz, 1H), 4.28 (dd, *J* = 15.7, 6.1 Hz, 1H), 4.05 (dd, *J* = 7.9,3.7 Hz, 1H), 3.54 - 3.44 (m, 1H), 3.26 - 3.17 (m, 1H), 1.87 - 1.70 (m, 3H), 1.66 - 1.54 (m, 1H). |
| | (2S)-N-{[3-(4-chlorophenoxy) phenyl]methyl}-1-(5-chlorothiophene-2-sulfonyl) pyyrrolidine-2-carboxamide | | |
| 18 | 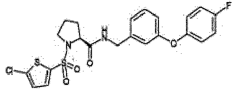 | 495 | ¹H NMR (400 MHz, DMSO) δ 8.62 (t, J = 6.0 Hz, 1H), 7.65 (d, J = 4.1 Hz, 1H), 7.44 - 7.33 (m, 3H), 7.10 (d, J = 7.8 Hz, 1H), 6.99 - 6.91 (m, 3H), 6.87 - 6.79 (m, 2H), 4.34 (dd, J = 15.5, 6.3 Hz, 1H), 4.28 (dd, J = 15.7, 6.1 Hz, 1H), 4.05 (dd, J = 8.0, 3.6 Hz, 1H), 3.53 - 3.45 (m, 1H), 3.26 - 3.17 (m, 1H), 1.86 - 1.70 (m, 3H), 1.66 - 1.54 (m, 1H). |
| | (2S)-1-(5-chlorothiophene-2-sulfonyl)-N-{[3-(4-fluorophenoxy)phenyl]methyl} pyrrolidine-2-carboxamide | | |
| 19 | 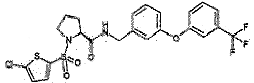 | 545 | ¹H NMR (400 MHz, DMSO) δ 8.63 (t, *J* = 6.0 Hz, 1H), 7.64 (d, *J* = 4.1 Hz, 1H), 7.61 (t, *J* = 8.6 Hz, 1H), 7.48 (dd, *J* = 7.5, 0.6 Hz, 1H), 7.41 - 7.35 (m, 2H), 7.30-7.25 (m, 2H), 7.12 (d, *J* = 7.8 Hz, 1H), 7.00 - 6.94 (m, 2H), 4.35 (dd, *J* = 15.6, 6.2 Hz, 1H), 4.28 (dd, *J* = 15.5, 5.9 Hz, 1H), 4.04 (dd, *J* = 8.0, 3.7 Hz, 1H), 3.55 - 3.43 (m, 1H), 3.26 - 3.16 (m, 1H), 1.88 - 1.69 (m, 3H), 1.63 - 1.51 (m, 1H). |
| | (2S)-1-(5-chlorothiophene-2-sulfonyl)-N-({3-[3-(trifluoromethyl)phenoxy] phenyl}methyl)pyrrolidine-2-carboxamide | | |
| 20 | 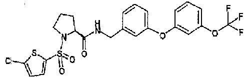 | 561 | ¹H NMR (400 MHz, DMSO) δ 8.62 (t, J = 5.9 Hz, 1H), 7.65 (d, J = 4.0 Hz, 1H), 7.41 - 7.33 (m, 4H), 7.12 - 7.06 (m, 3H), 6.98 - 6.91 (m, 2H), 4.33 (dd, J = 15.5, 5.9 Hz, 1H), 4.27 (dd, J = 15.7, 6.4 Hz, 1H), 4.08 - 4.02 (m, 1H), 3.48 (d, J = 4.6 Hz, 2H), 3.26 - 3.17 (m, 2H), 1.78 (dd, J = 9.5,6.2 Hz, 3H), 1.60 (d, J = 4.6 Hz, 1H). |
| | (2S)-1-(5-chlorothiophene-2-sulfonyl)-N-({3-[3-(trifluoromethoxy)phenoxy] phenyl}methyl)pyrrolidine-2-carboxamide | | |

**Table 19**

| Example No. | structure compound name | MS(BSI) *m*/*z*(M+H)⁺ | NMR |
|---|---|---|---|
| 21 | 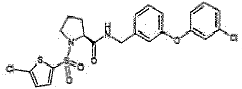 | 511 | ¹H NMR (400 MHz, DMSO) δ 8.61 (t, J = 6.1 Hz, 1H), 7.65 (d, J = 4.1 Hz, 1H), 7.45 - 7.39 (m, 2H), 7.38 - 7.31 (m, 2H), 7.07 (d, J = 7.8 Hz, 1H), 7.04 - 6.99 (m, 2H), 6.94 - 6.89 (m, 2H), 4.32 (dd, J = 15.6, 6.3 Hz, 1H), 4.26 (dd, J = 15.5,6.0 Hz, 1H), 4.05 (dd, J = 8.0,3.7 Hz, 1H), 3.48 - 3.46 (m, 1H), 3.26 - 3.17 (m, 1H), 1.85 - 1.70 (m, 3H), 1.65 -1.54 (m, 1H). |
| | (2S)-N-{[3-(3-chlorophenoxy) phenyl]methyl}-1-(5-chlorothiophene-2-sulfonyl) pyrrolidine-2-carboxamide | | |
| 22 | 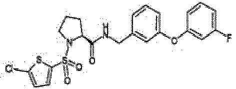 | 495 | ¹H NMR (400 MHz, DMSO) δ 8.60 (t, J = 6.1 Hz, 1H), 7.65 (d, J = 4.1 Hz, 1H), 7.37 (d, J = 4.1 Hz, 1H), 7.35 - 7.29 (m, 1H), 7.26 - 7.18 (m, 2H), 7.09 - 7.00 (m, 3H), 6.88 - 6.83 (m, 2H), 4.31 (dd, J = 15.7, 6.0 Hz, 1H), 4.25 (dd, J = 15.5, 6,0 Hz, 1H), 4.05 (dd, J = 8.0, 3.7 Hz, 1H), 3.53 - 3.44 (m, 1H), 3.26 - 3.17 (m, 1H), 1.85 - 1.69 (m, 3H), 1.64 - 1.54 (m, 1H). |
| | (2S)-1-(5-chlorothiophene-2-sulfonyl)-N-{[3-(3-fluorophenoxy)phenyl] methyl}pyrrolidine-2-carboxamide | | |
| 23 | 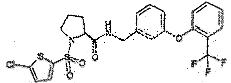 | 545 | ¹H NMR (400 MHz, DMSO) δ 8.62 (t, J = 6.6 Hz, 1H), 7.77 (dd, J = 7.9,0.9 Hz, 1H), 7.68 - 7.60 (m, 2H), 7.40 - 7.34 (m, 2H), 7.31 (t J = 7.6 Hz, 1H), 7.11 (d, J = 7.6 Hz, 1H), 7.01 (d, J = 8.4 Hz, 1M, 6.97 - 6.90 (m, 2H), 4.33 (dd, J = 25.4, 6.0 Hz, 2H), 4.26 (dd, J = 15.7, 6.3 Hz, 1H), 4.04 (dd, J = 7.9, 3.9 Hz, 1H), 3.52 - 3.45 (m, 1H), 3.25 - 3.16 (m, 1H), 1.88 -1.69 (m, 3H), 1.64 - 1.53 (m, 1H). |
| | (2S)-1-(5-chlorothiophene-2-sulfonyl)-N-({3-[2-(trifluoromethyl)phenoxy] phenyl}methyl)pyrrolidine-2-carboxamide | | |
| 24 | 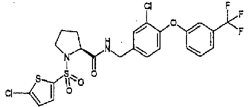 | 579 | - |
| | (2S)-1-(5-chlorothiophene-2-sulfonyl)-N-({3-chloro-4-[3-(trifluoromethyl)phenoxy] phenyl}methyl)pyrrolidine-2-carboxamide | | |

**Table 20**

| Example No. | structure compound name | MS(ESI) *m*/*z*(M+H)⁺ | NMR |
|---|---|---|---|
| 25 | 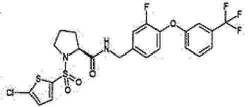 | 563 | - |
| | (2S)-1-(5-chlorothiopphene-2-sulfonyl)-N-({3-fluoro-4-[3-(trifluoromethyl)phenoxy] phenyl}methyl)pyrrolid ine-2-carboxamide | | |
| 26 | 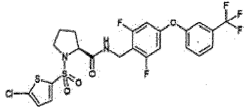 | 581 | - |
| | (2S)-1-(5-chlorothiophene-2-sulfonyl)-N-({2,6-difluoro-4-[3-(trifluoromethyl)phenoxy] phenyl}methyl)pyrrolidine-2-carboxamide | | |
| 27 | 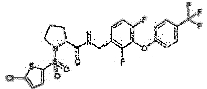 | 581 | - |
| | (2S)-1-(5-chlorothiophene-2-sulfonyl)-N-({2,4-difluoro-3-[4-(trifluoromethyl)phenoxy] phenyl}methyl)pyrrolidine-2-carboxamide | | |
| 28 | 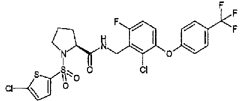 | 597 | - |
| | (2S)-N-({2-chloro-6-fluoro-3-[4-(trifluoromethyl)phenoxy] phenyl}methyl)-1-(5-chlorothiophene-2-sulfonyl) pyrrolidine-2-carboxamide | | |

### Example 29: Synthesis of (2S)-1-(5-chlorothiophene-2-sulfonyl)-N-({4-[3-(trifluoromethyl)phenoxy]phenyl}methyl)-2,5-dihydropyrrole-2-carboxamide (29)

To A-2 (0.018 g, 0.060 mmol) and C-1 (0.018 g, 0.060 mmol), WSC hydrochloride (0.017 g, 0.0090 mmol) and HOAt (0.012 g, 0.090 mmol) were added triethylamine (21 µL, 0.015 mmol) and dichloromethane (1 mL), and the mixture was stirred at room temperature for several hours. The solvent was evaporated, and the obtained residue was purified by high performance liquid chromatography (water-acetonitrile, each containing 0.1% trifluoroacetic acid) to give the title compound (0.028 g, 0.021 mmol, 21%).
MS(ESI) m/z 543 (M+H)⁺
1H NMR (400 MHz, CDCl3) δ 7.47 (d, J = 4.1 Hz, 1H), 7.43 (t, J = 8.0 Hz, 1H), 7.34 (d, J = 7.7 Hz, 1H), 7.33 - 7.27 (m, 2H), 7.26 (d, J = 4.6 Hz, 1H), 7.18 - 7.11 (m, 2H), 7.04 - 6.98 (m, 3H), 5.92 - 5.85 (m, 1H), 5.82 - 5.75 (m, 1H), 4.97 - 4.90 (m, 1H), 4.56 (dd, J = 15.1, 6.2 Hz, 1H), 4.45 (dd, J = 15.1, 5.7 Hz, 1H), 4.38 - 4.29 (m, 1H), 4.28 - 4.20 (m, 1H).

Compounds 30 - 40 described in Table 21 - Table 23 were synthesized using the corresponding A-1 - A-3, A-5 and A-6, A-10 - A-12, C-1 - C-4 and commercially available reagents and by an operation similar to Example 29.

**Table 21**

| Example No. | structure compound name | MS(ESI) *m*/*z* (M+H)⁺ | NMR |
|---|---|---|---|
| 29 | 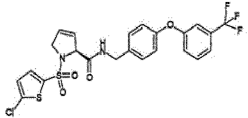 | 543 | ¹H NMR (400 MHz, CDCl3) δ 7.47 (d, J = 4.1 Hz, 1H), 7.43 (t, J = 8.0 Hz, 1H), 7.34 (d, J = 7.7 Hz, 1H), 7.33 - 7.27 (m, 2H), 7.26 (d, J = 4.6 Hz, 1H), 7.18 - 7.11 (m, 2H), 7.04 - 6.98 (m, 3H), 5.92 - 5.85 (m, 1H), 5.82 - 5.75 (m, 1H), 4.97 - 4.90 (m, 1H), 4.56 (dd, J = 15.1, 6.2 Hz, 1H), 4.45 (dd, J = 15.1, 5.7 Hz, 1H), 4.38-4.29 (m, 1H), 4.28 - 4.20 (m, 1H). |
| | (2S)-1-(5-chlorothiophene-2-sulfonyl)-N-({4-[3-(trifluoromethyl)phenoxy] phenyl}methyl)-2,5-dihydropyrrole-2-carboxamide | | |
| 30 | 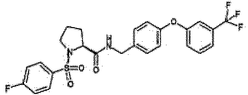 | 523 | ¹H NMR (400 MHz, CDCl₃) δ 7.92-7.85 (m, 2H), 7.44 (t, J = 8.0 Hz, 1H), 7.38 - 7.30 (m, 4H), 7.30 - 7.23 (m, 3H), 7.16 (dd, J = 8.2, 2.0 Hz, 1H), 7.05-6.98 (m, 2H), 4.55 (dd, J = 15.1, 6.1 Hz, 1H), 4.49 (dd, J = 15.1, 5.8 Hz, 1H), 4.18 (dd, J = 8.4, 2.4 Hz, 1H), 3.66-3.53 (m, 1H), 3.16 (td, J = 9.5,6.6 Hz, 1H), 2.27 - 2.16 (m, 1H), 1.85 - 1.59 (m, 3H). |
| | (2S)-1-(4-fluorophenyl) sulfonyl-N-({4-[3-(trifluoromethyl)phenoxy] phenyl}methyl)pyrrolidine-2-carboxamide | | |
| 31 | 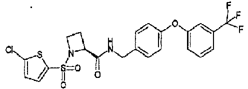 | 531 | ¹H NMR (400 MHz, CDCl3) δ 7.47 (d, J = 4.0 Hz, 1H), 7.43 (t, J = 8.0 Hz, 1H), 7.37 - 7.30 (m, 4H), 7.28 - 7.25 (m, 2H), 7.16 (dd, J = 8.2, 2.0 Hz, 1H), 7.10 (d, J = 4.0 Hz, 1H), 7.04 - 7.00 (m, 2H), 4.54 (dd, J = 15.7, 6.0 Hz, 1H), 4.50 (dd, J = 15.7,5.9 Hz, 1H), 4.39 (dd, J = 9.5,8.0 Hz, 1H), 3.88 (td, J = 8.3,5.0 Hz, 1H), 3.73 (q, J = 8.6 Hz, 1H), 2.49 - 2.32 (m, 2H). |
| | (2S)-1-(5-chlorothiophene-2-sulfonyl)-N-({4-[3-(trifluoromethyl)phenoxy] phenyl}methyl)azetidine-2-carboxamide | | |
| 32 | 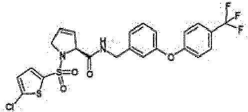 | 543 | ¹H NMR (400 MHz, CDCl3) δ 7.58 (d, J = 8.5 Hz, 2H), 7.43 (d, J = 4.0 Hz, 1H), 7.39 - 7.33 (m, 1H), 7.26 (s, 1H), 7.15 (t, J = 5.7 Hz, 1H), 7.11 (d, J = 8.0 Hz, 1H), 7.06 (d, J = 8.4 Hz, 2H), 7.01 (d, J = 4.0 Hz, 1H), 6.99 - 6.95 (m, 2H), 5.84 (dq, J = 6.5, 2.2 Hz, 1H), 5.78 - 5.72 (m, 1H), 4.93 - 4.86 (m, 1H), 4.57 (dd, J = 15.4, 6.4 Hz, 1H), 4.44 (dd, J = 15.4,5.7 Hz, 1H), 4.34 - 4.25 (m, 1H), 4.25 - 4.18 (m, 1H). |
| | (2S)-1-(5-chlorothiophene-2-sulfonyl)-N-({3-[4-(trifluoromethyl)phenoxy] phenyl}methyl)-2,5-dihydropyrrole-2-carboxamide | | |

**Table 22**

| Example No. | structure compound name | MS(ESI) *m*/*z* (M+H)⁺ | NMR |
|---|---|---|---|
| 33 | 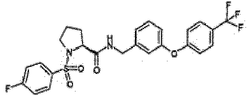 | 523 | ¹H NMR (400 MHz, CDCl₃) δ 7.89-7.81 (m, 2H), 7.58 (d, J = 8.5 Hz, 2H), 7.36 (dd, J = 15.4, 9.7, 6.0 Hz, 2H), 7.29 - 7.21 (m, 3H), 7.13 (d, J = 7.7 Hz, 1H), 7.07 (d, J = 8.4 Hz, 2H), 7.01 - 6.95 (m, 2H), 4.55 (dd, J = 15.5, 6.4 Hz, 1H), 4.49 (dd, J = 15.5, 6.1 Hz, 1H), 4.12 (dd, J = 8.2, 2.6 Hz, 1H), 3.54 (ddd, J = 10.0, 6.5, 3.4 Hz, 1H), 3.13 (td, J = 9.7,6.6 Hz, 1H), 2.26 - 2.10 (m, 1H), 1.79 - -1.56 (m, 3H). |
| | (2S)-1-(4-fluorophenyl) sulfonyl-N-({3-[4-(trifluoromethyl)phenoxy] phenyl}methyl)pyrrolidine-2-carboxamide | | |
| 34 | 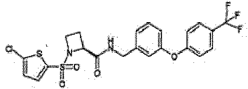 | 531 | ¹H NMR (400 MHz, CDCl₃) δ 7.58 (d, J = 8.5 Hz, 2H), 7.51 (t, J = 4.6 Hz, 1H), 7.44 (d, J = 4.0 Hz, 1H), 7.41 - 7.35 (m, 1H), 7.13 (d, J = 7.9 Hz, 1H), 7.10 (d, J = 4.0 Hz, 1H), 7.07 (d, J = 8.4 Hz, 2H), 7.02 - 6.97 (m, 2H), 4.56 (dd, J = 14.5, 5.3 Hz, 1H), 4.51 (dd, J = 14.5, 5.2 Hz, 1H), 4.42 (t, J = 8.8 Hz, 1H), 3.85 (td, J = 8.0,6.0 Hz, 1H), 3.71 (q, J = 8.7 Hz, 1H), 2.43 - 2.31 (m, 2H). |
| | (2S)-1-(5-chlorothiophene-2-sulfonyl)-N-({3-[4-(trifluoromethyl)phenoxy] phenyl}methyl)azetidine-2-carboxamide | | |
| 35 | 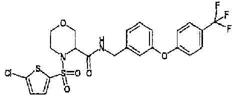 | 561 | ¹H NMR (400 MHz, DMSO) δ 8.59 (dd, J = 5.9, 5.7 Hz, 1H), 7.73 (d, J = 8.5 Hz, 2H), 7.50 (d, J = 4.1 Hz, 1H), 7.42 (t, J = 7.9 Hz, 1H), 7.23 (d, J = 4.1 Hz, 1H), 7.14 (d, J = 8.5 Hz, 2H), 7.10 (d, J = 8.0 Hz, 1H), 7.04 - 6.98 (m, 2H), 4.33 (d, J = 3.2 Hz, 1H), 4.29 (dd, J = 15.6, 5.8 Hz, 1H), 4.24 (dd, J = 15.6,5.7 Hz, 1H), 4.10 (d, J = 11.7 Hz, 1H), 3.77 (dd, J = 11.3, 2.9 Hz, 1H), 3.61 (td, J = 12.3, 3.6 Hz, 1H), 3.55 - 3.44 (m, 2H), 3.37 - 3.33 (m, 1H). |
| | 4-(5-chlorothiophene-2-sulfonyl)-N-({3-[4-(trifluoromethyl)phenoxy] phenyl}methyl)morpholine-3-carboxamide | | |
| 36 | 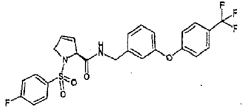 | 521 | - |
| | (2S)-1-(4-fluorophenyl) sulfonyl-N-({3-[4-(trifluoromethyl)phenoxy] phenyl}methyl)-2,5-dihydropyrrole-2-carboxamide | | |

**Table 23**

| Example No. | structure compound name | MS(ESI) *m*/*z* (M+H)⁺ | NMR |
|---|---|---|---|
| 37 | 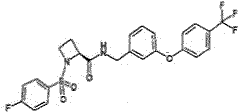 | 508 | - |
| | (2S)-1-(4-fluorophenyl) sulfonyl-N-({3-[4-(trifluoromethyl)phenoxy] phenyl}methyl)azetidine-2-carboxamide | | |
| 38 | 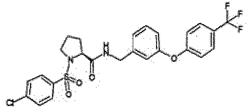 | 539 | - |
| | (2S)-1-(4-chlorophenyl) sulfonyl-N-({3-[4-(trifluoromethyl)phenoxy] phenyl}methyl)pyrrolidine-2-carboxamide | | |
| 39 | 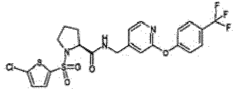 | 546 | ¹H NMR (400 MHz, DMSO) δ 8.76 (dd, J = 6.2, 6.0 Hz, 1H), 8.11 (d, J = 5.2 Hz, 1H), 7.77 (d, J = 8.4 Hz, 2H), 7.69 (d, J = 4.1 Hz, 1H), 7.38 (d, J = 4.1 Hz, ¹H), 7.32 (d, J = 8.4 Hz, 2H), 7.10 (d, J = 5.2 Hz, 1H), 6.99 (s, 1H), 4.41 (dd, J = 16.6, 6.2 Hz, 1H), 4.34 (dd, J = 16.6, 6.0 Hz, 1H), 4.09 (dd, J = 8.0,4.0 Hz, 1H), 3.57 - 3.50 (m, 1H), 3.28 - 3.21 (m, 1H), 1.94 - 1.77 (m, 3H), 1.64 (d, J = 4.7 Hz, 1H). |
| | (2S)-1-(5-chlorothiophene-2-sulfonyl)-N-({2-[4-(trifluoromethyl)phenoxy]-4-pyridyl}methyl)pyrrolidine-2-carboxamide | | |
| 40 | 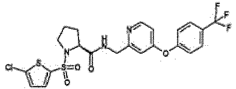 | 546 | - |
| | (2S)-1-(5-chlorothiophene-2-sulfonyl)-N-({4-[4-(trifluoromethyl)phenoxy]-2-pyridyl}methyl)pyrrolidine-2-carboxamide | | |

### Example 41: Synthesis of (2S)-1-(5-chlorothiophene-2-sulfonyl)-N-{[4-(phenoxymethyl)phenyl]methyl}pyrrolidine-2-carboxamide (41)

B-3 (50 mg, 0.12 mmol), phenol (13 µL, 0.15 mmol) and triphenylphosphine (48 mg, 0.18 mmol) were dissolved in dichloromethane (1 mL), diisopropyl azodicarboxylate (17 µL, 0.18 mmol) was added dropwise, and the mixture was stirred for several hours. The solvent was evaporated, and the obtained residue was purified by high performance liquid chromatography (water-acetonitrile, each containing 0.1% trifluoroacetic acid) to give the title compound (35 mg, 0.071 mmol, 59%).
MS(ESI) m/z 491 (M+H)⁺
¹H NMR (400 MHz, DMSO) δ 8.63 (t, J = 5.9 Hz, 1H), 7.67 (d, J = 4.1 Hz, 1H), 7.36 (d, J = 4.0 Hz, 2H), 7.35 - 7.21 (m, 5H), 7.00 (d, J = 7.8 Hz, 2H), 6.93 (t, J = 7.3 Hz, 1H), 5.07 (s, 2H), 4.37 (dd, J = 15.4, 6.2 Hz, 1H), 4.29 (dd, J = 15.4, 5.8 Hz, 1H), 4.10 (t, J = 5.8 Hz, 1H), 3.59 - 3.46 (m, 1H), 3.23 (dt, J = 9.7, 6.8 Hz, 1H), 1.94 - 1.77 (m, 3H), 1.71 - 1.56 (m, 1H).

Compounds 42 - 81 described in Table 24 - Table 34 were synthesized using the corresponding B-3 and B-4, B-10 - B-13, and commercially available reagents and by an operation similar to Example 41.

**Table 24**

| Example No. | structure compound name | MS(ESI) *m*/*z* (M+H)⁺ | NMR |
|---|---|---|---|
| 41 | 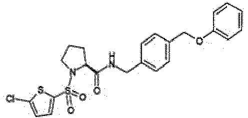 | 491 | ¹H NMR (400 MHz, DMSO) δ 8.63 (t, J = 5.9 Hz, 1H), 7.67 (d, J = 4.1 Hz, 1H), 7.36 (d, J = 4.0 Hz, 2H), 7.35 - 7.21 (m, 5H), 7.00 (d, J = 7.8 Hz, 2H), 6.93 (t, J = 7.3 Hz, 1H), 5.07 (s, 2H), 4.37 (dd, J = 15.4, 6.2 Hz, 1H), 4.29 (dd, J = 15.4, 5.8 Hz, 1H), 4.10 (t, J = 5.8 Hz, 1H), 3.59-3.46 (m, 1H), 3.23 (dt, J = 9.7, 6.8 Hz, 1H), 1.94 -1.77 (m, 3H), 1.71 - 1.56 (m, 1H). |
| | (2S)-1-(5-chlorothiophene-2-sulfonyl)-N-{[4-(phenoxymethyl)phenyl] methyl}pyrrolidine-2-carboxamide | | |
| 42 | 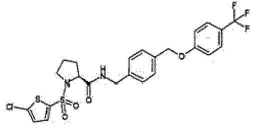 | 559 | ¹H NMR (400 MHz, DMSO) δ 8.60 (t, J = 5.7 Hz, 1H), 7.67 (d, J = 4.1 Hz, 1H), 7.65 (d, J = 8.6 Hz, 2H), 7.41 (d, J = 8.2 Hz, 2H), 7.37 (d, J = 4.1 Hz, 1H), 7.30 (d, J = 8.2 Hz, 2H), 7.19 (d, J = 8.5 Hz, 2H), 5.18 (s, 2H), 4.35 (dd, J = 15.6, 6.6 Hz, 1H), 4.27 (dd, J = 15.4, 5.7 Hz, 1H), 4.08 (t, J = 5.9 Hz, 1H), 3.60 - 3.47 (m, 1H), 3.28 - 3.20 (m, 1H), 1.92 - 1.78 (m, 3H), 1.70 - 1.57 (m, 1H). |
| | (2S)-1-(5-chlorothiophene-2-sulfonyl)-N-((4-{[4-(trifluoromethyl)phenoxy] methyl}phenyl)methyl) pyrrolidine-2-carboxamide | | |
| 43 | 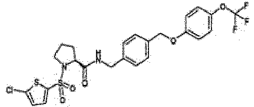 | 575 | ¹H NMR (400 MHz, DMSO) δ 8.60 (t, J = 6.0 Hz, 1H), 7.67 (d, J = 4.1 Hz, 1H), 7.40 (d, J = 8.2 Hz, 2H), 7.37 (d, J = 4.1 Hz, 1H), 7.29 (d, J = 8.3 Hz, 4H), 7.12 - 7.06 (m, 2H), 5.10 (s, 2H), 4.35 (dd, J = 15.3,6.2 Hz, 1H), 4.27 (dd, J =15.4, 5.6 Hz, 1H), 4.08 (t, J = 5.8 Hz, 1H), 3.56-3.48 (m, 1H), 3.28 - 3.21 (m, 1H), 1.89 - 1.79 (m, 3H), 1.67 - 1.57 (m, 1H). |
| | (2S)-1-(5-chlorothiophene-2-sulfonyl)-N-((4-{[4-(trifluoromethoxy)phenoxy] methyl}phenyl)methyl) pyrrolidine-2-carboxamide | | |
| 44 | 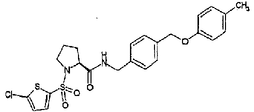 | 505 | ¹H NMR (400 MHz, DMSO) δ 8.59 (t, J = 6.0 Hz, 1H), 7.66 (d, J = 4.1 Hz, 1H), 7.38 (d, J = 5.0 Hz, 2H), 7.36 (s, 1H), 7.27 (d, J = 8.1 Hz, 2H), 7.07 (d, J = 8.5 Hz, 2H), 6.92 - 6.85 (m, 2H), 5.03 (s, 2H), 4.34 (dd, J = 15.3, 6.1 Hz, 1H), 4.26 (dd, J = 15.3, 5.7 Hz, 1H), 4.08 (t, J = 5.8 Hz, 1H), 3.56 - 3.51 (m, 1H), 3.23 (dt, J = 9.7, 6.9 Hz, 1H), 2.22 (s, 3H), 1.93 - 1.78 (m, 3H), 1.69 - 1.56 (m, 1H). |
| | (2S)-1-(5-chlorothiophene-2-sulfonyl)-N-({4-[(4-methylphenoxy)methyl] phenyl}methyl)pyrrolidine-2-carboxamide | | |

**Table 25**

| Example No. | structure compound name | MS(ESI) *m*/*z* (M+H)⁺ | NMR |
|---|---|---|---|
| 45 | 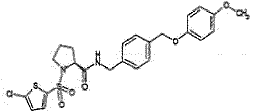 | 521 | ¹H NMR (400 MHz, DMSO) δ 8.59 (t, J = 5.8 Hz, 1H), 7.67 (d, J = 4.1 Hz, 1H), 7.37 (s, 1H), 7.37 (d, J = 7.8 Hz, 2H), 7.27 (d, J = 7.9 Hz, 2H), 6.97 - 6.88 (m, ¹H), 6.87 - 6.80 (m, 1H), 5.01 (s, 2H), 4.35 (dd, J = 15.5, 6.2 Hz, 1H), 4.26 (dd, J = 25.3, 5.7 Hz, 1H), 4.09 (t, J = 5.5 Hz, 1H), 3.69 (s, 3H), 3.57 - 3.47 (m, 1H), 3.28 - 3.18 (m, 1H), 1.90 - 1.77 (m, 3H), 1.68-1.57 (m, 1H). |
| | (2S)-1-(5-chlorothiophene-2-sulfonyl)-N-({4-[(4-methoxyphenoxy)methyl] phenyl}methyl)pyrrolidine-2-carboxamide | | |
| 46 | 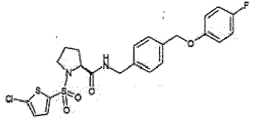 | 509 | ¹H NMR (400 MHz, DMSO) δ 8.60 (t, J = 6.0 Hz, 1H), 7.67 (d, J = 4.1 Hz, 1H), 7.38 (d, J = 8.6 Hz, 2H), 7.36 (s, 1H), 7.28 (d, J = 8.1 Hz, 2H), 7.16 - 7.07 (m, 2H), 7.01 (ddd, J = 6.8,5.4,3.1 Hz, 2H), 5.05 (s, 2H), 4.35 (dd, J = 15.4, 6.2 Hz, 1H), 4.27 (dd, J = 15.4, 5.8 Hz, 1H), 4.08 (t, J = 5.8 Hz, 1H), 3.57 - 3.48 (m, 1H), 3.30 - 3.19 (m, 1H), 1.92 -1.78 (m, 3H), 1.68-1.57 (m, 1H). |
| | (2S)-1-(5-chlorothiophene-2-sulfonyl)-N-({4-[(4-fluorophenoxy)methyl] phenyl}methyl)pyrrolidine-2-carboxamide | | |
| 47 | 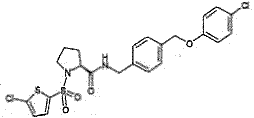 | 525 | ¹H NMR (400 MHz, DMSO) δ 8.60 (t, J = 6.0 Hz, 1H), 7.67 (d, J = 4.1 Hz, 1H), 7.38 (d, J = 8.3 Hz, 2H), 7.37 (d, J = 4.1 Hz, 1H), 7.35 - 7.29 (m, 2H), 7.29 (d, J = 8.2 Hz, 2H), 7.05 - 6.99 (m, 2H), 5.08 (s, 2H), 4.35 (dd, J -15.4, 6.2 Hz, 1H), 4.27 (dd, J = 15.3,5.8 Hz, 1H), 4.09 (t, J = 5.8 Hz, 1H), 3.55 - 3.52 (m, 1H), 3.28 - 3.19 (m, 1H), 1.90 - 1.78 (m, 3H), 1.70 -1.57 (m, 1H). |
| | (2S)-N-({4-[(4-chlorophenoxy) methyl]phenyl}methyl)-1-(5-chlorothiophene-2-sulfonyl) pyrrolidine-2-carboxamide | | |
| 48 | 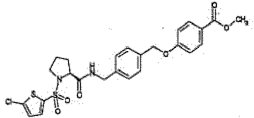 | 549 | ¹H NMR (400 MHz, DMSO) δ 8.60 (t, J = 5.9 Hz, 1H), 7.94 - 7.88 (m, 2H), 7.67 (d, J = 4.1 Hz, 1H), 7.41 (d, J = 8.2 Hz, 2H), 7.37 (d, J = 4.1 Hz, 1H), 7.30 (d, J = 8.2 Hz, 2H), 7.16 - 7.08 (m, 2H), 5.17 (s, 2H), 4.35 (dd, J = 15.2, 6.3 Hz, 1H), 4.27 (dd, J = 15.6, 5.8 Hz, 1H), 4.08 (t, J = 5.9 Hz, 1H), 3.81 (s, 3H), 3.57 - 3.47 (m, 2H), 3.28 - 3.18 (m, 2H), 1.89 - 1.79 (m, 3H), 1.67 - 1.57 (m, 1H). |
| | methyl 4-((4-({[(2S)-1-(5-chlorothiophene-2-sulfonyl) pyrrolidine-2-carbonyl]amino} methyl)phenyl)methoxy) benzoate | | |

**Table 26**

| Example No. | structure compound name | MS(ESI) *m*/*z* (M+H)+ | NMR |
|---|---|---|---|
| 49 | 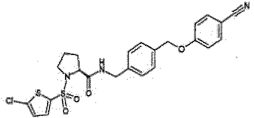 | 516 | ¹H NMR (400 MHz, DMSO) δ 8.61 (t, J = 6.0 Hz, 1H), 7.80 - 7.74 (m, 2H), 7.67 (d, J = 4.1 Hz, 1H), 7.41 (d, J = 8.2 Hz, 2H), 7.37 (d, J = 4.1 Hz, 1H), 7.30 (d, J = 8.2 Hz, 1H), 7.21 - 7.15 (m, 2H), 5.18 (s, 2H), 4.35 (dd, J = 15.4, 6.2 Hz, 1H), 4.27 (dd, J = 15.4, 5.9 Hz, 1H), 4.08 (t, J = 5.9 Hz, 1H), 3.50 (d, J = 6.2 Hz, 1H), 3.24 (dt, J = 9.8, 6.9 Hz, 1H), 1.92 - 1.78 (m, 3H), 1.67-1.56 (m, 1H). |
| | (2S)-1-(5-chlorothiophene-2-sulfonyl)-N-({4-[(4-cyanophenoxy)methyl]pheny} methyl)pyrrolidine-2-carboxamide | | |
| 50 | 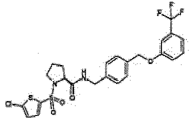 | 559 | ¹H NMR (400 MHz, DMSO) δ 8.64 (t, J = 6.0 Hz, 1H), 7.67 (d, J = 4.1 Hz, 1H), 7.53 (t, J = 8.5 Hz, 1H), 7.41 - 7.24 (m, 8H), 5.17 (s, 2H), 4.38 (dd, J = 15.4, 6.2 Hz, 1H), 4.30 (dd, J =15.4, 5.8 Hz, 1H), 4.10 (t, J = 5.8 Hz, 1H), 3.60 - 3.46 (m, 1H), 3.32 - 3.17 (m, 1H), 1.94 -1.76 (m, 3H), 1.69 -1.54 (m, 1H). |
| | (2S)-1-(5-chlorothiophene-2-sulfonyl)-N-((4-{[3-(trifluoromethyl)phenoxy] methyl}phenyl)methyl) pyrrolidine-2-carboxamide | | |
| 51 | 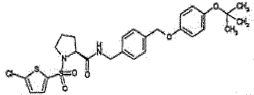 | 563 | - |
| | (2S)-N-({4-[(4-tert-butoxy phenoxy)methyl]phenyl} methyl)-1-(5-chlorothiophene-2-sulfonyl)pyrrolidine-2-carboxamidetyl | | |
| 52 | 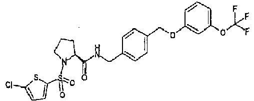 | 575 | ¹H NMR (400 MHz, DMSO) δ 8.60 (t, J = 6.0 Hz, 1H), 7.67 (d, J = 4.1 Hz, 1H), 7.41 (t, J = 8.2 Hz, 1H), 7.41 (d, J = 8.0 Hz, 2H), 7.37 (d, J = 4.1 Hz, 1H), 7.29 (d, J = 8.1 Hz, 2H), 7.05 (dd, J = 8.3, 2.2 Hz, 1H), 7.01 (s, 1H), 6.93 (d, J = 8.2 Hz, 1H), 5.12 (s, 2H), 4.35 (dd, J = 15.3, 6.1 Hz, 1H), 4.27 (dd, J = 15.4, 5.8 Hz, 1H), 4.09 (t, J = 5.8 Hz, 1H), 3.53 - 3.49 (m, 2H), 3.24 (dt, J = 9.8,7.0 Hz, 1H), 1.90 - 1.78 (m, 3H), 1.69 - 1.56 (m, 1H). |
| | (2S)-1-(5-chlorothiophene-2-sulfonyl)-N-((4-{[3-(trifluoromethoxy)phenoxy] methyl}phenyl)methyl) pyrrolidine-2-carboxamide | | |

**Table 27**

| Example No. | structure compound name | MS(ESI) *m*/*z*(M+H)+ | NMR |
|---|---|---|---|
| 53 | 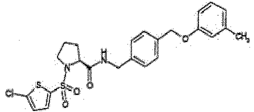 | 505 | ¹H NMR (400 MHz, DMSO) δ 8.60 (t, J = 6.0 Hz, 1H), 7.67 (d, J = 4.1 Hz, 1H), 7.38 (d, J = 8.5 Hz, 2H), 7.37 (d, J = 4.3 Hz, 1H), 7.28 (d, J = 8.0 Hz, 2H), 7.15 (t, J = 7.8 Hz, 1H), 6.83 (s, 1H), 6.78 (dd, J = 8.3,2.3 Hz, 1H), 6.74 (d, J = 7.4 Hz, 1H), 5.05 (s, 2H), 4.35 (dd, J = 15.3, 6.2 Hz, 1H), 4.27 (dd, J = 15.4, 5.7 Hz, 1H), 4.09 (t, J = 5.8 Hz, 1H), 3.55 - 3.49 (m, 1H), 3.24 (dt, J = 9.5, 6.9 Hz, 1H), 2.26 (s, 3H), 1.89 -1.79 (m, 3H), 1.68 - 1.57 (m, 1H). |
| | (2S)-1-(5-chlorothiophene-2-sulfonyl)-N-({4-[(3-methylphenoxy)methyl] phenyl}methyl)pyrrolidine-2-carboxamide | | |
| 54 | 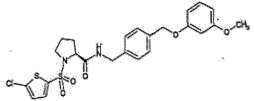 | 521 | ¹H NMR (400 MHz, DMSO) δ 8.60 (t, J = 6.0 Hz, 1H), 7.67 (d, J = 4.1 Hz, 1H), 7.39 (d, J = 82 Hz, 2H), 7.37 (d, J = 4.1 Hz, 1H), 7.28 (d, J = 8.2 Hz, 2H), 7.17 (t, J = 8.1 Hz, 1H), 6.58 (ddd, J = 8.2, 2.4, 0.7 Hz, 1H), 6.56 (t, J = 2.2 Hz, 1H), 6.51 (ddd, J = 8.2, 2.3, 0.7 Hz, 1H), 5.05 (s, 2H), 4.35 (dd, J = 15.3, 6.2 Hz, 1H), 4.27 (dd, J = 15.3, 5.8 Hz, 1H), 4.09 (t, J = 5.8 Hz, 1H), 3.72 (s, 3H), 3.52 (ddd, J = 9.4, 6.6, 4.7 Hz, 1H), 3.24 (dt, J = 9.7,7.0 Hz, 1H), 1.89 -1.79 (m, 3H), 1.69 - 1.57 (m, 1H). |
| | (2S)-1-(5-chlorothiophene-2-sulfonyl)-N-({4-[(3-methoxyphenoxy)methyl] phenyl)methyl)pyrrolidine-2-carboxamide | | |
| 55 | 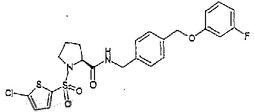 | 509 | ¹H NMR (400 MHz, DMSO) δ 8.60 (t, J = 6.0 Hz, 1H), 7.67 (d, J = 4.1 Hz, 1H), 7.40 (d, J = 8.1 Hz, 2H), 7.37 (d, J = 4.1 Hz, 1H), 7.35 - 7.27 (m, 3H), 7.29 (d, J = 8.1 Hz, 2H), 6.90 (dt, J = 11.3, 2.4 Hz, 1H), 6.85 (dd, J = 7.9,2.1 Hz, 1H), 6.76 (td, J = 8.6, 2.4 Hz, 1H), 5.09 (s, 2H), 4.35 (dd, J = 15.4, 6.1 Hz, 1H), 4.27 (dd, J = 15.3, 5.8 Hz, 1H), 4.09 (t, J = 5.8 Hz, 1H), 3.52 (ddd, J = 9.4,6.5,4.3 Hz, 1H), 3.28-3.19 (m, 1H), 1.91 - 1.78 (m, 3H), 1.69-1.58 (m, 1H). |
| | (2S)-1-(5-chlorothiophene-2-sulfonyl)-N-({4-[(3-fluorophenoxy)methyl]phenyl} methyl)pyrrolidine-2-carboxamide | | |
| 56 | 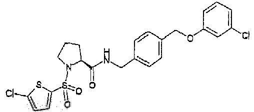 | 525 | ¹H NMR (400 MHz, DMSO) δ 8.60 (t, J = 6.0 Hz, 1H), 7.67 (d, J = 4.1 Hz, 1H), 7.39 (d, J = 8.2 Hz, 2H), 7.37 (d, J = 4.1 Hz, 1H), 7.30 (t, J = 8.2 Hz, 1H), 7.29 (d, J = 8.1 Hz, 2H), 7.09 (t, J = 2.2 Hz, 1H), 7.01 - 6.96 (m, 2H), 5.10 (s, 2H), 4.35 (dd, J = 15.3,6.2 Hz, 1H), 4.27 (dd, J = 15.4, 5.8 Hz, 1H), 4.08 (t, J = 5.8 Hz, 1H), 3.52 (ddd, J = 9.3, 6.5, 4.5 Hz, 1H), 3.28-3.19 (m, 1H), 1.92 - 1.79 (m, 3H), 1.69-1.57 (m, 1H). |
| | (2S)-N-({4-[(3-chlorophenoxy) methyl]phenyl}methyl)-1-(5-chlorothiophene-2-sulfonyl) pyrrolidine-2-carboxamide | | |

**Table 28**

| Example No. | structure compound name | MS(ESI) *m*/*z* (M+H)⁺ | NMR |
|---|---|---|---|
| 57 | 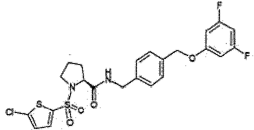 | 527 | ¹H NMR (400 MHz, DMSO) δ 8.61 (t, J = 6.0 Hz, 1H), 7.67 (d, J = 4.1 Hz, 1H), 7.40 (d, J = 8.2 Hz, 2H), 7.37 (d, J = 4.1 Hz, 1H), 7.30 (d, J = 8.2 Hz, 2H), 6.84-6.74 (m, 3H), 5.11 (s, 2H), 4.35 (dd, J = 15.4, 6.2 Hz, 1H), 4.27 (dd, J = 15.4, 5.8 Hz, 1H), 4.08 (t, J = 5.9 Hz, 1H), 3.52 (ddd, J = 9.5, 6.6,4.4 Hz, 1H), 3.28 - 3.19 (m, 1H), 1.89 - 1.79 (m, 3H), 1.68 -1.57 (m, 1H). |
| | (2S)-1-(5-chlorothiophene-2-sulfonyl)-N-({4-[(3,5-difluorophenoxy)methyl] phenyl)methyl)pyrrolidine-2-carboxamide | | |
| 58 | 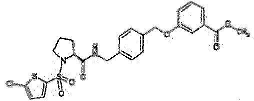 | 549 | ¹H NMR (400 MHz, DMSO) δ 8.60 (t, J = 6.0 Hz, 1H), 7.67 (d, J = 4.1 Hz, 1H), 7.55 (dd, J = 7.7,1.3 Hz, 1H), 7.55 - 7.52 (m, 1H), 7.47 - 7.39 (m, 3H), 7.37 (d, J = 4.1 Hz, 1H), 7.33 - 7.26 (m, 2H), 5.15 (s, 2H), 4.35 (dd, J = 15.8, 5.9 Hz, 1H), 4.27 (dd, J = 15.3,6.0 Hz, 1H), 4.09 (t, J = 5.8 Hz, 1H), 3.84 (s, 3H), 3.57 - 3.48 (m, 1H), 3.28 - 3.19 (m, 1H), 1.88 -1.80 (m, 3H), 1.68-1.57 (m, 1H). |
| | methyl 3-((4-({[(2S)-1-(5-chlorothiophene-2-sulfonyl) pyrrolidine-2-carbonyl]amino} methyl)phenyl)methoxy) benzoate | | |
| 59 | 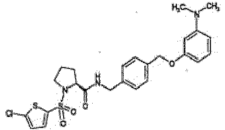 | 534 | ¹H NMR (400 MHz, DMSO) δ 8.60 (t, J = 6.0 Hz, 1H), 7.67 (d, J = 4.1 Hz, 1H), 7.39 (d, J = 8.2 Hz, 2H), 7.37 (d, J = 4.1 Hz, 1H), 7.28 (d, J = 8.2 Hz, 2H), 7.07 (t, J = 8.2 Hz, 1H), 6.35 (s, 3H), 5.04 (s, 2H), 4.35 (dd, J = 15.4, 6.3 Hz, 1H), 4.27 (dd, J = 15.3, 6.0 Hz, 1H), 4.09 (t, J = 5.9 Hz, 1H), 3.56 - 3.47 (m, 1H), 3.28 - 3.19 (m, 1H), 2.87 (s, 6H), 1.92 - 1.77 (m, 3H), 1.69 - 1.55 (m, 1H). |
| | (2S)-1-(5-chlorothiophene-2-sulfonyl)-N-((4-{[3-(dimethylamino)phenoxy] methyl}phenyl)methyl) pyrrolidine-2-carboxamide | | |
| 60 | 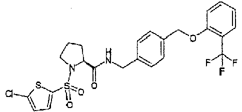 | 559 | ¹H NMR (400 MHz, DMSO) δ 8.60 (t, J = 6.0 Hz, 1H), 7.67 (d, J = 4.1 Hz, 1H), 7.65 - 7.58 (m, 2H), 7.37 (d, J = 4.1 Hz, 1H), 7.43 - 7.26 (m, 5H), 7.10 (t, J = 7.6 Hz, 1H), 5.25 (s, 2H), 4.36 (dd, J = 15.4, 6.2 Hz, 1H), 4.27 (dd, J = 15.6, 5.8 Hz, 1H), 4.09 (t, J = 5.8 Hz, 1H), 3.57 - 3.48 (m, 1H), 3.27 - 3.18 (m, 2H),1.92 -1.78 (m, 3H), 1.68 - 1.58 (m, 1H). |
| | (2S)-1-(5-chlorothiophene-2-sulfonyl)-N-((4-{[2-(trifluoromethyl)phenoxy] methyl}phenyl)methyl) pyrrolidine-2-carboxamide | | |

**Table 29**

| Example No. | structure compound name | MS(ESI) *m*/*z*(M+H)+ | NMR |
|---|---|---|---|
| 61 | 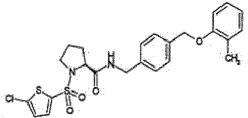 | 505 | ¹H NMR (400 MHz, DMSO) δ 8.60 (t, J = 5.8 Hz, 1H), 7.67 (d, J = 4.1 Hz, 1H), 7.41 (d, J = 8.1 Hz, 2H), 7.37 (d, J = 4.1 Hz, 1H), 7.29 (d, J = 8.1 Hz, 2H), 7.18-7.10 (m, 2H), 6.99 (d, J = 7.8 Hz, 1H), 6.84 (td, J = 7.5, 0.9 Hz, 1H), 5.09 (s, 2H), 4.36 (dd, J = 15.3, 6.2 Hz, 1H), 4.28 (dd, J = 15.3,5.8 Hz, 1H), 4.09 (t, J = 5.8 Hz, 1H), 3.53 (ddd, J = 9.5, 6.3, 4.1 Hz, 1H), 3.29 - 3.20 (m, 1H), 2.19 (s, 3H), 1.90-1.81 (m, 3H), 1.68 - 1.58 (m, 1H). |
| | (2S)-1-(5-chlorothiophene-2-sulfonyl)-N-({4-[(2-methylphenoxy)methyl] phenyl}methyl)pyrrolidine-2-carboxamide | | |
| 62 | 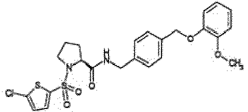 | 521 | ¹H NMR (400 MHz, DMSO) δ 8.61 (t, J = 6.0 Hz, 1H), 7.67 (d, J = 4.1 Hz, 1H), 7.39 (d, J = 9.0 Hz, 2H), 7.37 (d, J = 4.1 Hz, 1H), 7.29 (d, J = 8.1 Hz, 1H), 7.00 (ddd, J = 17.6, 7.7, 1.8 Hz, 1H), 6.88 (dtd, J = 17.8, 7.5, 1.7 Hz, 1H), 5.05 (s, 1H), 4.36 (dd, J = 15.4, 6.3 Hz, 1H), 4.27 (dd, J = 15.3, 5.8 Hz, 1H), 4.09 (t, J = 5.8 Hz, 1H), 3.76 (s, 2H), 3.54 (dt, J = 9.5, 7.7 Hz, 1H), 3.28 - 3.19 (m, 1H), 1.93 - 1.77 (m, 2H), 1.64 (dd, J = 10.8, 6.2 Hz, 1H). |
| | (2S)-1-(5-chlorothiophene-2-sulfonyl)-N-({4-[(2-methoxyphenoxy)methyl] phenyl}methyl)pyrrolidine-2-carboxamide | | |
| 63 | 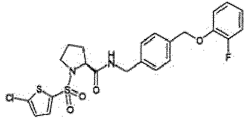 | 509 | ¹H NMR (400 MHz, DMSO) δ 8.60 (t, J = 6.0 Hz, 1H), 7.67 (d, J = 4.1 Hz, 1H), 7.40 (d, J = 8.1 Hz, 2H), 7.37 (d, J = 4.1 Hz, 1H), 7.29 (d, J = 8.2 Hz, 1H), 7.27-7.16 (m, 2H), 7.14 - 7.08 (m, 1H), 6.93 (tdd, J = 8.0, 4.7, 1.6 Hz, 1H), 5.15 (s, 2H), 4.35 (dd, J = 15.4, 6.3 Hz, 1H), 4.27 (dd, J = 15.4, 5.8 Hz, 1H), 4.09 (t, J = 5.9 Hz, 1H), 3.57 - 3.48 (m, 1H), 3.28 - 3.19 (m, 1H), 1.90 - 1.75 (m, 3H), 1.71 - 1.56 (m, 1H). |
| | (2S)-1-(5-chlorothiophene-2-sulfonyl)-N-({4-[(2-fluorophenoxy)methyl]phenyl} methyl)pyrrolidine-2-carboxamide | | |
| 64 | 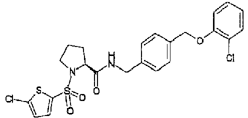 | 525 | ¹H NMR (400 MHz, DMSO) δ 8.61 (t, J = 6.0 Hz, 1H), 7.67 (d, J = 4.1 Hz, 1H), 7.44 (dd, J = 7.2, 2.3 Hz, 1H), 7.42 (d, J = 6.7 Hz, 2H), 7.37 (d, J = 4.1 Hz, 1H), 7.33 - 7.26 (m, 3H), 7.23 (dd, J = 8.3,1.5 Hz, 1H), 6.96 (dd, J = 7.6,1.3 Hz, 1H), 5.19 (s, 2H), 4.36 (dd, J = 15.3, 6.1 Hz, 1H), 4.28 (dd, J = 15.4, 5.7 Hz, 1H), 4.09 (t, J = 5.8 Hz, 1H), 3.57 - 3.48 (m, 1H), 3.28-3.19 (m, 1H), 1.86 (dd, J = 10.5, 6.2 Hz, 3H), 1.70 - 1.57 (m, 1H). |
| | (2S)-N-({4-[(2-chlorophenoxy) methyl]phenyl}methyl)-1-(5-chlorothiophene-2-sulfonyl) pyrrolidine-2-carboxamide | | |

**Table 30**

| Example No. | structure compound name | MS(ESI) m/z (M+H)⁺ | NMR |
|---|---|---|---|
| 65 | 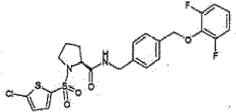 | 527 | ¹H NMR (400 MHz, DMSO) δ 8.60 (t, J = 6.1 Hz, 1H), 7.67 (d, J = 4.1 Hz, 1H), 7.37 (d, J = 4.1 Hz, 1H), 7.36 (d, J = 8.0 Hz, 2H), 7.27 (d, J = 8.2 Hz, 2H), 7.14-7.07 (m, 3H), 5.13 (s, 2H), 4.35 (dd, J = 15.5, 6.4 Hz, 1H), 4.27 (dd, J = 15.3, 5.9 Hz, 1H), 4.08 (t, J = 5.9 Hz, 1H), 3.52 (ddd, J = 9.3, 6.6, 4.4 Hz, 1H), 3.23 (dt, J = 9.8, 7.0 Hz, 1H), 1.89 - 1.79 (m, 3H), 1.68 - 1.57 (m, 1H). |
| | (2S)-1-(5-chlorothiophene-2-sulfonyl)-N-({4-[(2,6-difluorophenoxy)methyl] phenyl}methyl)pyrrolidine-2-carboxamide | | |
| 66 | 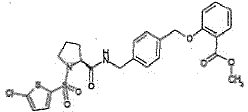 | 549 | - |
| | methyl 2-((4-({[(2S)-1-(5-chlorothiophene-2-sulfonyl) pyrrolidine-2-carbonyl]amino} methyl)phenyl)methoxy) benzoate | | |
| 67 | 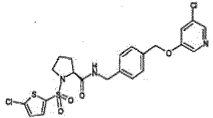 | 526 | - |
| | (2S)-N-({4-[(5-chloro-3-pyridyl) oxymethyl]phenyl}methyl)-1-(5-chlorothiophene-2-sulfonyl) pyrrolidine-2-carboxamide | | |
| 68 | 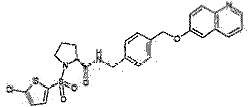 | 542 | ¹H NMR (400 MHz, DMSO) δ 8.87 (s, 1H), 8.62 (t, J = 6.1 Hz, 1H), 8.57 - 8.46 (m, 1H), 8.02 (d, J = 8.9 Hz, 1H), 7.71-7.65 (m, 1H), 7.67 (d, J = 4.1 Hz, 1H), 7.65 - 7.58 (m, 2H), 7.48 (d, J = 8.1 Hz, 2H), 7.37 (d, J = 4.1 Hz,1H), 7.32 (d, J = 8.1 Hz, 2H), 5.25 (s, 2H), 4.36 (dd, J = 15.4,6.2 Hz, 1H), 4.29 (dd, J = 15.5,5.8 Hz, 1H), 4.09 (t, J = 5.9 Hz, 1H), 3.56-3.54 (m, 1H), 3.29 - 3.19 (m, 1H), 1.90-1.78 (m, 3H), 1.70 - 1.56 (m, 1H). |
| | (2S)-1-(5-chlorothiophene-2-sulfonyl)-N-{[4-(6-quinolyloxymethyl)phenyl] methyl}pyrrolidine-2-carboxamide | | |

**Table 31**

| Example No. | structure compound name | MS(ESI) *m*/*z* (M+H)⁺ | NMR |
|---|---|---|---|
| 69 | 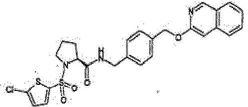 | 542 | ¹H NMR (400 MHz, DMSO) δ 9.07 (s, 1H), 8.59 (t, J = 6.0 Hz, 1H), 8.03 (dd, J = 8.3, 0.8 Hz, 1H), 7.81 (d, J = 7.8 Hz, 1H), 7.69 - 7.62 (m, 2H), 7.47 - 7.40 (m, 3H), 7.36 (d, J = 4.1 Hz, 1H), 7.32 - 7.24 (m, 3H), 5.42 (s, 2H), 4.34 (dd, J = 15.3, 6.3 Hz, 1H), 4.26 (dd, J = 15.3, 5.8 Hz, 1H), 4.08 (t, J = 5.9 Hz, 1H), 3.55 - 3.54 (m, 1H), 3.29 - 3.18 (m, 1H), 1.91 - 1.77 (m, 3H), 1.69 - 1.56 (m, 1H). |
| | (2S)-1-(5-chlorothiophene-2-sulfonyl)-N-{[4-(3-isoquinolyloxymethyl)phenyl] methyl}pyrrolidine-2-carboxamide | | |
| 70 | 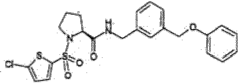 | 491 | - |
| | (2S)-1-(5-chlorothiophene-2-sulfonyl)-N-{[3-(phenoxymethyl)phenyl] methyl}pyrrolidine-2-carboxamide | | |
| 71 | 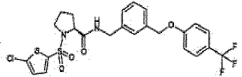 | 559 | - |
| | (2S)-1-(5-chlorothiophene-2-sulfonyl)-N-((3-{[4-(trifluoromethyl)phenoxyl] methyl}phenyl)methyl) pyrrolidine-2-carboxamide | | |
| 72 | 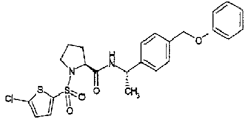 | 505 | ¹H NMR (400 MHz, DMSO) δ 8.40 (d, J = 8.1 Hz, 1H), 7.66 (d, J = 4.1 Hz, 1H), 7.40 (d, J = 8.2 Hz, 2H), 7.36 (t, J = 3.6 Hz, 1H), 7.33 (d, J = 8.2 Hz, 2H), 7.32-7.24 (m, 2H), 7.00 (d, J = 7.9 Hz, 2H), 6.93 (t, J = 7.3 Hz, 1H), 5.07 (s, 2H), 4.94 (p, J = 7.0 Hz, 1H), 4.10 (dd, J = 7.8,3.8 Hz, 1H), 3.55 - 3.43 (m, 1H), 3.23 (dt, J = 10.0, 6.8 Hz, 1H), 1.92 - 1.71 (m, 3H), 1.71 - 1.52 (m, 1H), 1.39 (d, J = 7.0 Hz, 3H). |
| | (2S)-1-(5-chlorothiophene-2-sulfenyl)-N-{(15)-1-[4-(phenoxymethyl)phenyl]ethyl} pyrrolidine-2-carboxamide | | |

**Table 32**

| Example No. | structure compound name | MS(ESI) *m*/*z* (M+H)⁺ | NMR |
|---|---|---|---|
| 73 | 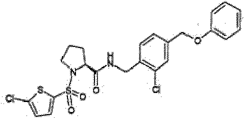 | 525 | ¹H NMR (400 MHz, DMSO) δ 8.64 (t, J = 5.9 Hz, 1H), 7.69 (d, J = 4.1 Hz, 1H), 7.52 (s, 1H), 7.41 - 7.36 (m, 3H), 7.33-7.26 (m, 2H), 7.03 - 6.97 (m, 2H), 6.94 (tt, J = 7.3, 1.0 Hz, 1H), 5.11 (s, 2H), 4.41 (dd, J = 16.0, 6.2 Hz, 1H), 4.30 (dd, J = 16.0, 5.7 Hz, 1H), 4.12 (t, J = 5.9 Hz, 1H), 3.59 - 3.48 (m, 1H), 3.30 - 3.20 (m, 1H), 1.91 - 1.82 (m, 3H), 1.69 -1.60 (m, 1H). |
| | (2S)-N-{[2-chloro-4-(phenoxymethyl)phenyl] methyl}-(5-chlorothiophene-2-sulfonyl)pyrrolidine-2-carboxamide | | |
| 74 | 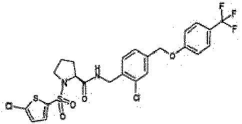 | 593 | ¹H NMR (400 MHz, DMSO) δ 8.65 (t, J = 5.9 Hz, 1H), 7.69 (d, J = 4.1 Hz, 1H), 7.66 (d, J = 8.7 Hz, 2H), 7.55 (s, 1H), 7.42 - 7.36 (m, 2H), 7.38 (d, J = 4.1 Hz, 1H), 7.20 (d, J = 8.6 Hz, 2H), 5.21 (s, 2H), 4.41 (dd, J = 16.0, 6.2 Hz, 1H), 4.31 (dd, J = 15.9, 5.7 Hz, 1H), 4.12 (t, J = 5.9 Hz, 1H), 3.58 - 3.48 (m, 1H), 3.30 - 3.18 (m, 1H), 1.93 -1.81 (m, 3H), 1.71 - 1.59 (m, 1H). |
| | (2S-1-(5-chlorothiophene-2-sulfonyl))-N-((2-chloro-4-{[4-(forifluoromethyl)phenoxy] methyl}phenyl)methyl) pyrrolidine-2-carboxamide | | |
| 75 | 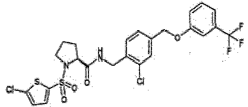 | 593 | ¹H NMR (400 MHz, DMSO) δ 8.65 (t, J = 5.9 Hz, 1H), 7.69 (d, J = 4.1 Hz, 1H), 7.58 - 7.50 (m, 2H), 7.44 - 7.36 (m, 2H), 7.38 (d, J = 4.1 Hz, 1H), 7.36 - 7.28 (m, 3H), 5.20 (s, 2H), 4.42 (dd, J = 15.9, 6.2 Hz, 1H), 4.31 (dd, J = 16.1, 5.7 Hz, 1H), 4.12 (t, J = 5.9 Hz, 1H), 3.57 - 3.49 (m, 1H), 3.29 - 3.21 (m, 1H), 1.93 - 1.81 (m, 3H), 1.70 - 1.59 (m, 1H). |
| | (2S-1-(5-chlorothiophene-2-sulfonyl))-N-((2-chloro-4-{[3-(forifluoromethyl)phenoxy] methyl}phenyl)methyl) pyrrolidine-2-carboxamide | | |
| 76 | 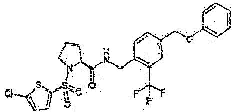 | 559 | ¹H NMR (400 MHz, DMSO) δ 8.74 (t, J = 5.9 Hz, 1H), 7.79 (s, 1H), 7.71 (d, J = 8.8 Hz, 1H), 7.70 (d, J = 4.1 Hz, 1H), 7.56 (d, J = 8.0 Hz, 1H), 7.38 (d, J = 4.1 Hz, 1H), 7.35 - 7.25 (m, 2H), 7.02 (d, J = 7.9 Hz, 2H), 6.95 (t, J = 7.3 Hz, 1H), 5.19 (s, 2H), 4.54 (dd, J = 16.1, 5.8 Hz, 1H), 4.43 (dd, J = 16.1, 5.4 Hz, 1H), 4.13 (t, J = 5.9 Hz, 1H), 3.58 - 3.51 (m, 1H), 3.25 (dt, J = 9.7,6.8 Hz, 1H), 1.96 -1.82 (m, 3H), 1.71 -1.59 (m, 1H). |
| | (2S)-1-(5-chlorothiophene-2-sulfonyl)-N-{[4-(phenoxymethyl)-2-(trifluoromethyl)phenyl] methyl}pyrrolidine-2-carboxamide | | |

**Table 33**

| Example No. | structure compound name | MS(ESI) *m*/*z*(M+H)⁺ | NMR |
|---|---|---|---|
| 77 | 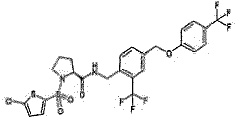 | 627 | ¹H NMR (400 MHz, DMSO) δ 8.75 (t, J = 5.9 Hz, 1H), 7.82 (s, 1H), 7.74 (d, J = 8.2 Hz, 1H), 7.70 (d, J = 4.1 Hz, 1H), 7.67 (d, J = 8.6 Hz, 2H), 7.58 (d, J = 8.0 Hz, 1H), 7.38 (d, J = 4.1 Hz, 1H), 7.22 (d, J = 8.6 Hz, 2H), 5.29 (s, 2H), 4.54 (dd, J = 16.2, 6.0 Hz, 1H), 4.43 (dd, J = 16.2, 5.3 Hz, 1H), 4.13 (t, J = 6.0 Hz, 1H), 3.59 - 3.51 (m, 1H), 3.26 (dt, J = 9.7,6.8 Hz, 1H), 1.97 - 1.81 (m, 3H), 1.70 - 1.58 (m, 1H). |
| | (2S)-1-(5-chlorothiophene-2-sulfonyl)-N-((2-(trifluoromethyl)-4-{[4-(trifluoromethyl)phenoxy] methyl}phenyl)methyl) pyrrolidine-2-carboxamide | | |
| 78 | 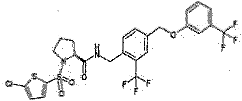 | 627 | ¹H NMR (400 MHz, DMSO) δ 8.74 (t, J = 5.9 Hz, 1H), 7.83 (s, 1H), 7.74 (d, J = 7.9 Hz, 1H), 7.70 (d, J = 4.1 Hz, 1H), 7.62-7.50 (m, 2H), 7.38 (d, J = 4.1 Hz, 1H), 7.42 - 7.28 (m, 3H), 5.28 (s, 2H), 4.54 (dd, J = 16.4, 6.0 Hz, 1H), 4.43 (dd, J = 16.2, 5.6 Hz, 1H), 4.13 (t, J = 5.9 Hz, 1H), 3.60 - 3.50 (m, 1H), 3.31 - 3.20 (m, 1H), 1.94-1.83 (m, 3H), 1.70 - 1.57 (m, 1H). |
| | (2S)-1-(5-chlorothiophene-2-sulfonyl)-N-((2-(trifluoromethyl)-4-{[3-(trifluoromethyl)phenoxy] methyl}phenyl)methyl) pyrrolidine-2-carboxamide | | |
| 79 | 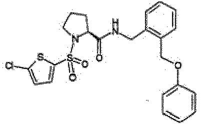 | 491 | ¹H NMR (400 MHz, DMSO) δ 8.55 (t, J = 5.8 Hz, 1H), 7.66 (d, J = 4.1 Hz, 1H), 7.46 (dd, J = 7.3,1.2 Hz, 1H), 7.39 - 7.26 (m, 6H), 7.07 - 7.01 (m, 2H), 6.96 (tt, J = 7.3, 1.0 Hz, 1H), 5.16 (s, 2H), 4.45 (dd, J = 15.5,6.2 Hz, 1H), 4.36 (dd, J = 15.5, 5.8 Hz, 1H), 4.10 (t, J = 5.8 Hz, 1H), 3.56 - 3.47 (m, 1H), 3.28 - 3.19 (m, 1H), 1.91-1.78 (m, 3H), 1.66 - 1.55 (m, 1H). |
| | (2S)-1-(5-chlorothiophene-2-sulfonyl)-N-{[2-(phenoxymethyl) phenyl]methyl}pyrrolidine-2-carboxamide | | |
| 80 | 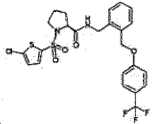 | 559 | ¹H NMR (400 MHz, DMSO) δ 8.58 (t, J = 5.8 Hz, 1H), 7.67 (d, J = 8.6 Hz, 2H), 7.66 (d, J = 4.0 Hz, 1H), 7.48 (dd, J = 7.3, 1.1 Hz, 1H), 7.42 - 7.27 (m, 4H), 7.24 (d, J = 8.6 Hz, 2H), 5.27 (s, 2H), 4.45 (dd, J = 15.5, 6.0 Hz, 1H), 4.37 (dd, J = 15.5,5.7 Hz, 1H), 4.10 (dd, J = 7.3, 4.3 Hz, 1H), 3.55 - 3.48 (m, 1H), 3.23 (dt, J = 10.0, 6.7 Hz, 1H), 1.89 - 1.78 (m, 3H), 1.67 - 1.56 (m, 1H). |
| | (2S)-1-(5-chlorothiophene-2-sulfonyl)-N-((2-{[4-(trifluoromethyl)phenoxy] methyl}phenyl)methyl) pyrrolidine-2-carboxamide | | |

**Table 34**

| Example No. | structure compound name | MS(ESI) *m*/*z*(M+H)⁺ | NMR |
|---|---|---|---|
| 81 | 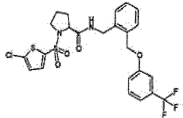 | 559 | ¹H NMR (400 MHz, DMSO) δ 8.56 (t, J = 5.9 Hz, 1H), 7.66 (d, J = 4.1 Hz, 1H), 7.55 (t, J = 7.9 Hz, 1H), 7.49 (dd, J = 7.0,1.2 Hz, 1H), 7.41 - 7.27 (m, 7H), 5.26 (s, 2H), 4.47 (dd, J = 15.6, 6.1 Hz, 1H), 4.37 (dd, J = 15.6,5.6 Hz, 1H), 4.10 (dd, J = 7.3, 4.4 Hz, 1H), 3.55 - 3.46 (m, 1H), 3.23 (dt, J = 10.0, 6.7 Hz, 1H), 1.89 - 1.78 (m, 3H), 1.67 -1.56 (m, 1H). |
| | (2S)-1-(5-chlorothiophene-2-sulfonyl)-N-((2-{[3-(trifluoromethyl)phenoxy] methyl}phenyl)methyl) pyrrolidine-2-carboxamide | | |

### Example 82: Synthesis of (2S)-1-[(4-fluorophenyl)sulfonyl]-N-{[4-(phenoxymethyl)phenyl]methyl}pyrrolidine-2-carboxamide (82)

To A-6 (0.027 g, 0.10 mmol) and C-5 (0.033 g, 0.10 mmol), WSC hydrochloride (0.037 g, 0.20 mmol) and HOAt (0.027 g, 0.20 mmol) were added triethylamine (42 µL, 0.30 mmol) and dichloromethane (1 mL), and the mixture was stirred at room temperature for several hours. The solvent was evaporated, and the obtained residue was purified by high performance liquid chromatography (water-acetonitrile, each containing 0.1% trifluoroacetic acid) to give the title compound (0.010 g, 0.021 mmol, 21%).
MS(ESI) m/z 469 (M+H)⁺
¹H NMR (400 MHz, DMSO) δ 8.56 (t, J = 6.0 Hz, 1H), 7.98 - 7.90 (m, 2H), 7.52 - 7.43 (m, 2H), 7.40 (d, J = 8.2 Hz, 2H), 7.32-7.24 (m, 4H), 7.04 - 6.97 (m, 2H), 6.93 (tt, J = 7.3, 1.0 Hz, 1H), 5.08 (s, 2H), 4.35 (dd, J = 15.3, 6.2 Hz, 1H), 4.27 (dd, J = 15.3, 5.8 Hz, 1H), 4.08 (dd, J = 8.0, 3.8 Hz, 1H), 3.50-3.42 (m, 1H), 3.18 (dt, J = 9.9, 6.9 Hz, 1H), 1.87 - 1.68 (m, 3H), 1.57 - 1.48 (m, 1H).

Compounds 83 - 90 described in Table 35 - Table 37 were synthesized using the corresponding A-1 and A-2, A-4, A-6 - A-9, C-5, C-8 - C-10, and commercially available reagents and by an operation similar to Example 82.

**Table 35**

| Example No. | structure compound name | MS(ESI) *m*/*z*(M+H)⁺ | NMR |
|---|---|---|---|
| 82 | 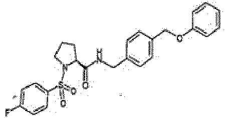 | 469 | ¹H NMR (400 MHz, DMSO) δ 8.56 (t, J = 6.0 Hz, 1H), 7.98 - 7.90 (m, 2H), 7.52-7.43 (m, 2H), 7.40 (d, J = 8.2 Hz, 2H), 7.32 - 7.24 (m, 4H), 7.04 - 6.97 (m, 2H), 6.93 (tt, J = 7.3, 1.0 Hz, 1H), 5.08 (s, 2H), 4.35 (dd, J = 15.3, 6.2 Hz, 1H), 4.27 (dd, J = 15.3, 5.8 Hz, 1H), 4.08 (dd, J = 8.0, 3.8 Hz, 1H), 3.50 - 3.42 (m, 1H), 3.18 (dt, J = 9.9, 6.9 Hz, 1H), 1.87 - 1.68 (m, 3H), 1.57-1.48 (m, 1H). |
| | (2S)-1-[(4-fluorophenyl) sulfonyl]-N-{[4-(phenoxymethyl)phenyl] methyl}pyrrolidine-2-carboxamide | | |
| 83 | 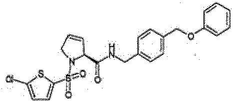 | 489 | - |
| | (2S)-1-(5-chlorothiophene-2-sulfonyl)-N-{[4-(phenoxyymethyl)phenyl] methyl}-2,5-dihydropyrrole-2-carboxamide | | |
| 84 | 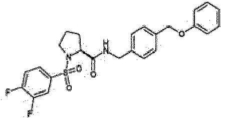 | 487 | - |
| | (2S)-1-[(3,4-difluorophenyl) sulfonyl]-N-{[4-(phenoxymethyl)phenyl] methyl}pyrrolidine-2-carboxamide | | |
| 85 | 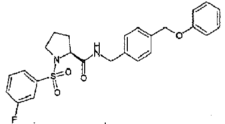 | 469 | - |
| | (2S)-1-[(3-fluorophenyl) sulfonyl]-N-{[4-(phenoxymethyl)phenyl] methyl}pyrrolidine-2-carboxamide | | |

**Table 36**

| Example No. | structure compound name | MS(ESI) *m*/*z*(M+H)⁺ | NMR |
|---|---|---|---|
| 86 | 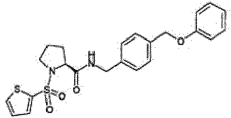 | 457 | - |
| | (2S)-N-{[4-(phenoxymethyl) phenyl]methyl}-1-(2-thienylsulfonyl)pyrrolidine-2-carboxamide | | |
| 87 | 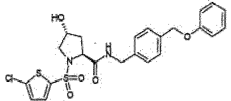 | 507 | ¹H NMR (400 MHz, DMSO) δ 8.65 (t, J = 6.0 Hz, 1H), 7.60 (d, J = 4.1 Hz, 1H), 7.39 (d, J = 8.2 Hz, 2H), 7.32 (d, J = 4.1 Hz, 1H), 7.31 - 7.25 (m, 4H), 6.99 (dd, J = 8.8, 1.0 Hz, 2H), 6.95 - 6.90 (m, 1H), 5.07 (s, 2H), 4.87 (d, J = 2.9 Hz, 1H), 4.31 (d, J = 5.9 Hz, 2H), 4.26 - 4.20 (m, 1H), 4.14 (t, J = 7.9 Hz, 1H), 3.58 (dd, J = 11.2,3.9 Hz, 1H), 3.19 (d, J = 11.2 Hz, 1H), 2.04 - 1.86 (m, 2H). |
| | (2S,4R)-1-(5-chlorothiophene-2-sulfonyl)-4-hydroxy-N-{[4-(phenoxymethyl)phenyl] methyl}pyrrolidine-2-carboxamide | | |
| 88 | 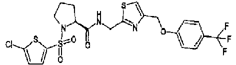 | 566 | - |
| | (2S)-1-(5-chlorothiophene-2-sulfonyl)-N-((4-{[4-(trifluoromethyl)phenoxy] methyl}thiazol-2-yl)methyl) pyrrolidine-2-carboxamide | | |
| 89 | 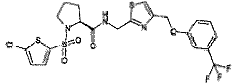 | 566 | ¹H NMR (400 MHz, DMSO) δ 9.01 (dd, J = 6.1, 5.9 Hz, 1H), 7.72 - 7.65 (m, 2H), 7.54 (t, J = 7.8 Hz, 1H), 7.38 (d, J = 4.1 Hz, 1H), 7.37 - 7.33 (m, 2H), 7.31 (d, J = 7.5 Hz, 1H), 5.21 (s, 2H), 4.60 (dd, J = 16.6, 6.1 Hz, 1H), 4.55 (dd, J = 16.6, 5.9 Hz, 1H), 4.12 (t, J = 5.6 Hz, 1H), 3.57 - 3.49 (m, 1H), 3.28 - 3.19 (m, 1H), 1.93 -1.79 (m, 3H), 1.72 - 1.60 (m, 1H). |
| | (2S)-1-(5-chlorothiophene-2-sulfonyl)-N-((4-{[3-(trifluoromethyl)phenoxy] methyl}thiazol-2-yl)methyl) pyrrolidine-2-carboxamide | | |

**Table 37**

| Example No. | structure compound name | MS(ESI) *m*/*z*(M+H)⁺ | NMR |
|---|---|---|---|
| 90 | 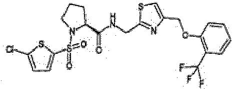 | 566 | ¹H NMR (400 MHz, DMSO) δ 9.01 (t, J = 6.1 Hz, 1H), 7.68 (d, J = 4.1 Hz, 1H), 7.67 - 7.61 (m, 2H), 7.56 (s, 1H), 7.42 (d, J = 8.8 Hz, 1H), 7.38 (d, J = 4.1 Hz, 1H), 7.12 (t, J = 7.6 Hz, 1H), 5.29 (s, 2H), 4.60 (dd, J = 16.2,6.1 Hz, 1H), 4.55 (dd, J = 16.2, 6.1 Hz, 1H), 4.12 (dd, J = 6.0, 5.5 Hz, 1H), 3.53 (ddd, J = 10.0, 6.6, 4.3 Hz, 1H), 3.24 (ddd, J = 11.6, 10.0, 5.3 Hz, 1H), 1.92 - 1.82 (m, 3H), 1.71 - 1.61 (m, 1H). |
| | (2S)-1-(5-chlorothiophene-2-sulfonyl)-N-((4-{[2-(trifluoromethyl)phenoxy] methyl}thiazol-2-yl)methyl) pyrrolidine-2-carboxamide | | |

### Example 91: Synthesis of (2S)-1-(5-chlorothiophene-2-sulfonyl)-N-((3-{[3-(trifluoromethyl)phenyl]methoxy}phenyl)methyl)pyrrolidine-2-carboxamide (91)

B-1 (40 mg, 0.10 mmol), benzyl alcohol (12 µL, 0.12 mmol) and triphenylphosphine (39 mg, 0.15 mmol) were dissolved in dichloromethane (1 mL), diisopropyl azodicarboxylate (32 µL, 0.15 mmol) was added dropwise, and the mixture was stirred for several hours. The solvent was evaporated, and the obtained residue was purified by high performance liquid chromatography (water-acetonitrile, each containing 0.1% trifluoroacetic acid) to give the title compound (21 mg, 0.042 mmol, 42%).
MS(ESI) m/z 491 (M+H)⁺
¹H NMR (400 MHz, DMSO) δ 8.50 (t, J = 5.9 Hz, 1H), 7.66 (d, J = 4.1 Hz, 1H), 7.44 (d, J = 7.0 Hz, 2H), 7.41 - 7.35 (m, 2H), 7.36 (d, J = 4.0 Hz, 1H), 7.35 - 7.29 (m, 1H), 7.19 (d, J = 8.5 Hz, 2H), 6.99 - 6.93 (m, 2H), 5.09 (s, 2H), 4.27 (dd, J = 15.0, 6.1 Hz, 1H), 4.19 (dd, J = 15.0, 5.7 Hz, 1H), 4.08 (t, J = 5.8 Hz, 1H), 3.55 - 3.47 (m, 1H), 3.23 (dt, J = 10.0, 7.0 Hz, 1H), 1.89 - 1.77 (m, 3H), 1.68 - 1.55 (m, 1H).

Compounds 92 - 106 described in Table 38 - Table 41 were synthesized using the corresponding B-1 and B-2, B-8 and B-9, and commercially available reagents and by an operation similar to Example 91.

**Table 38**

| Example No. | structure compound name | MS(ESI) *m*/*z*(M+H)⁺ | NMR |
|---|---|---|---|
| 91 | 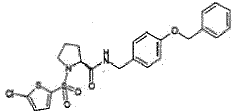 | 491 | ¹H NMR (400 MHz, DMSO) δ 8.50 (t, J = 5.9 Hz, 1H), 7.66 (d, J = 4.1 Hz, 1H), 7.44 (d, J = 7.0 Hz, 2H), 7.41 - 7.35 (m, 2H), 7.36 (d, J = 4.0 Hz, 1H), 7.35 - 7.29 (m, 1H), 7.19 (d, J = 8.5 Hz, 2H), 6.99 - 6.93 (m, 2H), 5.09 (s, 2H), 4.27 (dd, J = 15.0, 6.1 Hz, 1H), 4.19 (dd, J = 15.0, 5.7 Hz, 1H), 4.08 (t, J = 5.8 Hz, 1H), 3.55 - 3.47 (m, 1H), 3.23 (dt, J = 10.0, 7.0 Hz, 1H), 1.89 - 1.77 (m, 3H), 1.68 - 1.55 (m, 1H). |
| | (2S)-N-[4-(benzyloxyphenyl) methyl]-1-(5-chlorothiophene-2-sulfonyl)pyrrolidine-2-carboxamide | | |
| 92 | 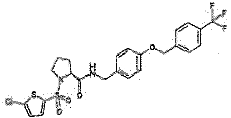 | 559 | ¹H NMR (400 MHz, DMSO) δ 8.51 (t, *J* = 6.0 Hz, 1H), 7.76 (d, *J* = 8.2 Hz, 2H), 7.66 (d, *J* = 4.1 Hz, 1H), 7.65 (d, *J* = 6.5 Hz, 2H), 7.36 (d, *J* = 4.1 Hz, 1H), 7.20 (d, *J* = 8.7 Hz, 2H), 7.00 - 6.94 (m, 2H), 5.22 (s, 2H), 4.27 (dd, J = 15.0, 6.2 Hz, 1H), 4.19 (dd, *J* = 15.0, 5.7 Hz, 1H), 4.07 (t, *J* = 5.8 Hz, 1H), 3.55 - 3.47 (m, 1H), 3.23 (dt, *J* = 9.9,6.9 Hz, 1H), 1.88 - 1.76 (m, 3H), 1.68 - 1.55 (m, 1H), |
| | (2S)-1-(5-chlorothiophene-2-sulfonyl)-N-((4-{[4-(trifluoromethyl)phenyl] methoxy}phenyl)methyl) pyrrolidine-2-carboxamide | | |
| 93 | 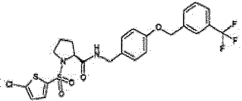 | 559 | - |
| | (2S)-1-(5-chlorothiophene-2-sulfonyl)-N-((4-{[3-(trifluoromethyl)phenyl] methoxy}phenyl)methyl) pyrrolidine-2-carboxamide | | |
| 94 | 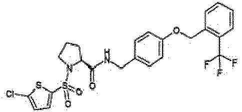 | 559 | ¹H NMR (400 MHz, DMSO) δ 8.52 (t, J = 5.9 Hz, 1H), 7.79 (d, J = 7.8 Hz, 1H), 7.77 - 7.68 (m, 2H), 7.66 (d, J = 4.1 Hz, 1H), 7.58 (t, J = 7.4 Hz, 1H), 7.36 (d, J = 4.1 Hz, 1H), 7.21 (d, J = 8.7 Hz, 2H), 7.00 - 6.93 (m, 2H), 5.22 (s, 2H), 4.28 (dd, J = 15.0, 6.1 Hz, 1H), 4.20 (dd, J = 15.0, 5.7 Hz, 1H), 4.08 (t, J = 5.8 Hz, 1H), 3.55 - 3.48 (m, 1H), 3.23 (dt, J = 9.9, 7.0 Hz, 1H), 1.88 - 1.79 (m, 3H), 1.66 - 1.57 (m, 1H). |
| | (2S)-1-(5-chlorothiophene-2-sulfonyl)-N-((4-{[2-(trifluoromethyl)phenyl] methoxy}phenyl)methyl) pyrrolidine-2-carboxamide | | |

**Table 39**

| Example No. | structure compound name | MS(ESI) *m*/*z*(M+H)⁺ | NMR |
|---|---|---|---|
| 95 | 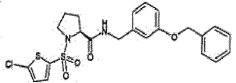 | 491 | ¹H NMR (400 MHz, DMSO) δ 8.59 (t, J = 6.0 Hz, 1H), 7.67 (d, J = 4.1 Hz, 1H), 7.46-7.35 (m, 5H), 7.35 - 7.29 (m, 1H), 7.23 (t, J = 7.9 Hz, 1H), 6.97 - 6.94 (m, 1H), 6.89 - 6.83 (m, 2H), 5.08 (s, 2H), 4.33 (dd, J = 15.4, 6.3 Hz, 1H), 4.25 (dd, J = 15.5,5.7 Hz, 1H), 4.09 (dd, J = 7.3, 4.4 Hz, 1H), 3.56 - 3.49 (m, 1H), 3.23 (dt, J = 10.1, 6.5 Hz, 1H), 1.90 - 1.79 (m, 3H), 1.67 - 1.59 (m, 1H). |
| | 2S)-N-[(3-benzyloxyphenyl) methyl]-1-(5-chlorothiophene-2-sulfonyl)pyrrolidine-2-carboxamide | | |
| 96 | 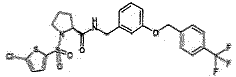 | 559 | ¹H NMR (400 MHz, DMSO) δ 8.59 (t, J = 6.1 Hz, 1H), 7.75 (d, J = 8.1 Hz, 2H), 7.68 (d, J = 4.1 Hz, 1H), 7.65 (d, J = 8.0 Hz, 2H), 7.37 (d, J = 4.1 Hz, 1H), 7.24 (t, J = 7.9 Hz, 1H), 6.98 - 6.95 (m, 1H), 6.91 - 6.85 (m, 2H), 5.21 (s, 2H), 4.34 (dd, J = 15.4, 6.2 Hz, 1H), 4.25 (dd, J = 15.6, 5.8 Hz, 1H), 4.09 (dd, J = 7.4, 4.3 Hz, 1H), 3.56 - 3.47 (m, 1H), 3.28 - 3.19 (m, 1H), 1.90 - 1.79 (m, 3H), 1.67 - 1.57 (m 1H). |
| | (2S)-1-(5-chlorothiophene-2-sulfonyl)-N-((3-{[4-(trifluoromethyl)phenyl] methoxy}phenyl)methyl) pyrrolidine-2-carboxamide | | |
| 97 | 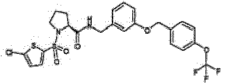 | 575 | ¹H NMR (400 MHz, DMSO) δ 8.59 (t, J = 5.9 Hz, 1H), 7.67 (d, J = 4.1 Hz, 1H), 7.56 (d, J = 8.7 Hz, 2H), 7.38 (d, J = 7.9 Hz, 2H), 7.37 (d, J = 4.1 Hz, 1H), 7.24 (t, J = 7.9 Hz, 1H), 6.97 - 6.94 (m, 1H), 6.90 - 6.84 (m, 2H), 5.12 (s, 2H), 4.34 (dd, J = 15.7,6.3 Hz, 1H), 4.25 (dd, J = 15.5, 6.0 Hz, 1H), 4.09 (dd, J = 7.3, 4.4 Hz, 1H), 3.56 - 3.48 (m, 1H), 3.24 (dt, J = 10.5,6.7 Hz, 1H), 1.92 - 1.79 (m, 3H), 1.69 - 1.57 (m, 1H). |
| | (2S)-1-(5-chlorothiophene-2-sulfonyl)-N-((3-{[4-(trifluoromethoxy)phenyl] methoxy}phenyl)methyl) pyrrolidine-2-carboxamide | | |
| 98 | 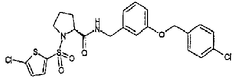 | 525 | ¹H NMR (400 MHz, DMSO) δ 8.59 (t, J = 6.0 Hz, 1H), 7.67 (d, J = 4.1 Hz, 1H), 7.45 (s, 4H), 7.37 (d, J = 4.1 Hz, 1H), 7.23 (t, J = 7.9 Hz, 1H), 6.95 - 6.92 (m, 1H), 6.89 - 6.83 (m, 2H), 5.09 (s, 2H), 4.33 (dd, J = 15.4, 6.2 Hz, 1H), 4.24 (dd, J = 15.5, 5.8 Hz, 1H), 4.09 (dd, J = 7.4, 4.3 Hz, 1H), 3.53 - 3.49 (m, 1H), 3.23 (dt, J = 10.1, 6.8 Hz, 1H), 1.91-1.78 (m, 3H), 1.68 - 1.57 (m, 1H). |
| | (2S)-N-({3-[(4-chlorophenyl) methoxy]phenyl}methyl)-1-(5-chlorothiophene-2-sulfonyl) pyrrolidine-2-carboxamide | | |

**Table 40**

| Example No. | structure compound name | MS(ESI) *m*/*z*(M+H)⁺ | NMR |
|---|---|---|---|
| 99 | 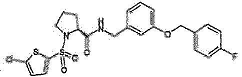 | 509 | ¹H NMR (400 MHz, DMSO) δ 8.59 (t, J = 6.0 Hz, 1H), 7.67 (d, J = 4.1 Hz, 1H), 7.51-7.45 (m, 2H), 7.37 (d, J = 4.1 Hz, 1H), 7.26 - 7.18 (m, 3H), 6.96 - 6.93 (m, 1H), 6.89-6.84 (m, 2H), 5.06 (s, 2H), 4.33 (dd, J = 15.3, 6.1 Hz, 1H), 4.25 (dd, J = 15.5, 5.8 Hz, 1H), 4.09 (dd, J = 7.2,4.5 Hz, 1H), 3.56 - 3.48 (m, 1H), 3.28 - 3.19 (m, 1H), 1.91 - 1.78 (m, 3H), 1.67 - 1.56 (m, 1H). |
| | (2S)-1-(5-chlorothiophene-2-sulfonyl)-N-({3-[(4-trifluorophenyl)methoxy] phenyl}methyl)pyrrolidine-2-carboxamide | | |
| 100 | 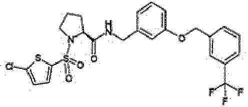 | 559 | ¹H NMR (400 MHz, DMSO) δ 8.60 (t, J = 6.0 Hz, 1H), 7.83 - 7.60 (m, 5H), 7.37 (d, J = 4.1 Hz, 1H), 7.25 (t, J = 7.8 Hz, 1H), 6.98 (s, 1H), 6.94 - 6.84 (m, 2H), 5.20 (s, 2H), 4.34 (dd, J = 15.6, 6.0 Hz, 1H), 4.25 (dd, J = 15.2, 5.8 Hz, 1H), 4.09 (dd, J = 7.5, 4.0 Hz, 1H), 3.56 - 3.47 (m, 1H), 3.28 - 3.19 (m, 1H), 1.91 - 1.78 (m, 3H), 1.67 - 1.57 (m, 1H). |
| | (2S)-1-(5-chlorothiophene-2-sulfonyl)-N-((3-{[3-(trifluoromethyl)phenyl] methoxy}phenyl)methyl) pyrrolidine-2-carboxamide | | |
| 101 | 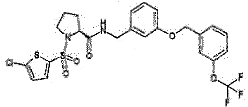 | 575 | ¹H NMR (400 MHz, DMSO) δ 8.60 (t, J = 6.0 Hz, 1H), 7.68 (d, J = 4.1 Hz, 1H), 7.53 (t, J = 7.9 Hz, 1H), 7.47 (d, J = 7.7 Hz, 1H), 7.42 (s, 1H), 7.37 (d, J = 4.1 Hz, 1H), 7.32 (dt, J = 8.0, 0.9 Hz, 1H), 7.24 (t, J = 7.9 Hz, 1H), 6.97 (s, 1H), 6.92 - 6.85 (m, 2H), 5.15 (s, 2H), 4.34 (dd, J = 15.5, 6.2 Hz, 1H), 4.25 (dd, J = 15.5, 5.8 Hz, 1H), 4.09 (dd, J = 7.2, 4.3 Hz, 1H), 3.56 - 3.49 (m, 1H), 3.24 (dt, J = 10.4, 6.7 Hz, 1H), 1.91 - 1.78 (m, 3H), 1.68 - 1.56 (m, 1H). |
| | (2S)-1-(5-chlorothiophene-2-sulfonyl)-N-((3-{[3-(trifluoromethoxy)phenyl] methoxy}phenyl)methyl) pyrrolidine-2-carboxamide | | |
| 102 | 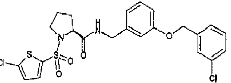 | 525 | ¹H NMR (400 MHz, DMSO) δ 8.59 (t, J = 6.0 Hz, 1H), 7.68 (d, J = 4.1 Hz, 1H), 7.49 (s, 1H), 7.43 - 7.35 (m, 4H), 7.24 (t, J = 7.9 Hz, 1H), 6.97 - 6.93 (m, 1H), 6.90 - 6.84 (m, 2H), 5.11 (s, 2H), 4.33 (dd, J = 15.5,6.2 Hz, 1H), 4.25 (dd, J = 15.5, 5.9 Hz, 1H), 4.09 (dd, J = 7.4,4.3 Hz, 1H), 3.57 - 3.48 (m, 1H), 3.24 (dt, J = 9.9,6.7 Hz, 1H), 1.90 - 1.78 (m, 3H), 1.68 - 1.57 (m, 1H). |
| | (2S)-N-({3-[(3-chlorophenyl) methoxy]phenyl}methyl)-1-(5-chlorothiophene-2-sulfonyl) pyrrolidine-2-carboxamide | | |

**Table 41**

| Example No. | structure compound name | MS(ESI) *m*/*z*(M+H)⁺ | NMR |
|---|---|---|---|
| 103 | 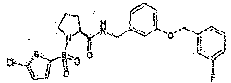 | 509 | ¹H NMR (400 MHz, DMSO) δ 8.59 (t, J = 6.0 Hz, 1H), 7.68 (d, J = 4.1 Hz, 1H), 7.43 (td, J = 8.0, 6.0 Hz, 1H), 7.37 (d, J = 4.1 Hz, 1H), 7.31 - 7.20 (m, 3H), 7.15 (tdd, J = 8.6, 2.3, 0.6 Hz, 1H), 6.98 - 6.93 (m, 1H), 6.91-6.83 (m, 2H), 5.11 (s, 2H), 4.33 (dd, J = 15.4, 6.2 Hz, 1H), 4.25 (dd, J = 15.5, 5.8 Hz, 1H), 4.09 (dd, J = 7.3, 4.4 Hz, 1H), 3.55 - 3.50 (m, 1H), 3.24 (dt, J = 10.6, 6.7 Hz, 1H), 1.91-1.79 (m, 3H), 1.68 - 1.57 (m, 1H). |
| | (2S)-1-(5-chlorothiophene-2-sulfonyl)-N-({3-[(3-trifluorophenyl)methoxy] phenyl}methyl)pyrrolidine-2-carboxamide | | |
| 104 | 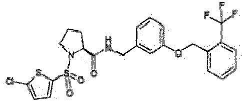 | 559 | ¹H NMR (400 MHz, DMSO) δ 8.61 (t, J = 6.0 Hz, 1H), 7.83 - 7.68 (m, 3H), 7.66 (d, J = 4.1 Hz, 1H), 7.58 (t, J = 7.5 Hz, 1H), 7.36 (d, J = 4.1 Hz, 1H), 7.25 (t, J = 7.9 Hz, 1H), 6.97 - 6.95 (m, 1H), 6.93 - 6.83 (m, 2H), 5.21 (s, 2H), 4.34 (dd, J = 15.5, 6.3 Hz, 1H), 4.26 (dd, J = 15.5, 5.9 Hz, 1H), 4.09 (dd, J = 7.5, 4.1 Hz, 1H), 3.57 - 3.48 (m, 1H), 3.23 (dt, J = 10.7, 6.8 Hz, 1H), 1.90 -1.77 (m, 3H), 1.68 - 1.58 (m, 1H). |
| | (2S)-1-(5-chlorothiophene-2-sulfonyl)-N-((3-{[2-(trifluoromethyl)phenyl] methoxy}phenyl)methyl) pyrrolidine-2-carboxamide | | |
| 105 | 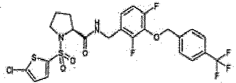 | 595 | - |
| | (2S)-1-(5-chlorothiophene-2-sulfonyl)-N-((2,4-difluoro-3-{[4-(trifluoromethyl)phenyl] methoxy}phenyl)methyl) pyrrolidine-2-carboxamide | | |
| 106 | 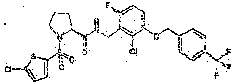 | 611 | - |
| | (2S)-N-((2-chloro-6-fluoro-3-{[4-(trifluoromethyl)phenyl] methoxy}phenyl)methyl)-1-(5-chlorothiophene-2-sulfonyl) pyrrolidine-2-carboxamide | | |

### Example 107: Synthesis of (2S)-N-[(3-benzyloxyphenyl)methyl]-1-(5-chlorothiophene-2-sulfonyl)-2,5-dihydropyrrole-2-carboxamide (107)

To A-2 (0.048 g, 0.17 mmol), C-6 (0.050 g, 0.15 mmol), WSC hydrochloride (0.047 g, 0.25 mmol) and HOAt (0.034 g, 0.25 mmol) were added triethylamine (68 µL, 0.50 mmol) and dichloromethane (1.5 mL), and the mixture was stirred at room temperature for several hours. The solvent was evaporated, and the obtained residue was purified by high performance liquid chromatography (water-acetonitrile, each containing 0.1% trifluoroacetic acid) to give the title compound (0.058 g, 0.12 mmol, 70%).
MS(ESI) m/z 489 (M+H)⁺

Compounds 108 - 113 described in Table 42 and Table 43 were synthesized using the corresponding A-2 and A-3, A-6 and A-7, A-10 - A-12, C-6 and C-7, and commercially available reagents and by an operation similar to Example 107.

**Table 42**

| Example No. | structure compound name | MS(ESI) *m*/*z*(M+H)⁺ | NMR |
|---|---|---|---|
| 107 | 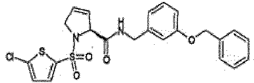 | 489 | - |
| | (2S)-N-[(3-benzyloxyphenyl) methyl]-1-(5-chlorothiophene-2-sulfonyl)-2,5-dihydropyrrole-2-carboxamide | | |
| 108 | 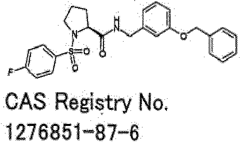 | 469 | - |
| | (2S)-N-[(3-benzyloxyphenyl) methyl]-1-(4-fluorophenyl) sulfonyl-pyrrolidine-2-carboxamide | | |
| 109 | 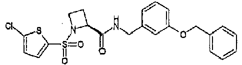 | 477 | - |
| | (2S)-N-[(3-benzyloxyphenyl) methyl]-1-(5-chlorothiophene-2-sulfonyl)azetidine-2-carboxamide | | |
| 110 | 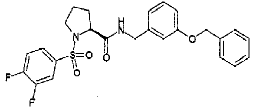 | 487 | - |
| | (2S)-N-[(3-benzyloxyphenyl) methyl]-1-(3,4-difluorophenyl) sulfonyl-pyrrolidine-2-carboxamide | | |

**Table 43**

| Example No. | structure compound name | MS(ESI) *m*/*z*(M+H)⁺ | NMR |
|---|---|---|---|
| 111 | 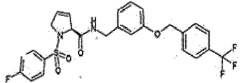 | 535 | - |
| | (2S)-1-(4-fluorophenyl)sulfonyl-N-((3-{[4-(trifluoromethyl) phenyl]methoxy}phenyl) methyl)-2,5-dihydropyrrole-2-carboxamide | | |
| 112 | 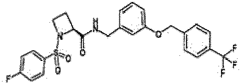 | 523 | - |
| | (2S)-1-(4-fluorophenyl)sulfonyl-N-((3-{[4-(trifluoromethyl) phenyl]methoxy}phenyl) methyl)azetidine-2-carboxamide | | |
| 113 | 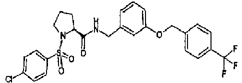 | 553 | - |
| | (2S)-1-(4-chlorophenyl)sulfonyl-N-((3-{[4-(trifluoromelhyl) phenyl]methoxy}phenyl) methyl)pyrrolidine-2-carboxamide | | |

Examples 114 - 119 described in Table 44 were synthesized using the corresponding B-14 and commercially available reagents and by an operation similar to Example 1.

**Table 44**

| Example No. | Structure | Compound name | **MS(ESI) *m*/*z* (M+H)⁺** |
|---|---|---|---|
| **114** | 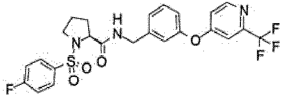 | (2S)-1-(4-fluorophenyl)sulfonyl-N-[[3-[[2-(trifluoromethyl)-4-pyridyl]oxy]phenyl] methyl]pyrrolidine-2-carboxamide | **524** |
| **115** | 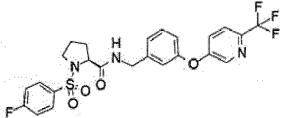 | (2S)-1-(4-fluorophenyl)sulfonyl-N-[[3-[[6-(trifluoromethyl)-3-pyridyl]oxy]phenyl] methyl]pyrrolidine-2-carboxamide | **524** |
| **116** | 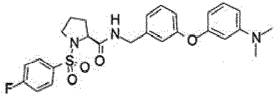 | (2S)-N-[[3-[3-(dimethylamino)phenoxy] phenyl]methyl]-1-(4-fluorophenyl) sulfonyl-pyrrolidine-2-carboxamide | **498** |
| **117** | 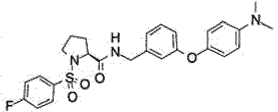 | (2S)-N-[[3-[4-(dimethylamino)phenoxy] phenyl]methyl]-1-(4-fluorophenyl) sulfonyl-pyrrolidine-2-carboxamide | **498** |
| **118** | 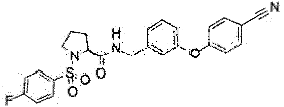 | (2S)-N-[[3-(4-cyanophenoxy)phenyl] methyl]-1-(4-fluorophenyl)sulfonyl-pyrrolidine-2-carboxamide | **480** |
| **119** | 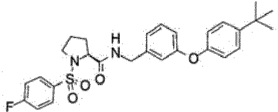 | (2S)-N-[[3-(4-tert-butylphenoxy) phenyl]methyl]-1-(4-fluorophenyl) sulfonyl-pyrrolidine-2-carboxamide | **511** |

Examples 120 - 150 described in Tables 45 - 49 were synthesized using the corresponding B-14 and B-15 and commercially available reagents and by an operation similar to Example 91.

**Table 45**

| Example No. | Structure | Compound name | **MS(ESI) *m*/*z* (M+H)⁺** |
|---|---|---|---|
| **120** | 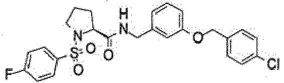 | (2S)-N-[[3-[(4-chlorophenyl)methoxy] phenyl]methyl]-1-(4-fluorophenyl) sulfonyl-pyrrolidine-2-carboxamide | **503** |
| **121** | 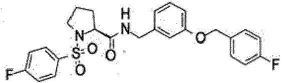 | (2S)-N-[[3-[(4-fluorophenyl)methoxy] phenyl]methyl]-1-(4-fluorophenyl) sulfonyl-pyrrolidine-2-carboxamide | **487** |
| **122** | 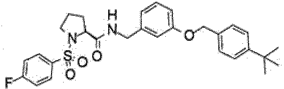 | (2S)-N-[[3-[(4-tert-butylphenyl) methoxy]phenyl]methyl]-1-(4-fluorophenyl)sulfonyl-pyrrolidine-2-carboxamide | **525** |
| **123** | 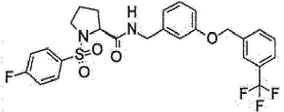 | (2S)-1-(4-fluorophenyl)sulfonyl-N-[[3-[[3-(trifluoromethyl)phenyl]methoxy] phenyl]methyl]pyrrolidine-2-carboxamide | **537** |
| **124** | 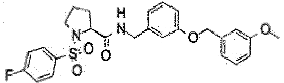 | (2S)-1-(4-fluorophenyl)sulfonyl-N-[[3-[(3-methoxyphenyl)methoxy]phenyl]methyl] pyrrolidine-2-carboxamide | **499** |
| **125** | 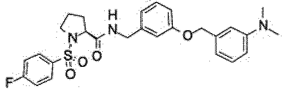 | (2S)-N-[[3-[[3-(dimethylamino) phenyl]methoxy]phenyl]methyl]-1-(4-fluorophenyl)sulfonyl-pyrrolidine-2-carboxamide | **512** |
| **126** | 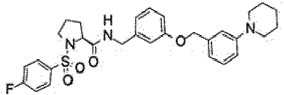 | (2S)-1-(4-fluorophenyl)sulfonyl-N-[[3-[[3-(1-piperidyl)phenyl]methoxy] phenyl]methyl]pyrrolidine-2-carboxamide | **552** |

**Table 46**

| Example No. | structure | compound name | **MS(ESI) *m*/*z* (M+H)⁺** |
|---|---|---|---|
| **127** | 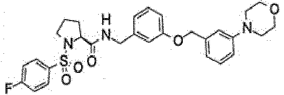 | (2S)-1-(4-fluorophenyl)sulfonyl-N-[[3-[(3-morpholinophenyl)methoxy]phenyl] methyl]pyrrolidine-2-carboxamide | **554** |
| **128** | 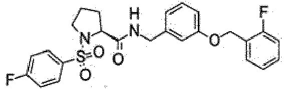 | (2S)-N-[[3-[(2-fluorophenyl)methoxy] phenyl]methyl]-1-(4-fluorophenyl) sulfonyl-pyrrolidine-2-carboxamide | **487** |
| **129** | 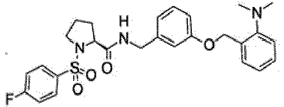 | (2S)-N-[[3-[[2-(dimethylamino)phenyl] methoxy]phenyl]methyl]-1-(4-fluorophenyl)sulfonyl-pyrrolidine-2-carboxamide | **512** |
| **130** | 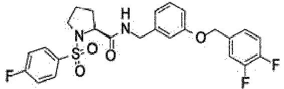 | (2S)-N-[[3-[(3,4-difluorophenyl)methoxy] phenyl]methyl]-1-(4-fluorophenyl) sulfonyl-pyrrolidine-2-carboxamide | **505** |
| **131** | 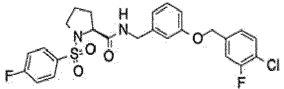 | (2S)-N-[[3-[(4-chloro-3-fluoro-phenyl) methoxy]phenyl]methyl]-1-(4-fluorophenyl)sulfonyl-pyrrolidine-2-carboxamide | **521** |
| **132** | 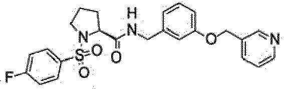 | (2S)-1-(4-fluorophenyl)sulfonyl-N-[[3-(3-pyridylmethoxy)phenyl]methyl] pyrrolidine-2-carboxamide | **470** |
| **133** | 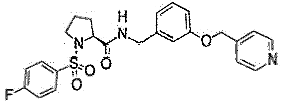 | (2S)-1-(4-fluorophenyl)sulfonyl-N-[[3-(4-pyridylmethoxy)phenyl]methyl] pyrrolidine-2-carboxamide | **470** |

**Table 47**

| Example No. | structure | compound name | **MS(ESI) *m*/*z* (M+H)⁺** |
|---|---|---|---|
| **134** | 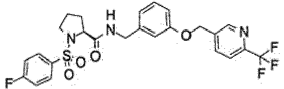 | (2S)-1-(4-fluorophenyl)sulfonyl-N-[[3-[[6-(trifluoromethyl)-3-pyridyl]methoxy] phenyl]methyl]pyrrolidine-2-carboxamide | **538** |
| **135** | 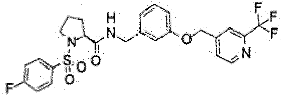 | (2S)-1-(4-fluorophenyl)sulfonyl-N-[[3-[[2-(trifluoromethyl)-4-pyridyl]methoxy] phenyl]methyl]pyrrolidine-2-carboxamide | **538** |
| **136** | 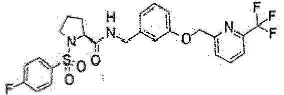 | (2S)-1-(4-fluorophenyl)sulfonyl-N-[[3-[[6-(trifluoromethyl)-2-pyridyl]methoxy] phenyl]methyl]pyrrolidine-2-carboxamide | **538** |
| **137** | 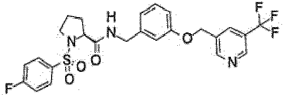 | (2S)-1-(4-fluorophenyl)sulfonyl-N-[[3-[[5-(trifluoromethyl)-3-pyridyl]methoxy] phenyl]methyl]pyrrolidine-2-carboxamide | **538** |
| **138** | 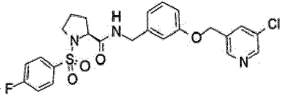 | (2S)-N-[[3-[(5-chloro-3-pyridyl)methoxy] phenyl]methyl]-1-(4-fluorophenyl) sulfonyl-pyrrolidine-2-carboxamide | **504** |
| **139** | 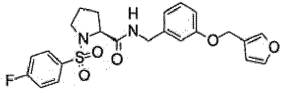 | (2S)-1-(4-fluorophenyl)sulfonyl-N-[[3-(3-furylmethoxy)phenyl]methyl]pyrrolidine-2-carboxamide | **459** |
| **140** | 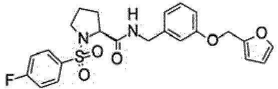 | (2S)-1-(4-fluorophenyl)sulfony]-N-[[3-(2-furylmethoxy)phenyl]methyl]pyrrolidine-2-carboxamide | **459** |

**Table 48**

| Example No. | structure | compound name | **MS(ESI) *m*/*z* (M+H)⁺** |
|---|---|---|---|
| **141** | 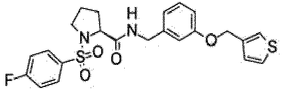 | (2S)-1-(4-fluorophenyl)sulfonyl-N-[[3-(3-thienylmethoxy)phenyl]methyl] pyrrolidine-2-carboxamide | **475** |
| **142** | 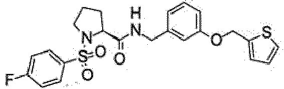 | (25)-1-(4-fluorophenyl)sulfonyl-N-[[3-(2-thienylmethoxy)phenyl]methyl] pyrrolidine-2-carboxamide | **475** |
| **143** | 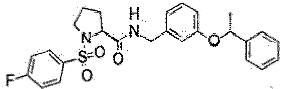 | (2S)-1-(4-fluorophenyl)sulfonyl-N-[[3-[(1R)-1-phenylethoxy]phenyl]methyl] pyrrolidine-2-carboxamide | **483** |
| **144** | 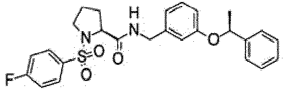 | (2S)-1-(4-fluorophenyl)sulfonyl-N-[[3-((1S)-1-phenylethoxy]phenyl]methyl] pyrrolidine-2-carboxamide | **483** |
| **145** | 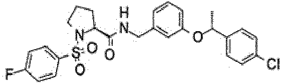 | (2S)-N-[[3-[(1R)-1-(4-chlorophenyl) ethoxy]phenyl]methyl]-1-(4-fluorophenyl)sulfonyl-pyrrolidine-2-carboxamide | **517** |
| **146** | 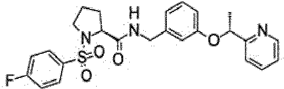 | (2S)-1-(4-fluorophenyl)sulfonyl-N-[[3-[(1R)-1-(2-pyridyl)ethoxy]phenyl]methyl] pyrrolidine-2-carboxamide | **484** |
| **147** | 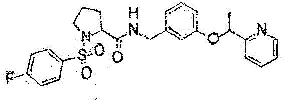 | (2S)-1-(4-fluorophenyl)sulfonyl-N-[[3-[(1S)-1-(2-pyridyl)ethoxy]phenyl]methyl] pyrrolidine-2-carboxamide | **484** |

**Table 49**

| Example No. | structure | compound name | **MS(ESI) *m*/*z* (M+H)⁺** |
|---|---|---|---|
| **148** | 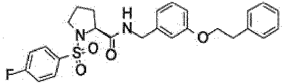 | (2S)-1-(4-fluorophenyl)sulfonyl-N-[(3-phenethyloxyphenyl)methyl]pyrrolidine-2-carboxamide | **483** |
| **149** | 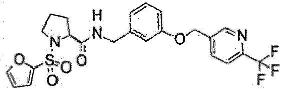 | (2S)-1-(2-furylsulfonyl)-N-[[3-[[6-(trifluoromethyl)-3-pyridyl]methoxy] phenyl]methyl]pyrrolidine-2-carboxamide | **510** |
| **150** | 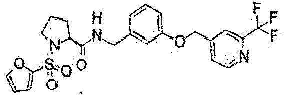 | (2S)-1-(2-furylsulfonyl)-N-[[3-[[2-(trifluoromethyl)-4-pyridyl]methoxy] phenyl]methyl]pyrrolidine-2-carboxamide | **510** |

Examples 151 - 184 described in Tables 50 - 54 were synthesized using the corresponding A-6, A-10, A-13 - A32, C-1 - C-2C, C-6 - C-7, C-11 and commercially available reagents and by an operation similar to Example 107.

**Table 50**

| Example No. | structure | compound name | **MS(ESI) *m*/*z* (M+H)⁺** |
|---|---|---|---|
| **151** | 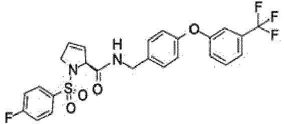 | (2S)-1-(4-fluorophenyl)sulfonyl-N-[[4-[3-(trifluoromethyl)phenoxy]phenyl] methyl]-2,5-dihydropyrrole-2-carboxamide | **521** |
| **152** | 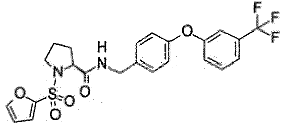 | (2S)-1-(2-furylsulfonyl)-N-[[4-{3-(trifluoromethyl)phenoxy]phenyl] methyl]pyrrolidine-2-carboxamide | **495** |
| **153** | 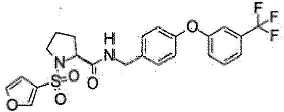 | (2S)-1-(3-furylsulfonyl)-N-[[4-[3-(trifluoromethyl)phenoxy]phenyl] methyl]pyrrolidine-2-carboxamide | **495** |
| **154** | 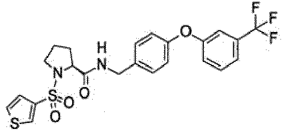 | (2S)-1-(3-thienylsulfonyl)-N-[[4-[3-(trifluoromethyl)phenoxy]phenyl]methyl] pyrrolidine-2-carboxamide | **511** |
| **155** | 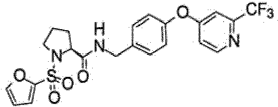 | (2S)-1-(2-furylsulfonyl)-N-[[4-[[2-(trifluoromethyl)-4-pyridyl]oxy]phenyl] methyl]pyrrolidine-2-carboxamide | **496** |
| **156** | 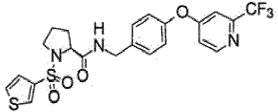 | (2S)-1-(3-thienylsulfonyl)-N-[[4-[[2-(trifluoromethyl)-4-pyridyl]oxy]phenyl] methyl]pyrrolidine-2-carboxamide | **512** |
| **157** | 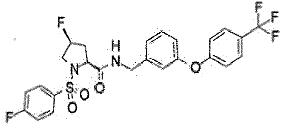 | (2S,4S)-4-fluoro-1-(4-fluorophenyl) sulfonyl-N-[[3-[4-(trifluoromethyl) phenoxy]phenyl]methyl]pyrrolidine-2-carboxamide | **541** |

**Table 51**

| Example No. | structure | compound name | **MS(ESI) *m*/*z* (M+H)⁺** |
|---|---|---|---|
| **158** | 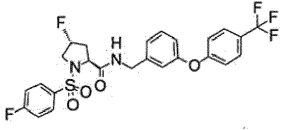 | (2S,4R)-4-fluoro-1-(4-fluorophenyl) sulfonyl-N-[[3-[4-(trifluoromethyl) phenoxy]phenyl]methyl]pyrrolidine-2-carboxamide | **541** |
| **159** | 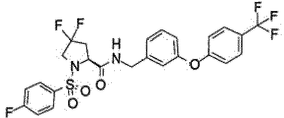 | (2S)-4,4-difluoro-1-(4-fluorophenyl) sulfonyl-N-[[3-[4-(trifluoromethyl) phenoxy]phenyl]methyl]pyrrolidine-2-carboxamide | **559** |
| **160** | 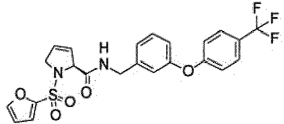 | (2S)-1-(2-furylsulfonyl)-N-[[3-[4-(trifluoromethyl)phenoxy]phenyl] methyl]-2,5-dihydropyrrole-2-carboxamide | **493** |
| **161** | 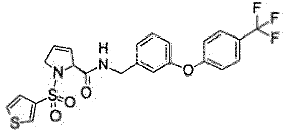 | (2S)-1-(3-thienylsulfonyl)-N-[[3-[4-(trifluoromethyl)phenoxy]phenyl] methyl]-2,5-dihydropyrrole-2-carboxamide | **509** |
| **162** | 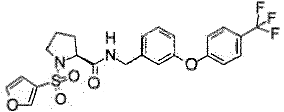 | (2S)-1-(3-furylsulfonyl)-N-[[3-[4-(trifluoromethyl)phenoxy]phenyl] methyl]pyrrolidine-2-carboxamide | **495** |
| **163** | 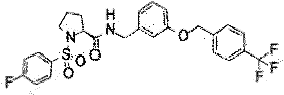 | (2S)-1-(4-fluprophenyl)sulfonyl-N-[[3-[[4-(trifluoromethyl)phenyl]methoxy]phenyl] methyl]pyrrolidine-2-carboxamide | **537** |
| **164** | 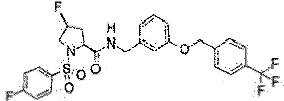 | (2S,4S)-4-fluoro-1-(4-fluorophenyl) sulfonyl-N-[[3-[[4-(trifluoromethyl) phenyl]methoxy]phenyl]methyl] pyrrolidine-2-carboxamide | **555** |

**Table 52**

| Example No. | structure | compound name | **MS(ESI) *m*/*z* (M+H)⁺** |
|---|---|---|---|
| **165** | 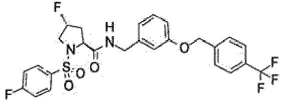 | (2S,4R)-4-fluoro-1-(4-fluorophenyl) sulfonyl-N-[[3-[[4-(trifluoromethyl) phenyl]methoxy]phenyl]methyl] pyrrolidine-2-carboxamide | **555** |
| **166** | 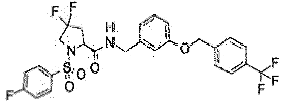 | (2S)4,4-difluoro-1-(4-fluorophenl) sulfonyl-N-[[3-[[4-(trifluoromethyl) phenyl]methoxy]phenyl]methyl] pyrrolidine-2-carboxamide | **573** |
| **167** | 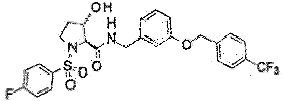 | (2S,3S)-1-(4-fluorophenyl)sulfonyl-3-hydroxy-N-[[3-[[4-(trifluoromethyl) phenyl]methoxy]phenyl]methyl] pyrrolidine-2-carboxamide | **553** |
| **168** | 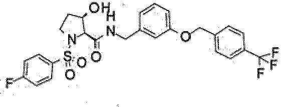 | (2S,3R)-1-(4-fluorophenyl)sulfonyl-3-hydroxy-N-[[3-[[4-(trifluoromethyl) phenyl]methoxy]phenyl]methyl] pyrrolidine-2-carboxamide | **553** |
| **169** | 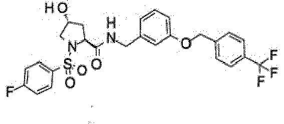 | (2S,4R)-1-(4-fluorophenyl)sulfonyl-4-hydroxy-N-[[3-[[4-(trifluoromethyl) phenyl]methoxy]phenyl]methyl] pyrrolidine-2-carboxamide | **553** |
| **170** | 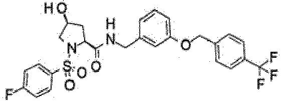 | (2S,4S)-1-(4-fluorophenyl)sulfonyl-4-hydroxy-N-[[3-[[4-(trifluoromethyl) phenyl]methoxy]phenyl]methyl] pyrrolidine-2-carboxamide | **553** |
| **171** | 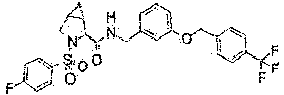 | (1R,4S,5S)-3-(4-fluorophenyl)sulfonyl-N-[[3-[[4-(trifluoromethyl)phenyl]methoxy] phenyl]methyl]-3-azabicyclo[3,1,0]hexane-4-carboxamide | **549** |

**Table 53**

| Example No. | structure | compound name | **MS(ESI) *m*/*z* (M+H)⁺** |
|---|---|---|---|
| **172** | 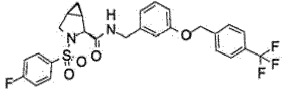 | (1S,4S,5R)-3-(4-fluorophenyl)sulfonyl-N-[[3-[[4-(trifluoromethyl)phenyl]methoxy] phenyl]methyl]-3-azabicyclo[3.1.0]hexane-4-carboxamide | **549** |
| **173** | 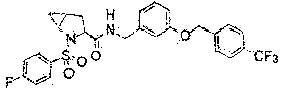 | (1R,3S,5R)-4-(4-fluorophenyl)sulfonyl-N-[[3-[[4-(trifluoromethyl)phenyl]methoxy] phenyl]methyl]-4-azabicyclo[3.1.0]hexane-3-carboxamide | **549** |
| **174** | 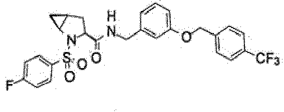 | (1S,3S,5S)-4-(4-fluorophenyl)sulfonyl-N-[[3-[[4-(trifluoromethyl)phenyl]methoxy] phenyl]methyl]-4-azabicyclo[3.1.0]hexane-3-carboxamide | **549** |
| **175** | 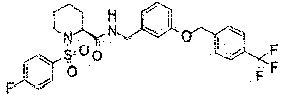 | (2S)-1-(4-fluorophenyl)sulfonyl-N-[[3-[[4-(trifluoromethyl)phenyl]methoxy]phenyl] methyl]piperidine-2-carboxamide | **551** |
| **176** | 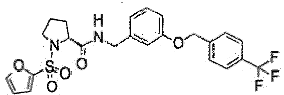 | (2S)-1-(2-furylsulfonyl)-N-[[3-[[4-(trifluoromethyl)phenyl]methoxy]phenyl] methyl]pyrrolidine-2-carhoxamide | **509** |
| **177** | 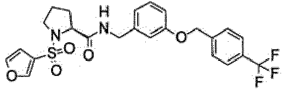 | (2S)-1-(3-furylsulfonyl)-N-[[3-[[4-(trifluoromethyl)phenyl]methoxy]phenyl] methyl]pyrrolidine-2-carboxamide | **509** |
| **178** | 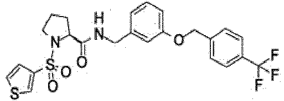 | (2S)-1-(3-thienylsulfonyl)-N-[[3-[[4-(trifluoromethyl)phenyl]methoxy]phenyl] methyl]pyrrolidine-2-carboxamide | **525** |

**Table 54**

| Example No. | structure | compound name | **MS(ESI) *m*/*z* (M+H)⁺** |
|---|---|---|---|
| **179** | 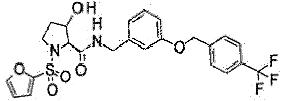 | (2S,3S)-1-(2-furylsulfonyl)-3-hydroxy-N-[[3-[[4-(trifluoromethyl)phenyl]methoxy] phenyl]methyl]pyrrolidine-2-carboxamide | **525** |
| **180** | 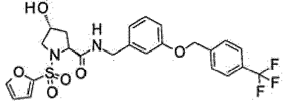 | (2S,4R)-1-(2-furylsulfonyl)-4-hydroxy-N-[[3-[[4-(trifluoromethyl)phenyl]methoxy] phenyl]methyl]pyrrolidine-2-carboxamide | **525** |
| **181** | 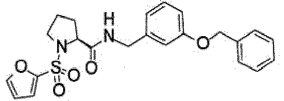 | (2S)-N-[(3-benzyloxyphenyl)methyl]-1-(2-furylsulfonyl)pyrrolidine-2-carboxamide | **441** |
| **182** | 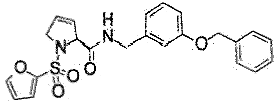 | (2S)-N-[(3-benzyloxyphenyl)methyl]-1-(2-furylsulfonyl)-2,5-dihydropyrrole-2-carboxamide | **439** |
| **183** | 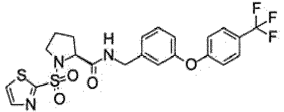 | (2S)-1-thiazol-2-ylsulfonyl-N-[[3-[4-(trifluoromethyl)phenoxy]phenyl]methyl] pyrrolidine-2-carboxamide | **512** |
| **184** | 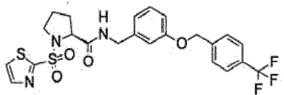 | (2S)-1-thiazol-2-ylsulfonyl-N-[[3-[[4-(trifluoromethyl)phenyl]methoxy]phenyl] methyl]pyrrolidine-2-carboxamide | **526** |

### Example 185: Synthesis of (2S)-1-(4-cyanophenyl)sulfonyl-N-[[3-[[4-(trifluoromethyl)phenyl]methoxy]phenyl]methyl]pyrrolidine-2-carboxamide (185)

D-3 (25 mg, 0.060 mmol) was dissolved in acetonitrile (1 mL), 4-cyanobenzenesulfonylchloride (15 mg, 0.072 mmol) and triethylamine (0.021 mL, 0.15 mmol) were added, and the mixture was stirred at room temperature overnight. The reaction mixture was concentrated under reduced pressure, and the obtained residue was purified by high performance liquid chromatography (water-acetonitrile, each containing 0.1% trifluoroacetic acid) to give the title compound (23 mg, 0.042 mmol, 71%).
MS (ESI) m/z 544 (M+H)⁺
¹H NMR (400 MHz, DMSO) δ 8.60 (t, J= 6.0, 6.0 Hz, 1H), 8.14-8.07 (m, 2H), 8.07 - 7.99 (m, 2H), 7.79 - 7.71 (m, 2H), 7.69-7.61 (m, 2H), 7.25 (dd, J= 8.3, 7. 5 Hz, 1H), 6. 98 (dd, J= 2.7, 1.5 Hz, 1H), 6.93 - 6.85 (m, 2H), 5.22 (s, 2H), 4.38 - 4.20 (m, 2H), 4.14 (dd, J = 7.2, 4.4 Hz, 1H), 3.56 - 3.44 (m, 1H), 3.28 - 3.18 (m, 1H), 1.89 - 1.74 (m, 3H), 1.63 - 1.48 (m, 1H).

Examples 186 - 215 described in Tables 55 - 59 were synthesized using the corresponding D-1 - D-4 and commercially available reagents and by an operation similar to Example 185.

**Table 55**

| Example No. | structure | compound name | **MS(ESI) *m*/*z* (M+H)⁺** |
|---|---|---|---|
| **185** | 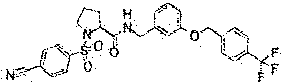 | (2S)-1-(4-cyanaphenyl)sulfonyl-N-[[3-[[4-(trifluoromethyl)phenyl]methoxy] phenyl]methyl]pyrrolidine-2-carboxamide | **544** |
| **186** | 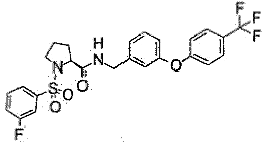 | (2S)-1-(3-fluorophenyl)sulfonyl-N-[[3-[4-(trifluoromethyl)phenoxy]phenyl]methyl] pyrrolidine-2-carboxamide | **523** |
| **187** | 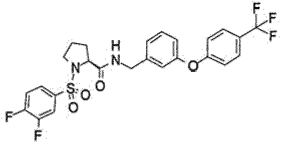 | (2S)-1-(3,4-difluorophenyl)sulfonyl-N-[[3-[4-(trifluoromethyl)phenoxy]phenyl] methyl]pyrrolidine-2-carboxamide | **541** |
| **188** | 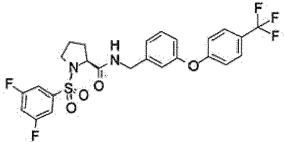 | (2S)-1-(3,5-difluorophenyl)sulfonyl-N-[[3-[4-(trifluoromethyl)phenoxy]phenyl] methyl]pyrrolidine-2-carboxamide | **541** |
| **189** | 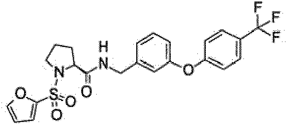 | (2S)-1-(2-furylsulfonyl)-N-[[3-[4-(trifluoromethyl)phenoxy]phenyl] methyl]pyrrolidine-2-carboxamide | **495** |
| **190** | 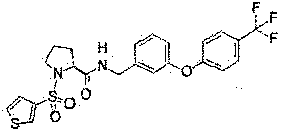 | (2S)-1-(3-thienylsulfonyl)-N-[[3-[4-(trifluoromethyl)phenoxy]phenyl]methyl] pyrrolidine-2-carboxamide | **511** |
| **191** | 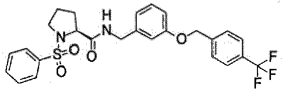 | (2S)-1-(benzenesulfonyl)-N-[[3-[[4-(trifluoromethyl)phenyl]methoxy]phenyl] methyl]pyrrolidine-2-carboxamide | **519** |

**Table 56**

| Example No. | structure | compound name | **MS(ESI) *m*/*z* (M+H)⁺** |
|---|---|---|---|
| **192** | 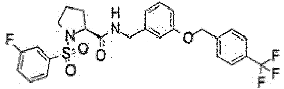 | (2S)-1-(3-fluorophenyl)sulfonyl-N-[[3-[[4-(trifluoromethyl)phenyl]methoxy]phenyl] methyl]pyrrolidine-2-carboxamide | **537** |
| **193** | 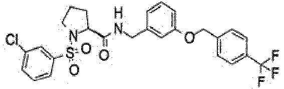 | (2S)-1-(3-chlorophenyl)sulfonyl-N-[[3-[[4-(trifluoromethyl)phenyl]methoxy]phenyl] methyl]pyrrolidine-2-carboxamide | **553** |
| **194** | 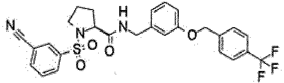 | (2S)-1-(3-cyanophenyl)sulfonyl-N-[[3-[[4-(trifluoromethyl)phenyl]methoxy]phenyl] methyl]pyrrolidine-2-carboxamide | **544** |
| **195** | 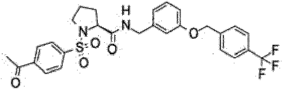 | (2S)-1-(4-acetylphenyl)sulfonyl-N-[[3-[[4-(trifluoromethyl)phenyl]methoxy]phenyl] methyl]pyrrolidine-2-carboxamide | **561** |
| **196** | 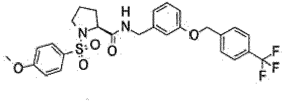 | (25)-1-(4-methoxyphenyl)sulfonyl-N-[[3-[[4-(trifluoromethyl)phenyl]methoxy] phenyl]methyl]pyrrolidine-2-carboxamide | **549** |
| **197** | 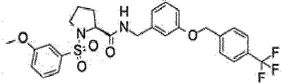 | (2S)-1-(3-methoxyphenyl)sulfonyl-N-[[3-[[4-(trifluoromethyl)phenyl]methoxy] phenyl]methyl]pyerrolidine-2-carboxamide | **549** |
| **198** | 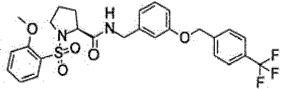 | (2S)-1-(2-methoxyphenyl)sulfonyl-N-[[3-[[4-(trifluoromethyl)phenyl]methoxy] phenyl]methyl]pyrrolidine-2-carboxamide | **549** |

**Table 57**

| Example No. | structure | compound name | **MS(ESI) m/z (M+H)⁺** |
|---|---|---|---|
| **199** | 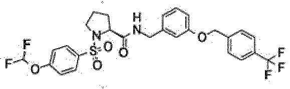 | (2S)-1-[4-(difluoromethoxy)phenyl] sulfonyl-N-[[3-[[4-(trifluoromethyl) phenyl]methoxy]phenyl]methyl] pyrrolidine-2-carboxamide | **585** |
| **200** | 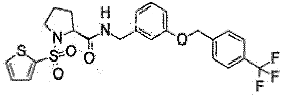 | (2S)-1-(2-thienylsulfonyl)-N-[[3-[[4-(trifluoromethyl)phenyl]methoxy]phenyl] methyl]pyrrolidine-2-carboxamide | **525** |
| **201** | 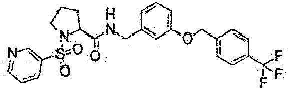 | (2S)-1-(3-pyridylsulfonyl)-N-[[3-[[4-(trifluoromethyl)phenyl]methoxy]phenyl] methyl]pyrrolidine-2-carboxamide | **520** |
| **202** | 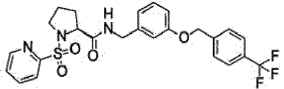 | (2S)-1-(2-pyridylsulfonyl)-N-[[3-[[4-(trifluoromethyl)phenyl]methoxy]phenyl] methyl]pyrrolidine-2-carboxamide | **520** |
| **203** | 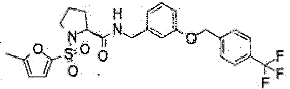 | (2S)-1-[(5-methyl-2-furyl)sulfonyl]-N-[[3-[[4-(trifluoromethyl)phenyl]methoxy] phenyl]methyl]pyrrolidine-2-carboxamide | **523** |
| **204** | 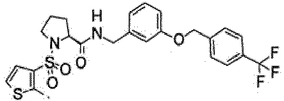 | (2S)-1-[(2-methyl-3-thienyl)sulfonyl]-N-[[3-[[4-(trifluoromethyl)phenyl]methoxy] phenyl]methyl]pyrrolidine-2-carboxamide | **539** |
| **205** | 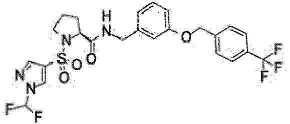 | (2S)-1-[1-(difluoromethyl)pyrazol-4-yl] sulfonyl-N-[[3-[[4-(trifluoromethyl) phenyl]methoxy]phenyl]methyl] pyrrolidine-2-carboxamide | **559** |

**Table 58**

| Example No. | structure | compound name | **MS(ESI) *m*/*z* (M+H)**⁺ |
|---|---|---|---|
| **206** | 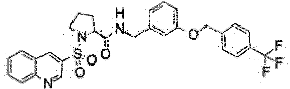 | (2S)-1-(3-quinolylsulfonyl)-N-[[3-[[4-(trifluoromethyl)phenyl]methoxy]phenyl] methyl]pyrrolidine-2-carboxamide | **570** |
| **207** | 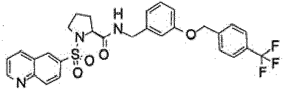 | (2S)-1-(6-quinolylsulfonyl)-N-[[3-[[4-(trifluoromethyl)phenyl]methoxy]phenyl] methyl]pyrrolidine-2-carboxamide | **570** |
| **208** | 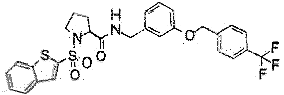 | (25)-1-(benzothiophen-2-ylsulfonyl)-N-[[3-[[4-(trifluoromethyl)phenyl]methoxy] phenyl]methyl]pyrrolidine-2-carboxamide | **575** |
| **209** | 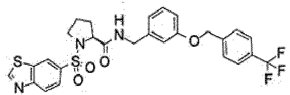 | (2S)-1-(1,3-benzothiazol-6-ylsulfonyl)-N-[[3-[[4-(trifluoromethyl)phenyl]methoxy] phenyl]methyl]pyrrolidine-2-carboxamide | **576** |
| **210** | 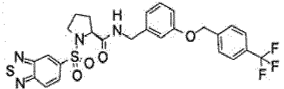 | (2S)-1-(2,1,3-benzothiadiazol-5-ylsulfonyl)-N-[[3-[[4-(trifluoromethyl) phenyl]methoxy]phenyl]methyl] pyrrolidine-2-carboxamide | **577** |
| **211** | 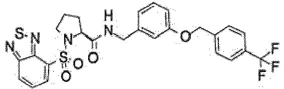 | (2S)-1-(2,1,3-benzothiadiazol-4-ylsulfonyl)-N-[[3-[[4-(trifluoromethyl) phenyl]methoxy]phenyl]methyl] pyrrolidine-2-carboxamide | **577** |
| **212** | 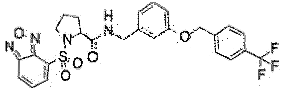 | (2S)-1-(2,1,3-benzoxadiazol-4-ylsulfonyl)-N-[[3-[[4-(trifluoromethyl)phenyl) methoxy]phenyl]methyl]pyrrolidine-2-carboxamide | **561** |

**Table 59**

| Example No. | structure | compound name | **MS(ESI) *m*/*z* (M+H)⁺** |
|---|---|---|---|
| **213** | 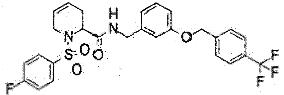 | (2S)-1-(4-fluorophenyl)sulfonyl-N-[[3-[[4-(trifluoromethyl)phenyl]methoxy]phenyl] methyl]-3,6-dihydro-2H-pyridine-2-carboxamide | **549** |
| **214** | 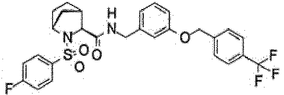 | (1S,2S,4R)-3-(4-fluorophenyl)sulfonyl-N-[[3-[[4-(trifluoromethyl)phenyl]methoxy] phenyl]methyl]-3-azabicyclo[2.2.1] heptane-2-carboxamide | **563** |
| **215** | 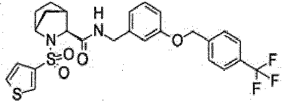 | (1S,2S,4R)-3-(3-thienylsulfonyl)-N-[[3-[[4-(trifluoromethyl)phenyl]methoxy]phenyl] methyl]-3-azabicyclo[2.2.1]heptane-2-carboxamide | **551** |

### Example 216: Synthesis of (2S)-1-(4-pyridylsulfonyl)-N-[[3-[[4-(trifluoromethyl)phenyl]methoxy]phenyl]methyl]pyrrolidine-2-carboxamide (216)

Under an argon atmosphere, to tetrahydrofuran (0.3 mL) were added 1.6 mol/L n-butyllithium (n-hexane solution, 0.13 mL, 0.21 mmol) and 1.0 mol/L di-n-butylmagnesium (n-heptane solution, 0.21 mL, 0.21 mmol), and the mixture was cooled to - 10°C. To the reaction mixture was added a solution of 4-bromopyridine (prepared by dissolving 4-bromopyridine hydrobromide (0.10 g, 0.51 mmol) in dichloromethane, washing with saturated aqueous sodium hydrogen carbonate, drying the dichloromethane layer over sodium sulfate, and evaporating the solvent) in tetrahydrofuran (0.6 mL), and the mixture was stirred at -10°C for 50 min. To the reaction mixture was added a solution of sulfuryl chloride (0.062 mL, 0.77 mmol) in toluene (0.6 mL), and the mixture was warmed to 10°C and stirred for 10 min. To the reaction mixture were added D-3 (30 mg, 0.072 mmol) and triethylamine (0.20 mL, 1.4 mmol), and the mixture was stirred at room temperature for 10 min. Ethyl acetate was added to the reaction mixture, the mixture was washed successively with saturated aqueous sodium hydrogen carbonate and saturated brine, and the organic layer was dried over sodium sulfate. The desiccant was filtered off, the solvent was evaporated, and the obtained residue was purified by high performance liquid chromatography (water-acetonitrile, each containing 0.1% trifluoroacetic acid) to give the title compound (24 mg, 0.046 mmol, 9%).
MS (ESI) m/z 520 (M+H)⁺
¹H NMR (400 MHz, DMSO-d₆,) δ 8.92 - 8.84 (m, 2H), 8.59 (t, J = 6.0, 6.0 Hz, 1H), 7.84 - 7.78 (m, 2H), 7.75 (d, J = 8.0 Hz, 2H), 7.65 (d, J = 8.0 Hz, 2H), 7.25 (t, J = 7.9, 7.9 Hz, 1H), 6.98 (dd, J = 2.5, 1.5 Hz, 1H), 6.93 - 6.85 (m, 2H), 5.22 (s, 2H), 4.36 - 4.21 (m, 2H), 4.17 (dd, J = 7.3, 4.1 Hz, 1H), 3.54 - 3.45 (m, 1H), 3.29 - 3.20 (m, 1H), 1.89 - 1.75 (m, 3H), 1.63 - 1.53 (m, 1H).

Examples 217 - 219 described in Table 60 were synthesized using the corresponding D-3 and commercially available reagents and by an operation similar to Example 216.

**Table 60**

| Example No. | structure | compound name | **MS(ESI) *m*/*z* (M+H)⁺** |
|---|---|---|---|
| **216** | 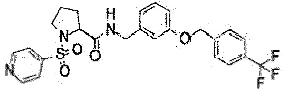 | (2S)-1-(4-pyridylsulfonyl)-N-[[3-[[4-(trifluoromethyl)phenyl]methoxy]phenyl] methyl]pyrrolidine-2-carboxamide | **520** |
| **217** | 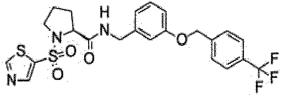 | (2S)-1-thiazol-5-ylsulfonyl-N-[[3-[[4-(trifluoromethyl)phenyl]methoxy]phenyl] methyl]pyrrolidine-2-carboxamide | **526** |
| **218** | 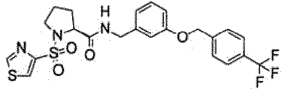 | (2S)-1-thiazol-4-ylsulfonyl-N-[[3-[[4-(trifluoromethyl)phenyl]methoxy]phenyl] methyl]pyrrolidine-2-carboxamide | **526** |
| **219** | 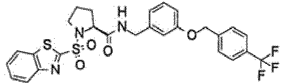 | (2S)-1-(1,3-benzothiazol-2-ylsulfonyl)-N-[[3-[[4-(trifluoromethyl)phenyl]methoxy] phenyl]methyl]pyrrolidine-2-carboxamide | **576** |

### Example 220: Synthesis of (2S)-1-imidazol-1-ylsulfonyl-N-[[3-[[4-(trifluoromethyl)phenyl]methoxy]phenyl]methyl]pyrrolidine-2-carboxamide (220)

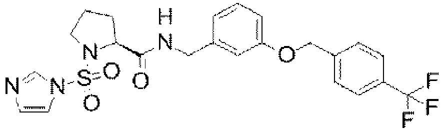

To a solution of sulfonyldiimidazole (62 mg, 0.31 mmol) in dichloromethane was added methyl trifluoromethanesulfonate (0.034 mL, 0.31 mmol), and the mixture was stirred at room temperature for 2 hr. The reaction mixture was concentrated under reduced pressure, D-3 (0.10 g, 0.24 mmol) and triethylamine (0.034 mL, 0.24 mmol) were added to the obtained residue, and the mixture was stirred at room temperature for 2 hr. The reaction mixture was concentrated under reduced pressure, and the obtained residue was purified by silica gel column chromatography (dichloromethane-methanol) to give the title compound (67 mg, 0.13 mmol, 55%).
MS (ESI) m/z 509 (M+H)⁺

### Experimental Example 1: Measurement of TRPA1 antagonist activity human TRPA1 expression plasmid

As cDNA encoding human TRPA1 (GenBank accession No. NM_0078332), a commercially available product was purchased (manufactured by Kazusa DNA Research Institute, clone No.: pFN21AB7348, item No.: FHC07217). Using this as a template, full-length human TRPA1 gene was amplified using the primer sequences shown below, by reaction using DNA polymerase (manufactured by Stratagene, trade name: PfuUltra High-Fidelity DNA Polymerase).

primer 1: 5'-AACTTTAGTAAGCTTCGATCGCCATGAAG-3' (SEQ ID NO:1)
primer 2: 5'-GTACCGATCTAGAATTCGTTTA**CTA**AGGCTCAAG-3' (SEQ ID NO:2)

A recognition site (underlined) of restriction enzyme HindIII was added to the 5' side, and XbaI site (underlined) was added to the 3' side, and GTT of the template sequence was changed to termination codon TAG (bold). The obtained double stranded DNA was enzyme-digested with HindIII and XbaI, and introduced into a multicloning site of expression plasmid pcDNA3.1/zeo(+) (manufactured by Invitrogen) to give a human TRPA1 expression plasmid.

### Cell preparation

Human embryonic kidney-derived 293T cells were cultured in Dulbecco's Modified Eagle Medium containing 10% fetal bovine serum, 10 unit penicillin, and 10 µg streptomycin, plated on a petri dish having a diameter of 10 cm at 3×10⁶ cells, and cultured for 24 hr. A medium containing a reduced amount of serum (manufactured by Invitrogen, trade name: OPTI-MEM, 600 µL), a gene insertion reagent (manufactured by Mirus Bio, trade name: Mirus TransIT-293, 18 µL), and human TRPA1 expression plasmid (6 µg) were mixed, the total amount of the mixture was added to the cells on the petri dish to allow for gene transfer. The cells were recovered about 8 hr later, plated on a poly-D-lysine coated 384 well black/clear bottom plate at 7,500 - 12,000 cells/well, and cultured overnight.

### Measurement of intracellular calcium increase

The 384 well plate was recovered, and the medium was removed. A calcium-bonded fluorescent indicator (manufactured by Molecular Device, trade name: Calcium4 Assay Kit, 40 µL) dissolved in assay buffer (1xHBSS, 20 mM HEPES, pH 7.2) was added, and the cells were stained in a 37°C incubator for 1 hr. The cells were taken out at room temperature and left standing for 15 min or more. A test substance (10 µL) was added by a 384 well type dispenser, and the mixture was incubated at room temperature for 10 min. Then, allylisothiocyanate (12.5 µL) at a final concentration of 20 µM was added by a fluorescence imaging plate reader (manufactured by Molecular Device, FLIPR), and changes in the relative fluorescence intensity were measured for 5 min.

### Test substance preparation

The test substance was dissolved in dimethyl sulfoxide and serially diluted with an assay buffer containing 0.1% bovine serum albumin (1xHBSS, 20 mM HEPES, pH 7.2) to a 5-fold concentration of the evaluation concentration. Allylisothiocyanate, which is a known TRPA1 activator, was dissolved in dimethyl sulfoxide to 100 mM, and further diluted 5-fold (100 µM) of the final concentration, like the test substance.

### Calculation of antagonist activity

Under the test substance-free conditions, the maximum variation range of the fluorescence intensity before and after allylisothiocyanate stimulation was defined to be 100% activity rate, and the variation range before and after buffer stimulation was defined as 0% activity rate. The activity rate on addition of the test substance was determined, and the numerical value obtained by subtracting the activity rate from 100 was defined to be an inhibitory rate. IC50, which is the concentration of the test substance necessary for reaching the 50% inhibitory rate, was calculated from the sigmoid approximate curve by a spreadsheet software Excel-Fit.

The results are shown in Tables 61-63. As shown, the compound of the present invention exhibited a superior TRPA1 antagonist activity.

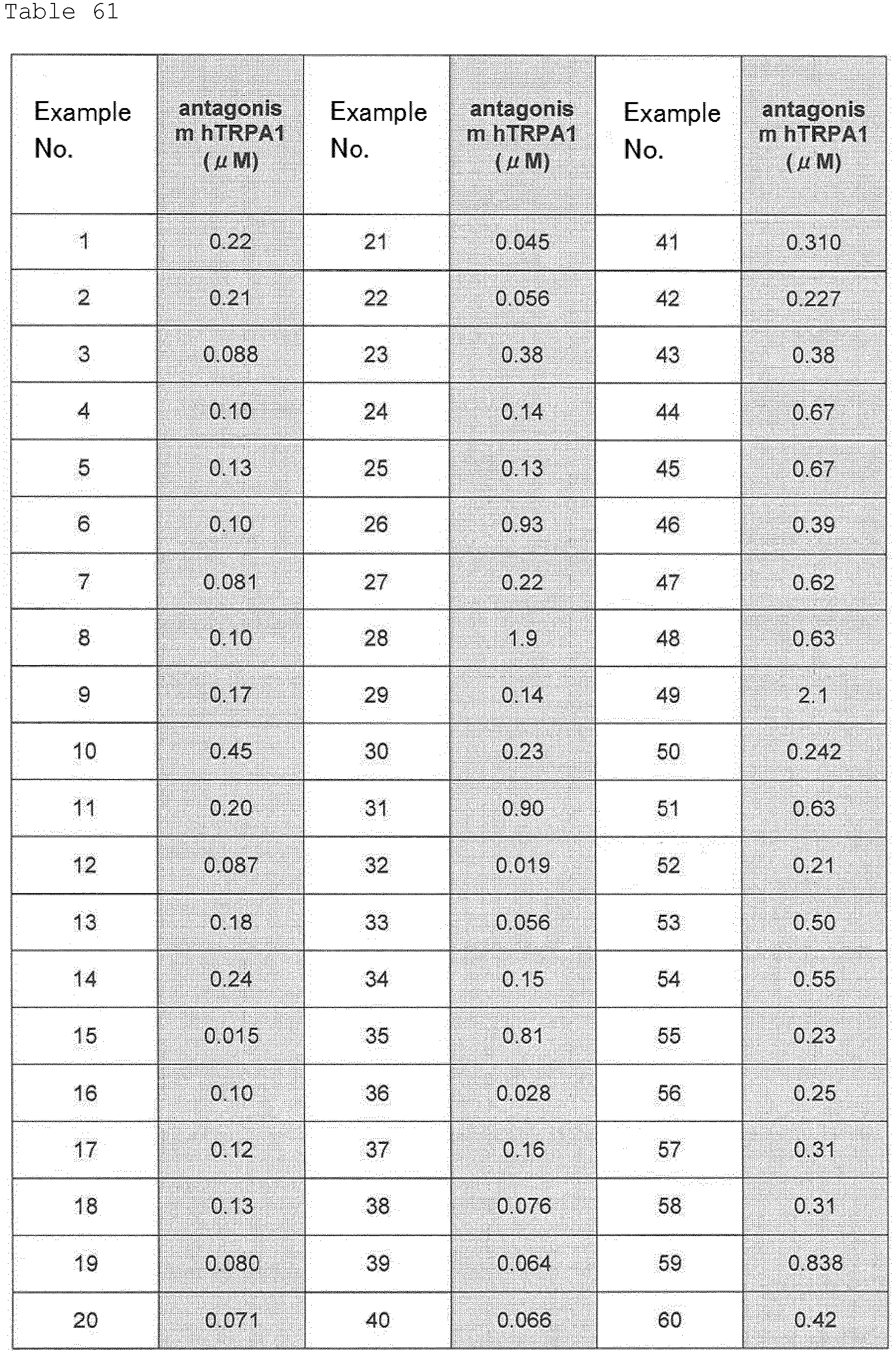

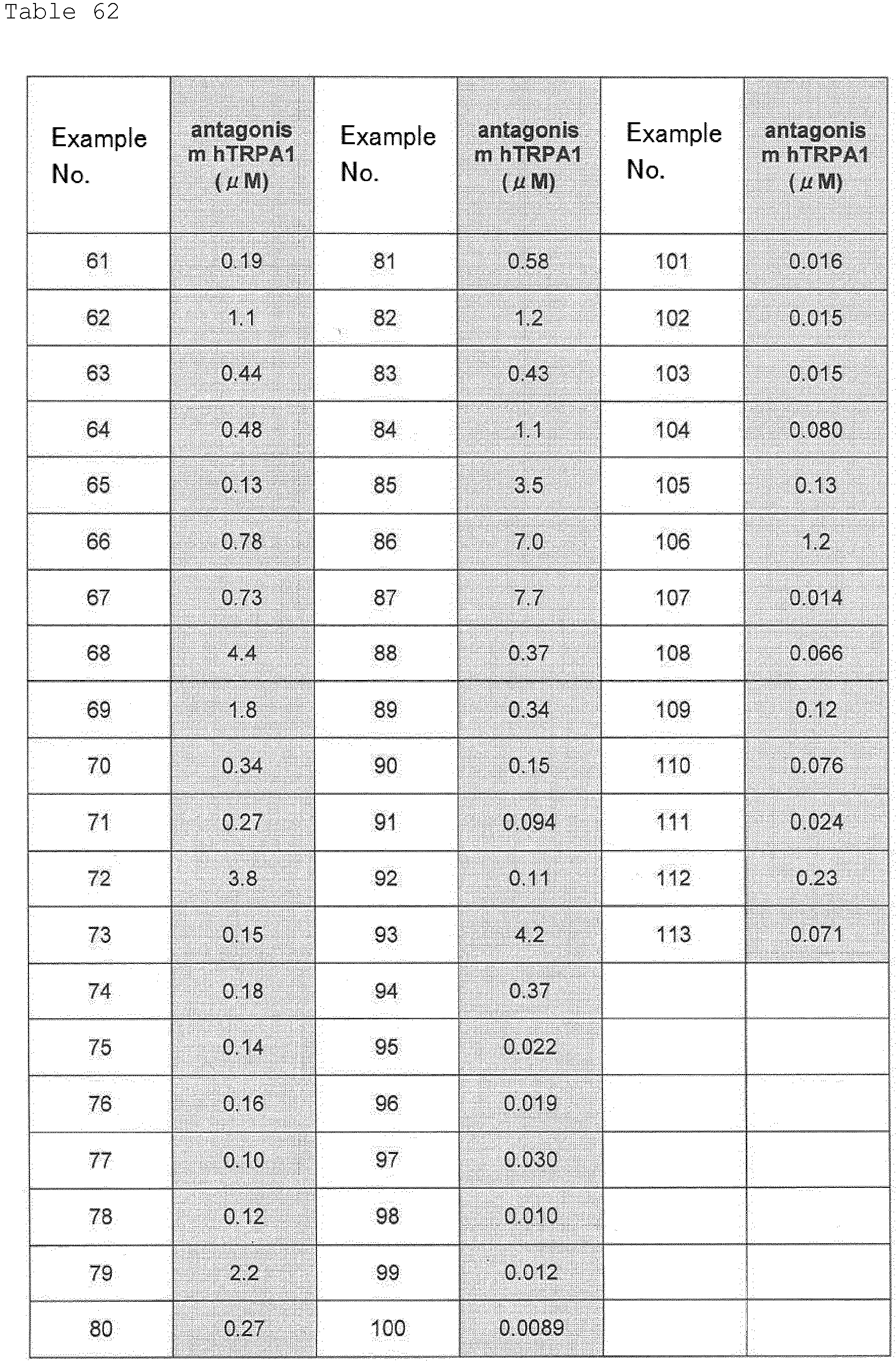

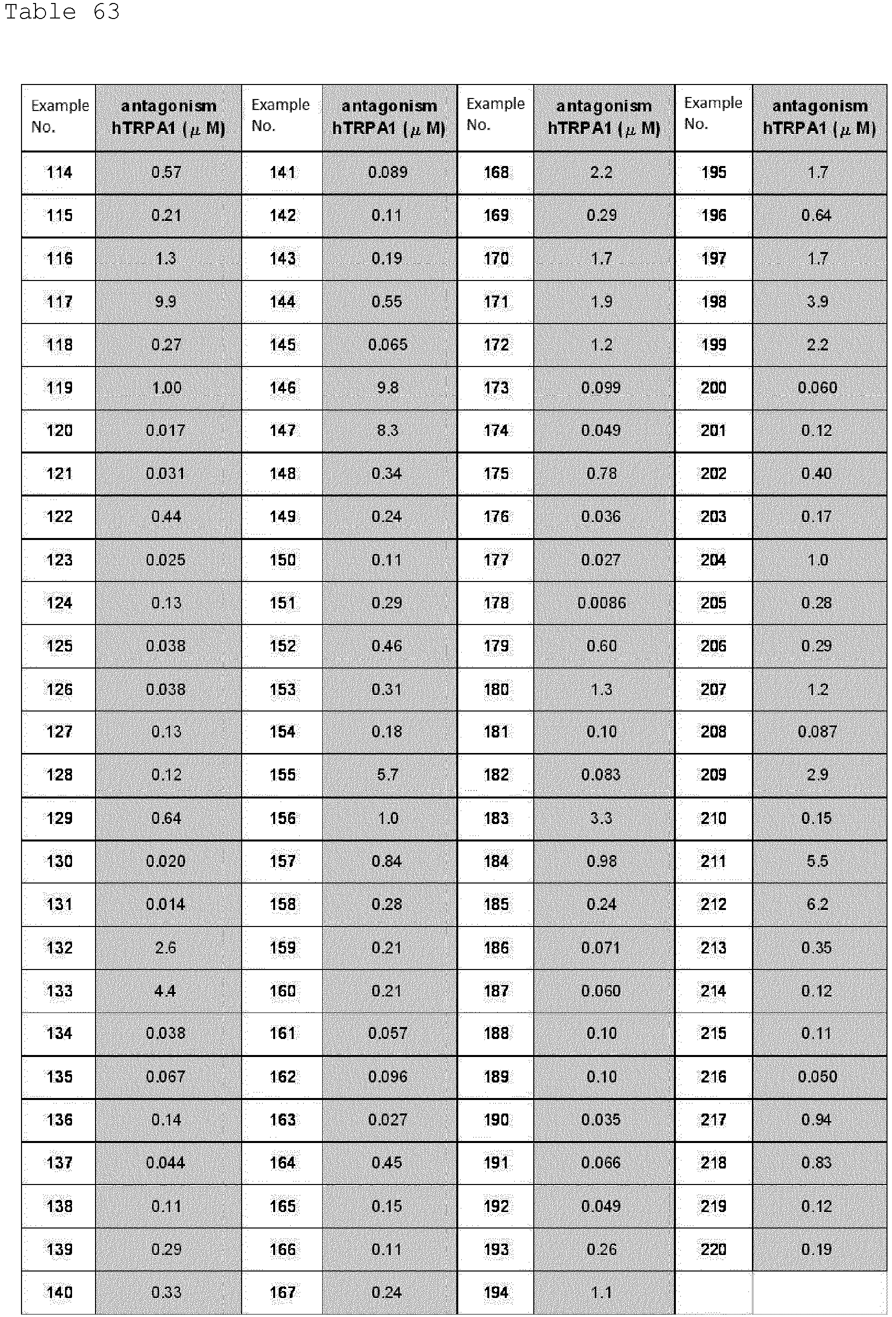

### Experimental Example 2: AITC-induced pain behavior evaluation test

To evaluate the effectiveness of the test substance in vivo, allylisothiocyanate (AITC)-induced pain behavior evaluation test was performed using mice.

AITC is a selective agonist of the TRPA1 channel, and causes a pain behavior by TRPA1 activation when administered to animal. Therefore, the intensity of the TRPA1 antagonist action of the test substance in the living body can be evaluated by measuring the pain behavior after AITC administration.

### 1. Administration of test substance to animal

As the animal, male ICR mice (6- to 8-week-old) are used. The mice are fasted the previous day of the test. The test substance is intraperitoneally or orally administered for evaluation. In the case of intraperitoneal administration, the substance is administered 30 min before the AITC administration. In the oral administration, the substance is administered 60 min before the AITC administration.

### 2. AITC-induced pain behavior evaluation

AITC (0.1%) is subcutaneously administered to the sole of the left leg of mouse, and the time when the mouse shows a behavior of licking the sole of the leg (Licking time) in 5 min immediately after the AITC administration is measured.

### 3. Calculation of inhibitory rate

The licking time of the vehicle administration group in each test is taken as 100%, and the activity rate by administration of each test substance (Licking time on test substance administration/Licking time of vehicle administration group x 100) is determined, and the numerical value obtains by subtracting the activity rate from 100 is calculated as an inhibitory rate.

By the above-mentioned method, it can be confirmed that the compound of the present invention has a superior TRPA1 antagonist activity, is superior in vivo kinetics, and shows superior efficacy in animal model.

The effectiveness of the compound of the present invention was confirmed by the above-mentioned evaluation test.

### Industrial Applicability

The compound of the present invention has a superior TRPA1 antagonist activity, and therefore, is useful for the prophylaxis /or treatment of diseases involving TRPA1 (e.g., pain associated diseases, digestive tract diseases, lung diseases, bladder diseases, inflammatory diseases, dermatic diseases, and neurological diseases).

This application is based on patent application No. 2012-276283 (filing date December 18, 2012) filed in Japan, the contents of which are encompassed in full herein.

## Claims

1. A medicament composed of a compound represented by the formula (I): 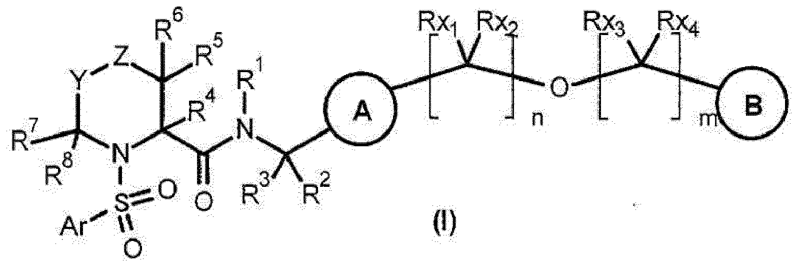 wherein
Ar is a C₆₋₁₀ aryl group optionally having 1 - 4 substituents or a C₁₋₉ heteroaryl group optionally having 1 - 4 substituents;
Y is -C(Ry1)(Ry2)-, or a single bond;
Z is -C(Rz1)(Rz2)-, an oxygen atom, a sulfur atom, or a single bond;
n is 0 or 1;
m is 0 or 1;
provided n+m≤1;
partial structure (1) 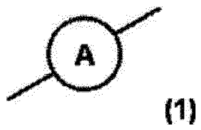 is a phenylene group optionally having 1 - 4 substituents, or a divalent group optionally having 1 - 3 substituents of 5-membered or 6-membered heteroaromatic ring containing any 1 - 3 hetero atoms selected from oxygen atom, nitrogen atom and sulfur atom;
partial structure (2) 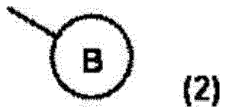 is a C₆₋₁₀ aryl group optionally having 1 - 4 substituents or a C₁₋₉ heteroaryl group optionally having 1 - 4 substituents;
R¹ is a hydrogen atom, a C₁₋₆ alkyl group, a C₂₋₆ alkenyl group,
a C₂₋₆ alkynyl group, a halogeno C₁₋₆ alkyl group, a cyclic C₃₋₆ alkyl group, or a C₁₋₆ alkyl group substituted by cyclic C₃₋₆ alkyl group;
R² and R³ are the same or different and each is a hydrogen atom, a C₁₋₆ alkyl group, a C₂₋₆ alkenyl group, a C₂₋₆ alkynyl group; R⁴ - R⁸, Ry1 - Ry2, Rz1 and Rz2 are the same or different and each is a hydrogen atom, a C₁₋₆ alkyl group, a C₂₋₆ alkenyl group, a C₂₋₆ alkynyl group, a halogeno C₁₋₆ alkyl group, a C₁₋₆ alkoxy group, a hydroxyl group, a halogeno C₁₋₆ alkoxy group, an amino group, an amino group mono- or di-substituted by a C₁₋₆ alkyl group, or a halogeno group;
respective R⁴ - R⁸, Ry1 - Ry2, Rz1 or Rz2 on adjacent carbon atom are optionally joined to form a double bond and/or a ring; respective R⁵ and R⁶, R⁷ and R⁸, Ry1 and Ry2, or Rz1 and Rz2 on the same carbon atom are optionally joined to form a ring; Rx1 - Rx4 are the same or different and each is a hydrogen atom, a C₁₋₆ alkyl group, a C₂₋₆ alkenyl group, a C₂₋₆ alkynyl group, a C₁₋₆ alkyl group substituted by a hydroxyl group, a C₁₋₆ alkyl group substituted by an amino group, a C₁₋₆ alkyl group substituted by an amino group mono- or di-substituted by a C₁₋₆ alkyl group;
and respective Rx1 and Rx2, or Rx3 and Rx4 on the same carbon atom are optionally joined to form a ring, or a pharmaceutically acceptable salt thereof.

2. The medicament according to claim 1, wherein Y and/or Z are/is a single bond.

3. The medicament according to claim 1 or 2, wherein, in the aforementioned formula (I),
Ar is a phenyl group optionally having 1 or 2 substituents or a 5- or 6-membered monocyclic heteroaryl group optionally having 1 or 2 substituents.

4. The medicament according to any one of claims 1 to 3, wherein, in the aforementioned formula (I),
the partial structure (1) is either a phenylene group optionally having 1 - 3 substituents, or a divalent group of a pyridine ring optionally having 1 - 3 substituents.

5. The medicament according to any one of claims 1 to 3, wherein, in the aforementioned formula (I),
the partial structure (1) is 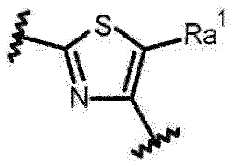 in the above-mentioned structure, Ra¹ is a hydrogen atom or a substituent.

6. The medicament according to any one of claims 1 to 5, wherein, in the aforementioned formula (I),
the partial structure (2) is a phenyl group optionally having 1 - 3 substituents, or a pyridyl group, a quinolyl group or an isoquinolyl group, each of which optionally has 1 - 3 substituents.

7. The medicament according to any one of claims 1 to 6, wherein, in the aforementioned formula (I),
n=0.

8. The medicament according to any one of claims 1 to 7, wherein, in the aforementioned formula (I),
R² and R³ are each a hydrogen atom.

9. The medicament according to any one of claims 1 to 8, wherein, in the aforementioned formula (I),
the partial structure (2) is a phenyl group optionally having a substituent or a pyridyl group optionally having a substituent.

10. The medicament according to any one of claims 1 to 9, which is a TRPA1 antagonist.

11. The medicament according to claim 10, which is for the prophylaxis and/or treatment of a disease involving TRPA1.

12. The medicament according to claim 11, wherein the disease involving TRPA1 is selected from the group consisting of pain associated diseases, inflammatory diseases, digestive tract diseases, lung diseases, bladder diseases, dermatic diseases, and neurological diseases.

13. The medicament according to claim 11, wherein the disease involving TRPA1 is selected from chronic pain, acute pain, asthma, chronic obstructive pulmonary disease, functional gastrointestinal disorder, erosive esophagitis, inflammatory bowel disease, and pruritus.

14. The compound represented by the formula (IA): 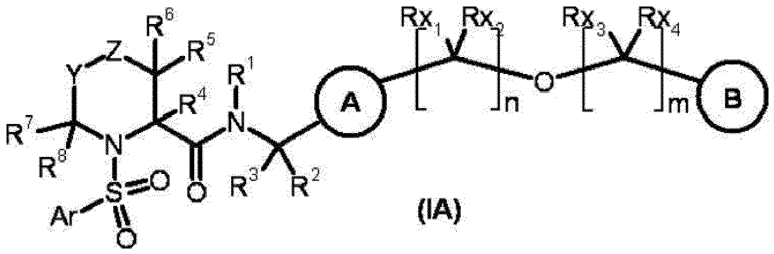 wherein
Ar is a C₆₋₁₀ aryl group optionally having 1 - 4 substituents or a C₁₋₉ heteroaryl group optionally having 1 - 4 substituents;
Y is -C(Ry1)(Ry2)-, or a single bond;
Z is -C(Rz1)(Rz2)-, an oxygen atom, a sulfur atom, or a single bond;
n is 0 or 1;
m is 0 or 1;
provided n+m≤1;
partial structure (1) 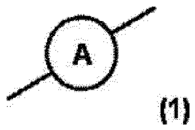 is a phenylene group optionally having 1 - 4 substituents, or a divalent group optionally having 1 - 3 substituents of 5-membered or 6-membered heteroaromatic ring containing any 1 - 3 hetero atoms selected from oxygen atom, nitrogen atom and sulfur atom;
partial structure (2) 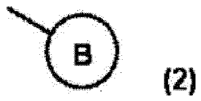 is a C₆₋₁₀ aryl group optionally having 1 - 4 substituents or a C₁₋₉ heteroaryl group optionally having 1 - 4 substituents;
R¹ is a hydrogen atom, a C₁₋₆ alkyl group, a C₂₋₆ alkenyl group,
a C₂₋₆ alkynyl group, a halogeno C₁₋₆ alkyl group, a cyclic C₃₋₆ alkyl group, or a C₁₋₆ alkyl group substituted by a cyclic C₃₋₆ alkyl group;
R² and R³ are the same or different and each is a hydrogen atom, a C₁₋₆ alkyl group, a C₂₋₆ alkenyl group, a C₂₋₆ alkynyl group;
R⁴ - R⁸, Ry1 - Ry2, Rz1 and Rz2 are the same or different and each is a hydrogen atom, a C₁₋₆ alkyl group, a C₂₋₆ alkenyl group, a C₂₋₆ alkynyl group, a halogeno C₁₋₆ alkyl group, a C₁₋₆ alkoxy group, a hydroxyl group, a halogeno C₁₋₆ alkoxy group, an amino group, an amino group mono- or di-substituted by a C₁₋₆ alkyl group, or a halogeno group;
respective R⁴ - R⁸, Ry1 - Ry2, Rz1 or Rz2 on adjacent carbon atom are optionally joined to form a double bond and/or a ring;
respective R⁵ and R⁶, R⁷ and R⁸, Ry1 and Ry2, or Rz1 and Rz2 on the same carbon atom are optionally joined to form a ring;
Rx1 - Rx4 are the same or different and each is a hydrogen atom, a C₁₋₆ alkyl group, a C₂₋₆ alkenyl group, a C₂₋₆ alkynyl group, a C₁₋₆ alkyl group substituted by a hydroxyl group, a C₁₋₆ alkyl group substituted by an amino group, a C₁₋₆ alkyl group substituted by an amino group mono- or di-substituted by a C₁₋₆ alkyl group;
and respective Rx1 and Rx2, or Rx3 and Rx4 on the same carbon atom are optionally joined to form a ring, or a pharmaceutically acceptable salt thereof, excluding the following compounds:
N-[[2-(2-fluorophenoxy)phenyl]methyl]-1-(2-thienylsulfonyl)pyrrolidine-2-carboxamide;
ethyl 5-[[2-[[[(2-phenoxyphenyl)methyl]amino]carbonyl]-1-pyrrolidinyl]sulfonyl]-2-furancarboxylate;
N-[[3-fluoro-4-(3-pyridinyloxy)phenyl]methyl]-1-(phenylsulfonyl)-pyrrolidine-2-carboxamide;
N-[(3-phenoxyphenyl)methyl]-1-(2-thienylsulfonyl)-pyrrolidine-2-carboxamide;
N-[(2-phenoxyphenyl)methyl]-1-(2-thienylsulfonyl)-pyrrolidine-2-carboxamide;
N-[[6-(2,5-dimethylphenoxy)-3-pyridinyl]methyl]-1-(phenylsulfonyl)-pyrrolidine-2-carboxamide;
N-[[2-(4-fluorophenoxy)-4-pyridinyl]methyl]-1-(phenylsulfonyl)-pyrrolidine-2-carboxamide;
N-[[6-(3-fluorophenoxy)-3-pyridinyl]methyl]-1-(phenylsulfonyl)-pyrrolidine-2-carboxamide;
1-(phenylsulfonyl)-N-[[3-(2-pyridinylmethoxy)phenyl]methyl]-pyrrolidine-2-carboxamide;
(2S)-1-[(4-methylphenyl)sulfonyl]-N-[[2-(phenylmethoxy)-4-pyridinyl]methyl]-pyrrolidine-2-carboxamide;
1-[(4-fluorophenyl)sulfonyl]-N-[[6-(4-methoxyphenoxy)-3-pyridinyl]methyl]-pyrrolidine-2-carboxamide;
1-[(4-fluorophenyl)sulfonyl]-N-[[3-methoxy-4-(3-pyridinylmethoxy)phenyl]methyl]-pyrrolidine-2-carboxamide;
N-[1-[4-(3-pyridinylmethoxy)phenyl]ethyl]-1-(2-thienylsulfonyl)-pyrrolidine-2-carboxamide;
N-[[2-(phenylmethoxy)phenyl]methyl]-1-(2-thienylsulfonyl)-pyrrolidine-2-carboxamide;
1-[(4-fluorophenyl)sulfonyl]-N-[[3-methoxy-4-(phenylmethoxy)phenyl]methyl]-pyrrolidine-2-carboxamide;
N-[[2-(phenylmethoxy)phenyl]methyl]-1-(2-thienylsulfonyl)-pyrrolidine-2-carboxamide;
N-[[6-(4-fluorophenoxy)-3-pyridinyl]methyl]-1-(2-thienylsulfonyl)-pyrrolidine-2-carboxamide;
1-[(4-fluorophenyl)sulfonyl]-N-[1-[3-(2-pyridinylmethoxy)phenyl]ethyl]-pyrrolidine-2-carboxamide;
N-[[4-(phenoxymethyl)phenyl]methyl]-1-(2-thienylsulfonyl)-pyrrolidine-2-carboxamide;
N-[[3-(phenylmethoxy)phenyl]methyl]-1-(2-thienylsulfonyl)-pyrrolidine-2-carboxamide;
1-[(4-fluorophenyl)sulfonyl]-N-[[4-(3-pyridinylmethoxy)phenyl]methyl]-pyrrolidine-2-carboxamide;
1-[(4-fluorophenyl)sulfonyl]-N-[[2-(phenylmethoxy)-4-pyridinyl]methyl]-pyrrolidine-2-carboxamide;
N-[[3-(2-pyridinylmethoxy)phenyl]methyl]-1-[(2,4,6-trimethylphenyl)sulfonyl]-pyrrolidine-2-carboxamide;
1-(phenylsulfonyl)-N-[[3-(2-pyridinylmethoxy)phenyl]methyl]-pyrrolidine-2-carboxamide;
N-[[2-(phenylmethoxy)-4-pyridinyl]methyl]-1-(2-thienylsulfonyl)-pyrrolidine-2-carboxamide;
N-[[6-(2,5-dimethylphenoxy)-3-pyridinyl]methyl]-1-(2-thienylsulfonyl)-2-piperidinecarboxamide;
N-[[2-(phenoxymethyl)phenyl]methyl]-1-(2-thienylsulfonyl)-pyrrolidine-2-carboxamide;
1-[(4-fluorophenyl)sulfonyl]-N-[[3-(phenylmethoxy)phenyl]methyl]-pyrrolidine-2-carboxamide;
1-[(4-fluorophenyl)sulfonyl]-N-[[3-fluoro-4-(3-pyridinyloxy)phenyl]methyl]-pyrrolidine-2-carboxamide;
N-[[3-methoxy-4-(4-pyridinylmethoxy)phenyl]methyl]-1-(phenylsulfonyl)-pyrrolidine-2-carboxamide;
(2S)-1-[(4-methylphenyl)sulfonyl]-N-[1-[3-(2-pyridinylmethoxy)phenyl]ethyl]-pyrrolidine-2-carboxamide;
1-[(3,4-dimethoxyphenyl)sulfonyl]-N-[(3-phenoxyphenyl)methyl]-pyrrolidine-2-carboxamide;
N-[1-[3-methoxy-4-(4-pyridinylmethoxy)phenyl]ethyl]-1-(phenylsulfonyl)-pyrrolidine-2-carboxamide;
2-[(3,4-dimethoxyphenyl)sulfonyl]-1,2,3,4-tetrahydro-N-[(3-phenoxyphenyl)methyl]-isoquinoline-3-carboxamide;
(2S)-1-[(3,5-dimethyl-4-isoxazolyl)sulfonyl]-N-[[4-(4-fluorophenoxy)phenyl]methyl]-pyrrolidine-2-carboxamide;
1,2,3,4-tetrahydro-2-[(4-methylphenyl)sulfonyl]-N-[(3-phenoxyphenyl)methyl]-isoquinoline-3-carboxamide;
(2S)-N-[[3-methoxy-4-(4-pyridylmethoxy)phenyl]methyl]-1-(p-tolylsulfonyl)pyrrolidine-2-carboxamide;
(2S)-N-[(4-benzyloxy-3-methoxy-phenyl)methyl]-1-(p-tolylsulfonyl)pyrrolidine-2-carboxamide;
(2S)-1-(4-fluorophenyl)sulfonyl-N-[[3-(2-pyridylmethoxy)phenyl]methyl]pyrrolidine-2-carboxamide;
(2S)-N-[[6-(2-ethoxyphenoxy)-3-pyridyl]methyl]-1-(p-tolylsulfonyl)pyrrolidine-2-carboxamide;
N-[[6-(2,5-dimethylphenoxy)-3-pyridyl]methyl]-1-(4-fluorophenyl)sulfonyl-pyrrolidine-2-carboxamide; and
N-[[6-(4-fluorophenoxy)-3-pyridyl]methyl]-1-(4-fluorophenyl)sulfonyl-pyrrolidine-2-carboxamide.

15. The compound according to claim 14, wherein Y and/or Z are/is a single bond, or a pharmaceutically acceptable salt thereof.

16. The compound according to claim 14 or 15, wherein, in the aforementioned formula (IA),
Ar is a phenyl group optionally having 1 or 2 substituents or a 5- or 6-membered monocyclic heteroaryl group optionally having 1 or 2 substituents, or a pharmaceutically acceptable salt thereof.

17. The compound according to any one of claims 14 to 16, wherein, in the aforementioned formula (IA),
the partial structure (1) is either a phenylene group optionally having 1 - 3 substituents, or a divalent group of a pyridine ring optionally having 1 - 3 substituents, or a pharmaceutically acceptable salt thereof.

18. The compound according to any one of claims 14 to 16, wherein, in the aforementioned formula (IA),
the partial structure (1) is 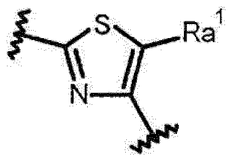 in the above-mentioned structure, Ra¹ is a hydrogen atom or a substituent,
or a pharmaceutically acceptable salt thereof.

19. The compound according to any one of claims 14 to 18, wherein, in the aforementioned formula (IA),
the partial structure (2) is a phenyl group optionally having 1 - 3 substituents, or a pyridyl group, a quinolyl group or an isoquinolyl group, each of which optionally has 1 - 3 substituents, or a pharmaceutically acceptable salt thereof.

20. The compound according to any one of claims 14 to 19, wherein, in the aforementioned formula (IA),
n=0, or a pharmaceutically acceptable salt thereof.

21. The compound according to any one of claims 14 to 20, wherein, in the aforementioned formula (IA),
R² and R³ are each a hydrogen atom, or a pharmaceutically acceptable salt thereof.

22. The compound according to any one of claims 14 to 21, wherein, in the aforementioned formula (IA),
the partial structure (2) is a phenyl group optionally having a substituent or a pyridyl group optionally having a substituent, or a pharmaceutically acceptable salt thereof.


### SEQUENCE LISTING

<110> AJINOMOTO CO., INC.
<120> Heterocyclic amide derivatives and pharmaceutical agents containing
   them
<130> 092101
<150> JP2012-276283
   <151> 2012-12-18
<160> 2
<170> PatentIn version 3.4
<210> 1
   <211> 29
   <212> DNA
   <213> Artificial
<220>
   <223> primer 1
<400> 1
   aactttagta agcttcgatc gccatgaag 29
<210> 2
   <211> 34
   <212> DNA
   <213> Artificial
<220>
   <223> primer 2
<400> 2
   gtaccgatct agaattcgtt tactaaggct caag 34

### SEQUENCE LISTING

<110> AJINOMOTO CO., INC.
<120> Heterocyclic amide derivatives and pharmaceutical agents containing
   them
<130> KMN/FP7138464
<140> EP13864883.7
   <141> 2013-12-17
<150> JP2012-276283
   <151> 2012-12-18
<160> 2
<170> PatentIn version 3.4
<210> 1
   <211> 29
   <212> DNA
   <213> Artificial
<220>
   <223> primer 1
<400> 1
   aactttagta agcttcgatc gccatgaag 29
<210> 2
   <211> 34
   <212> DNA
   <213> Artificial
<220>
   <223> primer 2
<400> 2
   gtaccgatct agaattcgtt tactaaggct caag 34